# EUROPEAN PATENT APPLICATION

(11) **EP 2 759 602 A1**
(43) Date of publication of application: **30.07.2014**
(21) Application number: 13152788.9
(22) Date of filing: 25.01.2013
(51) Int. Cl.: C12Q 1/68

(54) **Non-invasive prenatal genetic diagnostic methods**

(71) Applicant: Charité - Universitätsmedizin Berlin, 10117 Berlin (DE)
(72) Inventor: Kamhieh-Milz, Julian, 13349 Berlin (DE); Bal, Gürkan, 13353 Berlin (DE); Salama, Abdulgabar, Prof.Dr.med., 13467 Berlin (DE)
(74) Representative: Sokolowski, Fabian

(57) **Abstract**

Provided are non-invasive methods for determining the genetic status of a fetus, *i.e.* aneuplodies. Furthermore, non-invasive methods are provided for determining the fetal gender. In addition, the present invention relates to devices and kits adapted or useful for the methods of the present invention and to a use of non-coding RNA(s) (ncRNA) and/or precursor(s) thereof for non-invasive diagnostic methods of evaluation of fetal disorders and/or pathologies based on evaluation of differential expression of ncRNAs and/or precursors thereof.

## Description

### Field of the invention

The present invention generally relates to non-invasive methods for determining the genetic status of a fetus. Chromosomal aberrations such as fetal aneuploidy, in particular Trisomy 21 (T21) or also known as Down syndrome (DS), may be diagnosed from a biological sample via non-invasive means by use of the methods of the present invention which are based on the evaluation of differential expression of non-coding RNAs (ncRNA), in particular microRNAs (miRNAs). Furthermore, non-invasive methods are provided for determination of fetal gender. In addition, the present invention relates to devices and kits adapted or useful for the methods of the present invention and the use of an ncRNA or precursors thereof for non-invasive diagnostic methods for evaluation of pregnancy-associated disorders and/or fetal gender.

### BACKGROUND OF THE INVENTION

Chromosomal aneuploidy is a main cause of human prenatal and postnatal morbidity and mortality, and the most frequent genetic cause of mental retardation. The most common aneuploidy amongst live-born babies is T21, phenotypically manifested as DS [1, 2]. DS occurs in approximately 1 in 700 live births worldwide, with the incidence rising with advanced maternal age [3]. Prenatal testing for trisomies is available to women of advanced maternal age, with ultrasound findings consistent with DS or with previous fetuses with chromosomal abnormalities [4]. Currently, fetal genetic material is collected through procedures such as amniocentesis and chorionic villous sampling to enable the definitive diagnosis of fetal genetic diseases.

The two most commonly utilized non-invasive prenatal diagnostic (NIPD) tests are ultrasound and maternal serum marker testing. Currently, prenatal screening via ultrasound in the first trimester employs nuchal translucency (NT) and the assessment of the presence of nasal bones [6]. Maternal serum screening and ultrasound can be used for identification of individuals at risk for fetal aneuploidy, but are hampered by non-optimal sensitivities and high false-positive rates [14, 15]. In accordance with the operating mode in most hospitals, each woman with a positive result is offered, in most cases, unnecessarily, invasive testing. This redundant exposure involves various risks including fetal loss and entails ethical, legal and economic implications [5]. There is, therefore, a desire to develop non-invasive screening tests which have a high level of sensitivity and a negligible rate of false positives for the determination of the genetic status of a fetus, *e.g.,* genetic abnormalities such as aneuploidies. These and further technical problems, as defined in detail further below, have been solved by the embodiments characterized in the claims and described herein below.

### SUMMARY OF THE INVENTION

Since the detection of fetal cells in maternal blood, NIPD for the detection of chromosomal aneuploidies has been desired. Due to recent advances in whole-genome sequencing using fetal DNA fragments, first non-invasive methods to detect fetal T21 have been developed and described, as outlined in the international application WO 1998/039474. Herein, data are presented concerning the surprising identification of ncRNAs, in particular miRNAs present in maternal plasma that differentiate between DS fetuses and healthy pregnancies, and methods based on such differential expression for the early diagnosis of fetal DS. These findings suggest that cell-free extracellular ncRNAs such as miRNAs present a surprising potential new biomarker and cost-efficient alternative in NIPD.

Based on the experimental data provided herein, the present invention relates in general to a non-invasive method for determination of the genetic status of the fetus based on the analysis of a body fluid sample from a subject. Said method is used for evaluation whether the fetus has an increased or decreased number of specific chromosomes compared to a normal fetus for determination of genetic aberrations of the fetus, *e.g.,* chromosomal aberrations also known as aneuploidies, and/or in comparison to other fetuses for fetal gender evaluation. The analysis of a body fluid sample comprises detection and optionally quantification of one or more specific sequences of ncRNAs, such as miRNAs and or precursors thereof as defined in detail subsequently. The analysis step is performed preferably with molecules specifically binding to one or more sequences to be analyzed. Preferably, these molecules are nucleic acids, such as a probes or primers that are substantially complementary to the analyzed sequence. In one embodiment, the method of the present invention comprises analysis of at least two specific sequences, such as ncRNAs and/or precursors thereof.

In one embodiment, the ncRNAs and precursors thereof suitable for the method of the present invention are selected by the p-value calculated in respect of their differential expression values in comparison to the expression in a control sample, evaluated by, *e.g.,* NGS (Next Generation Sequencing) or real time PCR based methods as described herein, for example, in the Examples 1 and 2. In one embodiment, ncRNAs and precursors thereof selected as suitable for and used in the methods of the present invention have p-values of below or equal to 0.2, more preferably below or equal 0.15. In a preferred embodiment, the ncRNAs and precursors thereof selected as suitable for and used in the methods of the present invention have p-values of below or equal 0.1 and even more preferred below or equal to 0.05. Furthermore, in one preferred embodiment, calculation of p-values is performed in respect of raw and/or normalized Cqs (quantification cycles) and/or NRQs (normalized relative quantities). Preferably, Cqs are normalized by Global mean normalization (global mean of common targets; GN) or Quantile normalization (QN). In this concern, NRQs are preferably normalized by GN. Furthermore, the p-values are FDR (false discovery rate) adjusted [17-19]. Clustering and classification of data sets obtained by, e.g., NGS or HT-qPCR are preferably performed on raw, GN and QN Cqs and on raw and GN NRQs of data sets selected in agreement with the above identified criteria concerning the p-value, i.e. wherein the p-values of the measured expression levels of the respective ncRNAs and/or precursors thereof are below or equal the respective border p-values as indicated above.

Furthermore, the present invention provides data concerning specific ncRNAs, such as specific miRNAs, snRNAs and precursors thereof which, when analyzed by the method of the present invention, demonstrate, compared to a control sample, different or comparable levels of said specific ncRNAs and precursors thereof depending on whether the sample tested originates from a pregnant subject whose fetus has another or the same genetic status as the fetus from the female providing the control sample. Preferably, the asserted difference is constituted by a differing chromosomal total number or differing number of specific chromosomes, due to a differing fetal gender or to a chromosomal anomaly, *i.e.* aneuploidy. Evaluation of the specific ncRNAs and precursors thereof as defined herein below, is indicative therefore of a normal euploidy or the same fetal gender when a comparable level in the analyzed and control sample are determined, and indicative of an increased risk of the fetus for fetal chromosomal aneuploidy or of a different fetal gender, when a deviating, *i.e.* decreased or an increased level of the specific ncRNAs and precursors thereof is determined in the aforementioned samples.

Early detection of genetical aberrations such as chromosomal aneuploidy is critical for prospective parents and their decisions concerning the respective pregnancy. In this respect, in one embodiment the method of the present invention is provided, wherein the sample to be analyzed is obtained during early pregnancy, preferably within the first or up to the beginning of the second trimester. In a preferred embodiment, the sample to be analyzed is obtained during early pregnancy, preferably within the first trimester or up to about 12, 13, 14, 15 and most preferably up to 16 weeks after gestation. Furthermore, when determining fetal chromosomal aneuploidy, the present invention provides a method, wherein the chromosomal aneuploidy is Trisomy 21 (Down syndrome).

The present invention further relates to a variety of methods for determining the presence and/or amount of miRNA and/or precursors thereof, comprising amplification methods of the miRNA and/or precursor thereof, preferably by a polymerase chain reaction (PCR), more preferably by a quantitative PCR method such as real time PCR (abbreviated as qRT-PCR or RT-PCR and high throughput versions of such methods such as HT-qPCR); determination methods based on hybridization of the miRNA and/or precursor thereof, or of the products obtained by an aforementioned amplification method with a binding molecule specific for the miRNA and/or precursor thereof; and/or methods based on sequencing of the products of the aforementioned amplification or proliferation methods are provided in this respect as well.

In this respect, the present invention further provides aforementioned methods, wherein the analysis is performed on an affinity matrix comprising a solid support having bound at least one of the primers and/or probes mentioned herein above and below, and preferably comprising a wafer. Said affinity matrix may as well be a chip. Furthermore, the present invention provides devices and kits which are adapted for or useful in the non-invasive methods of the present invention, and it also relates to the use of one or more of the non-coding RNAs (ncRNAs) and/or precursors thereof as defined herein below for a non-invasive method of evaluation of a disorder associated with pregnancy and/or pathology, preferably wherein said disorder is DS and/or of the fetal gender.

### BRIEF DESCRIPTION OF THE DRAWINGS

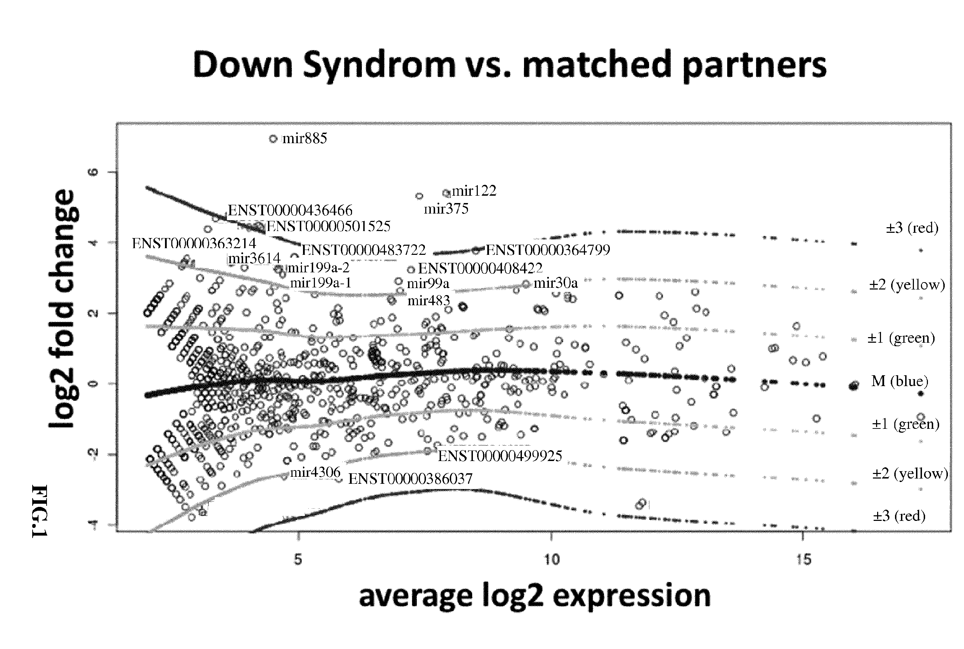

- **Figure 1:**: **NGS - Differential expression based on MA-plots.** The data for grouped miRNA were uploaded and mir-486 was excluded (high abundance miRNA found in all samples). Detection of differential expression was based on evaluation of MA plots via comparison of the T21 pool with the corresponding matched partner pool [samples were subsequently normalized to have a total intensity of 1*10⁶ (roughly similar to the total expression in the DS sample). To avoid zero expression (and infinite log values), pseudo-counts of 1 were inputted for miRNAs with zero reads associated. Standard deviation in M (log2 fold change) was fitted across A (average log2 expression). In the figure: the standard deviation of ±1 (green), ±2 (yellow) and ±3 (red) as well as the average M (blue) are shown. Based on the z-score, p-values and FDRs were calculated. Targets with a FDR < 0.05 are labeled. Using this approach, 28 potential biomarkers were identified.

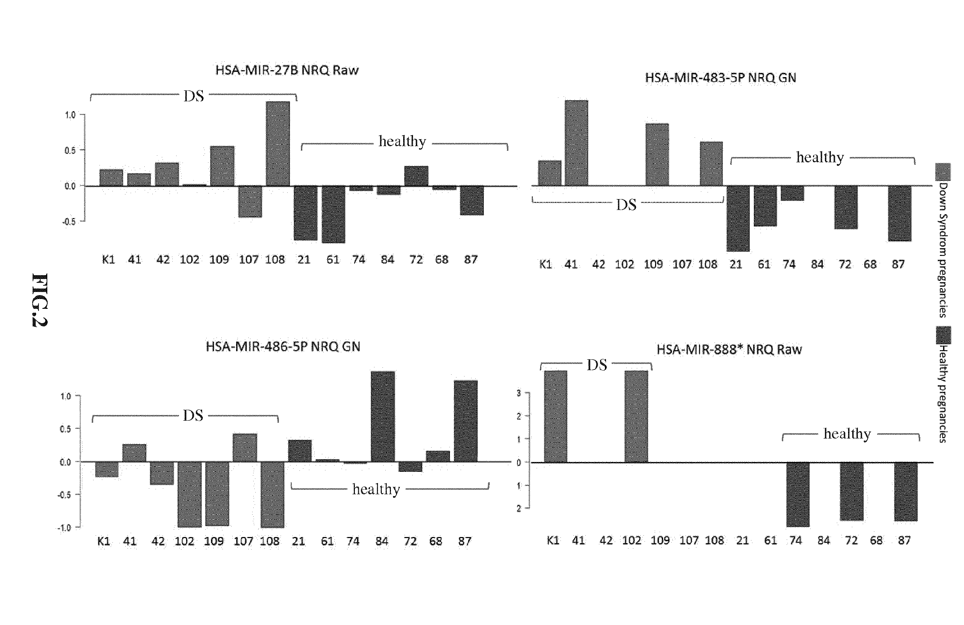

- **Figure 2:**: **Column plot of significantly differentially expressed miRNAs based on NRQs.** Column plots of selected miRNAs representing an expression profile between DS pregnancies versus healthy pregnancies. Although a particular trend of these miRNAs can be observed, a single miRNA that discriminated DS pregnancies from healthy pregnancies does not exist.

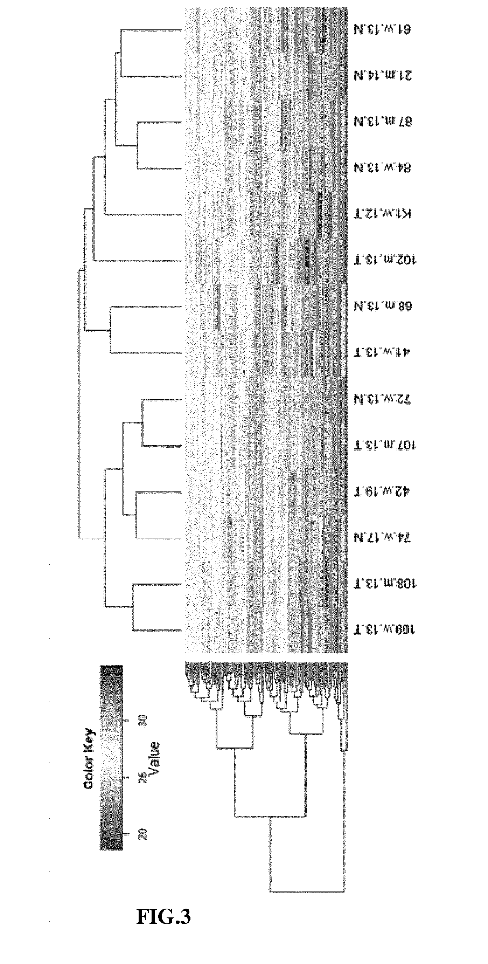

- **Figure 3:**: **Heat map cluster analysis of the full set of identified miRNAs.** Presented is the cluster analysis of quantile normalized (QN) Cqs. The Euclidian distance was used that is known to group genes with similar absolute Cqs together. If Euclidean distance is used, the cqs values are clustered based on their absolute value. This distinguishes in the heat map low and high cqs values (generating "bands" of similar microRNAs). The attribute T and N represents trisomic- and normal (healthy) pregnancies, respectively. As demonstrated, using the full set of identified miRNAs from plasma samples from pregnant women does not allow the differentiation between DS pregnancies and healthy pregnancies.

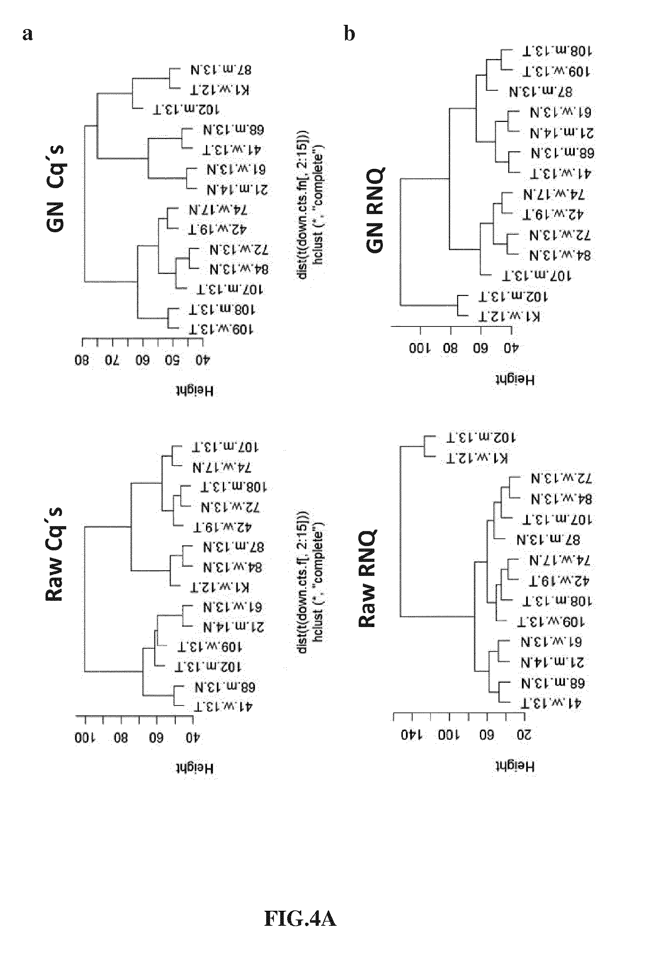

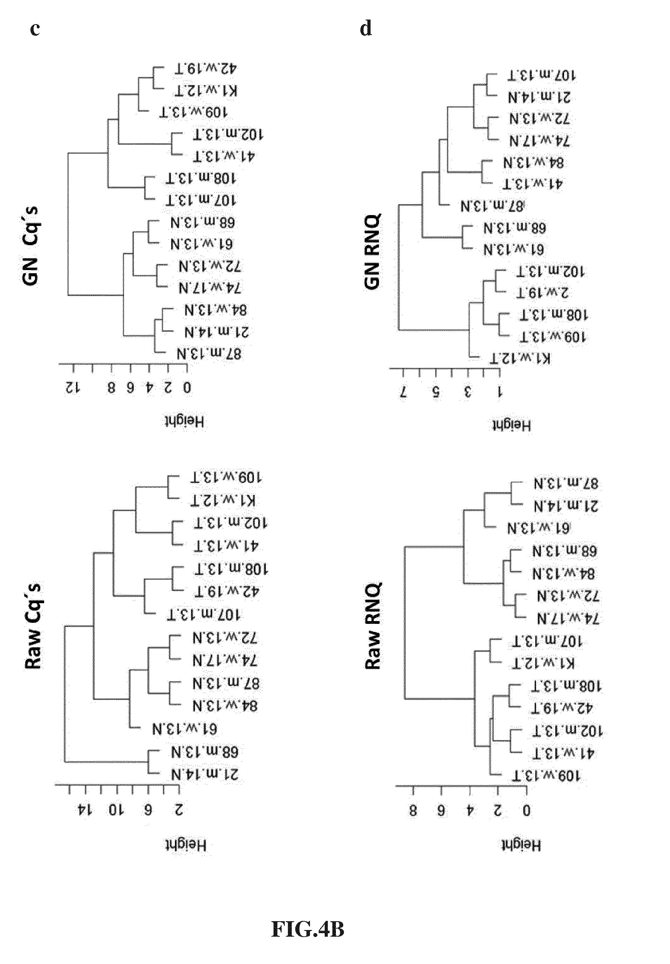

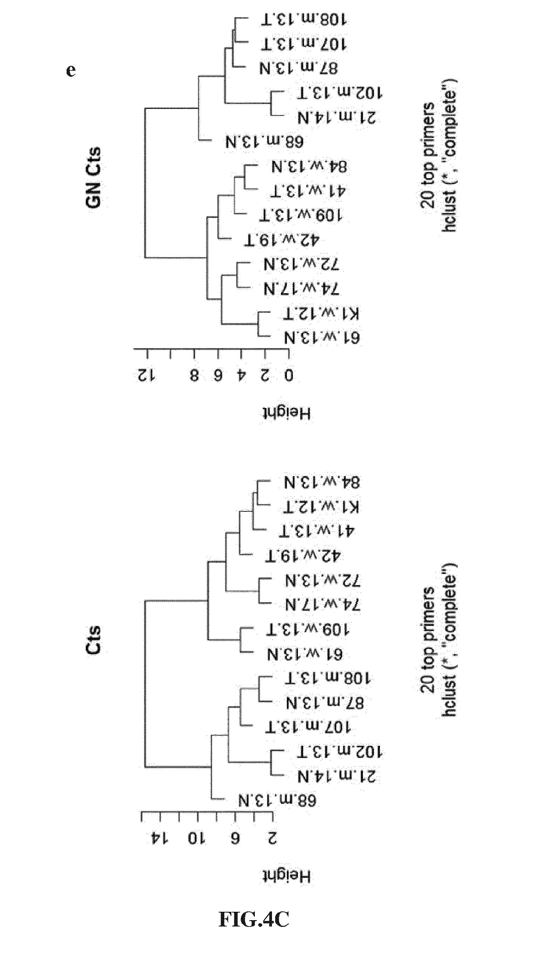

- **Figure 4:**: **Cluster analysis of subsets of miRNAs.** Clustering based on the Cqs and **(a)** and on the NRQ **(b)** of the full set **(4A)** and on subsets of most differentially expressed miRNAs **(4B).** The subset clustering was based on Cqs of the 20 **(4B-c)** and on the NRQ of the 10 most differentially expressed miRNAs **(4B-d).** Since it appears that a clear distinction between DS and normal samples cannot be achieved based on the full set of miRNAs **(4A)** or by single miRNA (due to NA values or lacking differential expression), clustering was repeated using a subset of miRNAs for which differential expression was observed. Represented in Fig. **4B****,** clustering based on the Cq's of the 20 most differentially expressed miRNAs **(4B-c)** and clustering based on the NRQ of the 10 most differentially expressed miRNAs **(4B-d)** was performed. The attribute T and N represents trisomic- and normal (healthy) pregnancies, respectively. As demonstrated here, by the integration of miRNAs with most differential expression, a clustering can be achieved that distinguishes DS from healthy pregnancies. Only GN NRQ failed to separate both groups from each another. This is a clear indication of the feasibility of such an approach for classification. Fig. **4C** shows a clustering of a subset of miRNAs to determine the fetal sex. Attributes "m" and "w" represent male- or female gender of the fetus respectively.

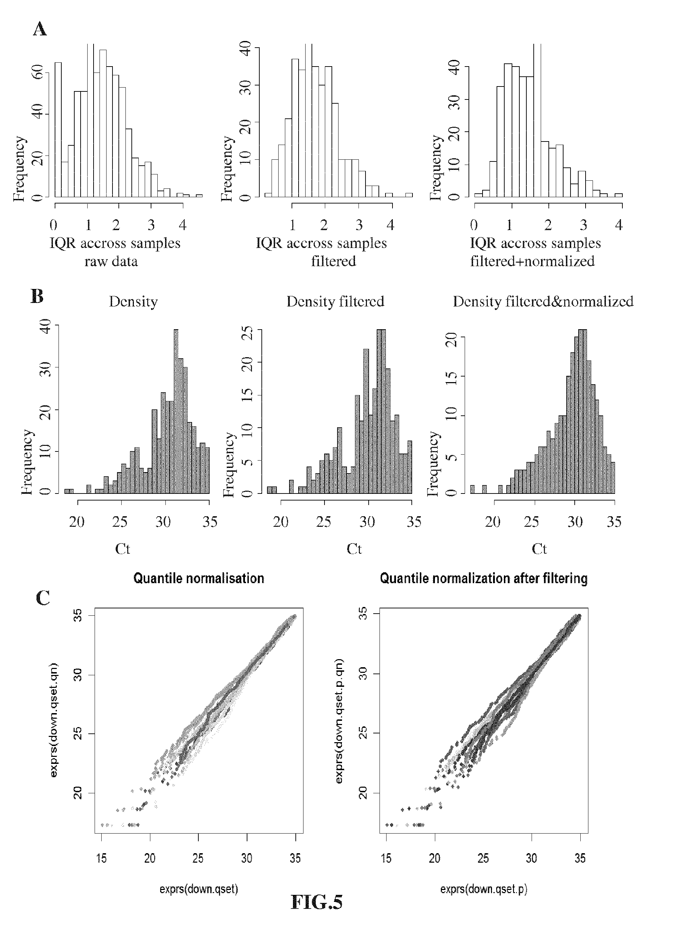

- **Figure 5:**: **The effect of filtering and normalization of the data.** Depicted are **(A):** inter quantile range (IQR) and **(B):** the distribution of Ct values. Filtering and normalization of the samples for further analysis did not significantly changed the the raw data, indicating a robust detection. **(C):** Correlation of the raw data after raw normalisation (RN) and quantile normalization (QN) before and after filtering. Normalization was performed on Cqs values directly (this is different from the NRQ values for which the relative quantities on linear scale were first calculated and then normalized). Using the HT-qPCR package, only the quantile normalization could be performed (as the other could not handle non available values). In general, RN and QN data correlated well.

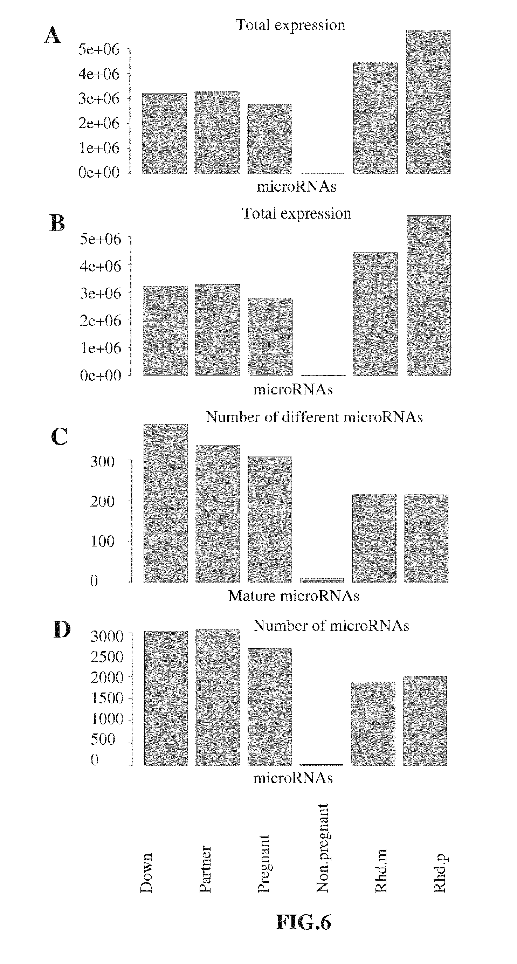

- **Figure 6:**: **Analysis of NGS data obtained for six different samples collected:** Down, Partner, Pregnant, Non-pregnant, RHD+ (Rhesus D) & RHD-. **(A)** Total expression for miRNAs (based on tables for grouped miRNAs). **(B)** Total expression for mature miRNAs. **(C)** Number of miRNAs (based on tables for grouped miRNAs) **(D)** Number of mature miRNAs

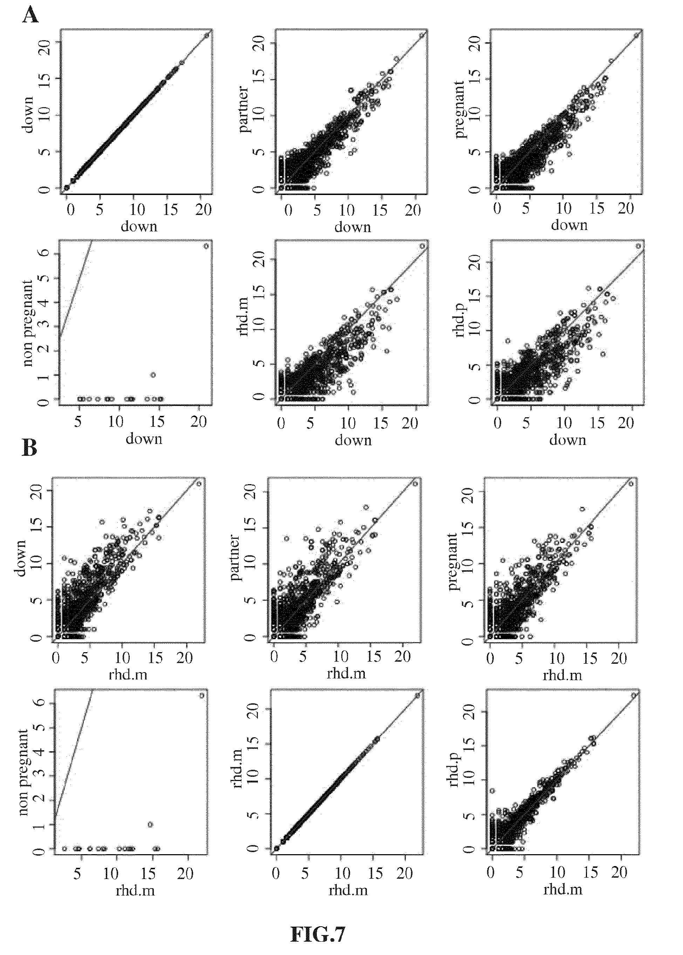

- **Figure 7:**: **Analysis of NGS data - overall comparison of expression.** The plots demonstrate the logged expression of the premature miRNA of DS vs. all, or respectively, of RHD-. The line represents equal expression. It is noticeable that expression in DS is more similar to expression in Partner and Pregnant, compared to RHD+ and RHD-. Comparison with the non-pregnant sample was not possible.

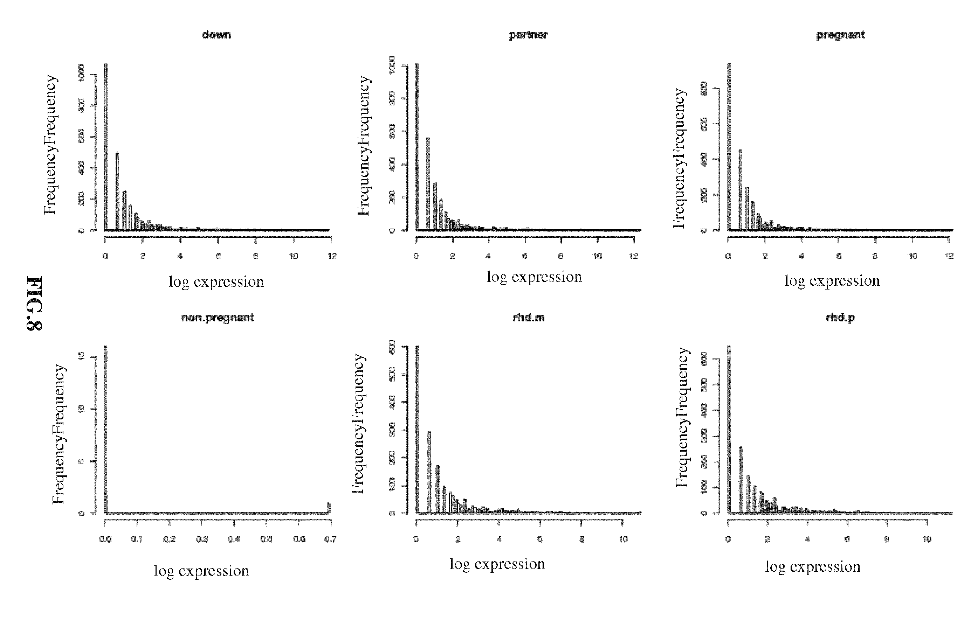

- **Figure 8:**: Analysis of NGS data: Distribution of expression values. Plots for premature miRNAs following exclusion of mir-486 demonstrate Poisson-like distributions.

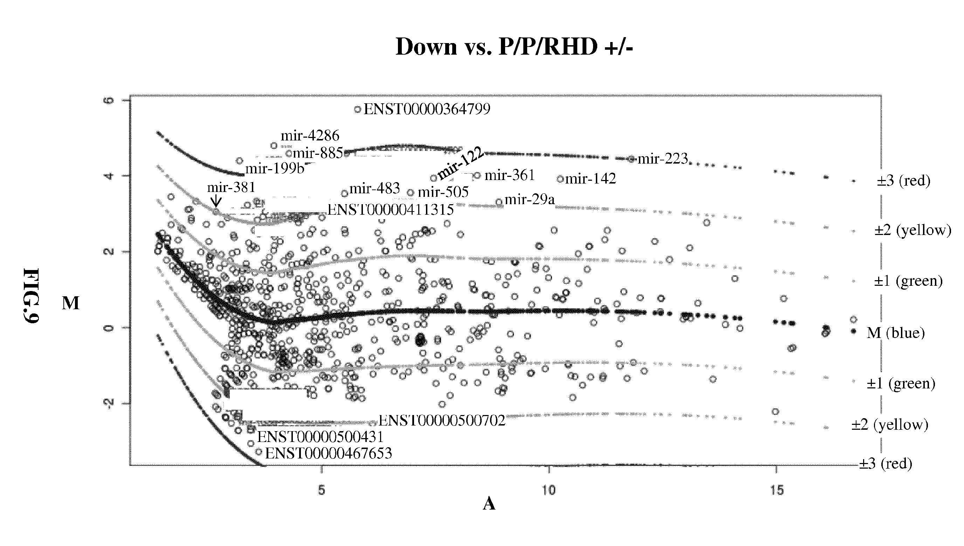

- **Figure 9:**: Analysis of NGS data: For the comparison between Down and Partner, 40 miRNAs have FDR < 0.05. Detected down-regulated miRNAs should be treated with caution, as the MA plot is not balanced for low A.

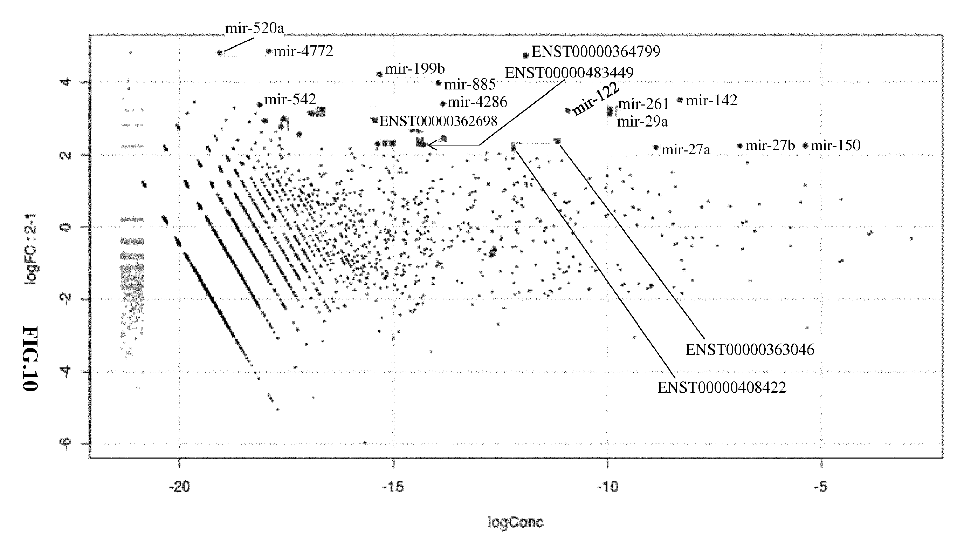

- **Figure 10:**: Analysis of NGS data/ EdgeR-analysis: Differential expression based on edgeR. Evaluation was also performed using a Bioconductor package dedicated to the analysis of digital expression. The package fits observed expression values with a negative binomial distribution and then uses a modified Fisher exact test to detect significant changes. Notably, the approach is originally designed for experiments with replicates and might deliver over-optimistic results thus. For the comparison Down and Partner, edgeR detects 355 miRNAs with FDR < 0.05.

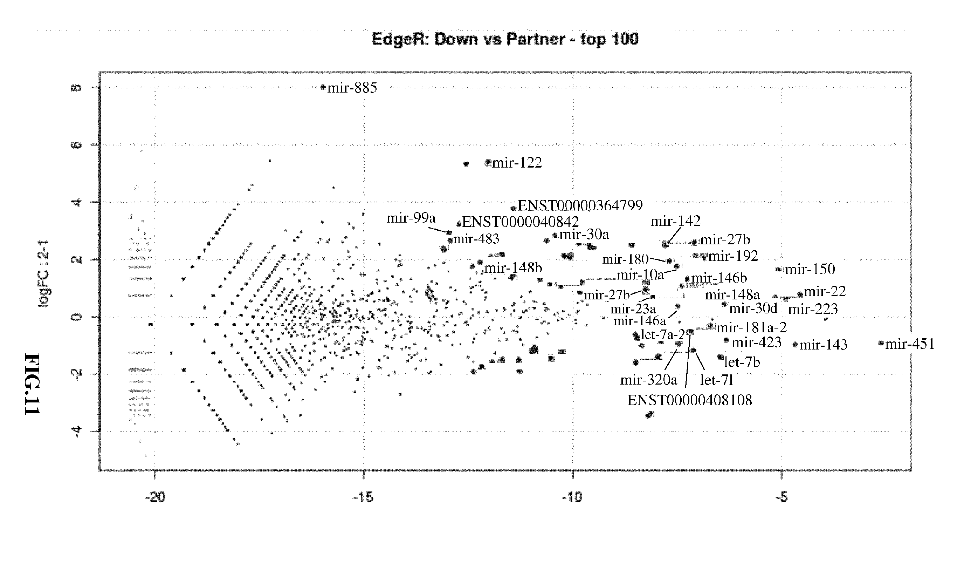

- **Figure 11:**: Analysis of NGS data/EdgeR-analysis: For the comparison Down and Partner/pregnant/RHD+/-, edgeR detects 126 miRNAs with FDR <0.05.

### DETAILED DESCRIPTION OF THE INVENTION

The demonstration of the presence of fetal nucleic acids in maternal peripheral blood has raised great interest in the field of non-invasive prenatal diagnostics (NIPD) [20-24], which has led to the development of a number of NIPD tests such as investigation of the fetal RhD status in RhD negative women [5, 25, 26], sex-linked diseases [27], and β-thalassemia [28]. Furthermore, quantitative aberrations of fetus-derived mRNA transcripts have been shown in conditions such as preeclampsia [29]. These findings suggest that the detection of circulating fetal nucleic acids holds much promise for NIPD [7, 30]. However, quantification by conventional real-time PCR of circulatory placental mRNA molecules is rather limited [31].

In recent years, there has been progress in NIPD of T21 research. The RNA-SNP allelic ratio test was developed by determining the ratio between polymorphic alleles of placenta-specific 4 (PLAC4) mRNA, a transcript on chromosome 21, in maternal plasma. This enabled direct detection of fetal chromosomal aneuploidies, but was limited only to the heterozygous fetus [32]. Digital PCR [31], NGS [33] and proteomics [34-37] have been applied for the NIPD of T21 varying in sensitivities, specificities though, and being often time-consuming and expensive. Analysis of fetal-specific differentially methylated regions (DMRs) for the NIPD of T21 in maternal peripheral blood has been recently described, demonstrating a high level of sensitivity and specificity [38]. This method relies on immunorecognition to detect and precipitate methylated DNA, which is then hybridized to a DNA array. However, this method is prone towards a sequence bias towards GC-poor regions. In addition, ligation-mediated PCR requires blunt-end ligation leading to poor efficiency, and due to its lower resolution (∼200bp), not allowing for an accurate single base characterization of methylation.

Experimental data provided within the present invention indicates ncRNAs and in particular miRNAs as a possible new class of molecular markers. MiRNAs are a family of small, 18-22 nucleotide long, non-protein-coding RNAs that have emerged as key post-transcriptional regulators of gene expression [30, 39]. Alterations in miRNA expression have been observed in several human pathologies [30] and miRNAs appear to play a fundamental role in diverse biologic and pathologic processes, such as cell proliferation, differentiation, apoptosis, animal development, carcinogenesis, cardiovascular disease, and primary muscular disorders [40-42].

To date, differential expression profiles of placental ncRNA between normal and DS pregnancies have not yet been investigated. NcRNA and, in particular, miRNA analysis may offer new possibilities for NIPD of chromosomal aneuploidies, particularly in light of the data provided by Kuhn *et al.* [43] indicating that five miRNAs localized on Chromosome 21 (miR-99a, let-7c, miR-125b-2, miR-155, and miR-802) are overexpressed in DS brain and heart specimens. International application WO 2009/025852 describes the use of miRNAs for determination of in vivo cell death, which may be associated with a diverse range of disorders such as pathogen or viral infections, brain stroke, Alzheimer's disease, Parkinson disease and DS of the fetus in particular via urine analyses. The method of the present invention, however, is in particular suitable for the specific diagnosis of DS of the fetus regardless of the occurrence or a change in cell death rates in the analyzed subject.

Within the present invention, a report is provided on the use of high-throughput technologies in the NIPD of T21 pregnancies. For the first time, the present invention presents data on a subset of identified ncRNAs in maternal plasma which allow for the differentiation between T21 and healthy pregnancies, which may have great impact on NIPD. Only one (miRNA-155) of the miRNAs examined by Kuhn *et al.* [43] in adult T21 patients has been observed to be differentially expressed within experimental data provided by the present invention. A possible explanation why the other miRNAs were not found is their possible involvement in later developmental stages, however, lack of their differential expression as revealed herein shows that there is no simple correlation between the presence of genes expressing particular miRNAs in triplicate and their applicability in diagnostics, e.g., in prenatal aneuploidy tests.

Therefore, the present invention generally relates to a non-invasive method for evaluation of fetal chromosomal aneuploidy and/or the fetal gender comprising analyzing a body fluid sample obtained from a subject for one or more specific sequences of cell-free non-coding RNAs (ncRNAs) and/or precursors thereof, wherein said analyzing comprises the step of detecting and optionally quantitating said ncRNAs and/or precursors thereof. Furthermore, in a preferred embodiment, the step of detecting and optionally quantitating said ncRNAs and/or precursors thereof is performed with a primer and/or probe that is substantially complementary to a part of the sequence of said specific ncRNA(s) and/or precursor(s) thereof. The present invention is illustrated with miRNAs but other ncRNAs such as snRNAs, e.g., RNU12, would be used as well.

The aforementioned determination methods may be performed via separate means for determination of any single task in respect of the genetic status of a fetus as defined above. In one embodiment however, the present invention also provides a method for combining the aforementioned determination methods, for determining aneuploidy, fetal gender and potentially further information concerning the genetic status of the fetus by analyzing a sample from a subject. Such a combined method may further reduce diagnostic costs and decrease efforts for the patient.

In one embodiment, the method of the present invention comprises analysis of at least 3, 4, 5, 6, 7, 8, 9, 10, 11, 12, 13, 14, 15, 16, 17, 18, 19, 20, 21-25, 26-30, 31-40, 41-50, 51-75, 76-100, 101-149, 150, 200, 250, 300, 350, 400, 450 or at least about 500 specific sequences of ncRNAs and/or precursors thereof. Furthermore in one preferred embodiment, the method of the present invention is provided, wherein at least two specific sequences of ncRNAs and/or precursors thereof are analyzed. In one embodiment of the present invention, the ncRNA is a microRNA (miRNA) or a small nuclear RNA (snRNA).

Due to the non-invasive nature of the method of the present inventions, biological samples to be analyzed by these methods may be taken without any risk for the developing fetus or its mother at any time during pregnancy. The nucleic acid may be isolated from the sample according to any of a number of methods well known to those of skill in the art, as described, e.g., in detail in Chapter 3 by Tijssen [45] or in the examples section in respect of ncRNAs and/or precursors thereof. One of such skills will appreciate that where expression levels of genes, ncRNAs and/or precursors thereof are to be detected; preferably RNA (mRNA, ncRNA such as miRNA, snRNA and/or precursors thereof) is isolated. Data obtained within the present invention and by others indicates ncRNAs such as miRNAs to be highly stable, making them an ideal candidate for diagnostic purposes[44].

Depending on the aim of the test, an analysis early in the pregnancy might be preferable, to determine whether the fetus demonstrates a normal chromosomal constitution or whether it shows any abnormality in the chromosome number. Therefore, in one embodiment of the present invention, the biological samples are obtained early in pregnancy. In one embodiment, the method of the present invention is provided, wherein the sample is obtained from a female within the first trimester or up to 16 weeks after gestation.

In a particularly preferred embodiment the method of the present invention as defined herein above and below is provided, wherein the chromosomal aneuploidy is Trisomy 21 (Down syndrome).

Expression of miRNAs and circulating pregnancy associated (fetal and maternal) miRNAs, including deregulated, disease-associated miRNAs, have been detected in human placenta, respectively in maternal blood, serum or plasma [30, 39, 46-50]. Biological samples which might be used by the methods of the present invention may be obtained from a body fluid obtained from a subject to be analyzed, such as amniotic fluid, urine, saliva and solutions thereof, blood, blood serum and blood plasma. In a preferred embodiment, the method of the present invention is provided, wherein the sample is a body fluid comprising blood, blood serum and/or blood plasma.

Experimental data obtained within the scope of the present invention indicates that detection and optionally quantitation of snRNAs, such as RNU12 might be used as well within the method of the present invention for determination of aneuploidy, in particular of T21 of a fetus as an additional factor evaluated together with the differential expression of specific ncRNAs and/or precursors thereof as defined herein above and below. Therefore, the method of the present invention encompasses the use of any method indicative in respect of the expression of specific RNA(s), ncRNAs such as miRNAs, precursors thereof and/or snRNAs such as RNU12, as defined herein, *e.g.,* in Table 12, which means any method may be used to determine qualitatively whether and/or quantitatively at which levels said nucleic acid is expressed. For example, methods capable of amplification of such nucleic acids may be used, such as PCR based methods, preferably quantitative PCR methods, such as RT-PCR methods. In one embodiment of the present invention, the amplified product will incorporate a means of detection such that the amplification may be detected and quantified.

In a preferred embodiment, the means of detection will be a label incorporated into one of the primers used to amplify the fragment or alternatively as a labeled nucleotide incorporated during amplification. It is envisioned that the label may be used to partially identify the sequence information of the amplified product. This label can include a chromophore, a fluorophore, an affinity label, an enzyme a metal chelate or a dye. Examples of suitable labels and methods or their incorporation into nucleic acid molecules are known to the skilled in the art and described, *e.g.,* in paragraph [0050] on pages 14-17 of the international application WO 2009/079215 A1, the disclosure content of which is hereby incorporated by reference; methods of their incorporation in Smith et al., Nature, 321:674-9 (1986); Agarawal et al., Nucleic Acids Res., 14:6227-45 (1986); Chu et al., Nucleic Acids Res., 16:3671-91 (1988).

The products of amplification may be labeled for analysis, but other means of analysis may be employed as well. Non-limiting examples of such analysis methods are: polyacrylamide gel electrophoresis, capillary gel electrophoresis, mass spectrophotometry, energy transfer, real-time PCR™, or the Biostar or Luminex technologies.

Analysis may also be performed to quantify the products, *e.g.,* in a multi-well plate, on a gel, on a membrane, or on a solid matrix. Such quantification may be facilitated by measuring the ratio of each amplified product to a co-amplified reference-gene, or by measuring the ratio of each amplified product to a panel of co-amplified reference-genes. Where the analysis takes place on a solid substrate, it is envisioned that the solid substrate may be a DNA chip.

The detection method used for profiling of ncRNAs such as miRNAs plays an important role on the outcome. The expression levels of RNAs, ncRNAs and/or precursors thereof are preferably measured by one of three methods within the present invention: real-time reverse transcription PCR (qPCR), microarray and massively parallel/NGS. However, NGS is expensive and it is not free from biases with most related to library preparation methods such RNA ligation and PCR amplification [51, 52]. Microarrays have high-throughput capabilities [53], however, this technique is not suitable for applications with low input materials, such as those derived from the human plasma sample. The Smart Chip Technology (WaferGen Biosystems, Inc; Fremont, USA) overcomes the aforementioned restrictions by allowing high throughput-quantitative PCR (HT-qPCR) profiling of 1100 miRNA simultaneously of one sample on one single chip with a high level of accuracy and reproducibility.

Furthermore, said specific RNA, ncRNA and/or precursors thereof in the analyzed sample or products of the above mentioned proliferation methods might be determined by the use of hybridization methods with molecules (probes) specifically binding these. Such binding molecules are preferably other nucleic acids. These nucleic acid molecules may be DNA or RNA; preferably the probes are single-stranded molecules. The method of preparing and labeling a probe to be hybridized with said nucleic acid molecule which has to be determined in the sample is not particularly limited insofar as the probe has a complementary sequence to the target sequence in said nucleic acid molecule; see, *e.g.,* methods for preparation of labeled DNA/RNA probes, and conditions for hybridization thereof to target nucleotide sequences as described in Chapters 10 and 11 of Molecular Cloning: A Laboratory Manual, [56]; the techniques described in U.S. Pat. No. 4,734,363; the nick translation method of Rigby et al, [54]; the random priming method of Fienberg et al. [55]; the method for attachment of fluorescent labels of Foster *et al.,* [58], see in particular the Materials and Methods section on pages 747-750, the entire disclosure content of the aforementioned documents are herein incorporated by reference. According to the present invention, the probes can be directly or indirectly labeled, *e.g.,* directly with a radioactive molecule or a fluorescent molecule, or indirectly by labeling the probe by a first molecule *(e.g.,* biotin, DIG, or an antibody) which is recognized by a further molecule, *e.g.,* a labeled antibody, or with colorimetric labels as the colored products of reactions catalyzed by enzymes such as beta-galactosidase, alkaline phosphatase, and horseradish peroxidase to allow detection of hybridization. Any label can be attached to the probe, which does not interfere substantially with the hybridization reaction, and generates a detectable signal. A hybridization method, which may be used for determination of ncRNAs and/or precursors thereof of the present invention, is the so-called "Northern" blotting technique; see, e.g., Chapter 7 of "Molecular Cloning: A Laboratory Manual" [56], the entire disclosure of which is incorporated by reference.

Therefore, in one embodiment the method of the present invention is provided, wherein analyzing the presence and/or amount of the ncRNAs and/or precursors thereof comprises:
(a) amplification of the ncRNA(s) and/or precursor(s) thereof by a polymerase chain reaction (PCR), preferably by a real time polymerase chain reaction (qRT-PCR) ;
(b) hybridization of the ncRNA(s) and/or precursor(s) thereof or of the product(s) obtained in (a) with one or more binding molecules specific for the ncRNAs and/or precursors thereof; and/or
(c) sequencing of the proliferation product(s) of (a).

Analysis of a sample in respect of presence, absence and/or amount of ncRNAs and/or precursors thereof comprised therein, or of amplification products thereof can be performed in different experimental environments. In one embodiment, the quantification is performed by measuring the ratio of each amplified product to a co-amplified reference gene/ncRNA or precursor thereof. In one embodiment, the quantification is performed by measuring the ratio of each amplified product to a panel of co-amplified reference-genes. In a preferred embodiment, the analysis of amplification products is performed by real-time PCR. In accordance to the present invention, DNA amplification and/or the analysis of amplification products for determination and/or quantification of specific products, can be performed in a multi-well plate, on a membrane or preferably on a solid matrix. In one preferred embodiment, the solid matrix is a DNA chip [59-62].

A biochip in general might be defined as a solid surface preferably, but not limited to the form of a planar slice. Preferred materials for the planar slice, or for the surface of a biochip in general are glass or a silicon oxide layer formed on a silicon wafer. The surface of a biochip may be chemically modified (*e*.*g*., with poly(L-lysine), for immobilization of molecules designed for specific binding of the RNAs, ncRNAs and/or precursors thereof such as primers and/or probes nucleic acids on the surface. These molecules may be spotted via contact printing using robotic pins or other equivalent delivering technologies such as inkjet printing [63-65] onto the surface of a biochip and be used within the methods of the present invention.

Therefore, in one embodiment, the method of the present invention is provided, wherein analyzing the presence and/or amount of the ncRNAs and/or precursors thereof is performed on an affinity matrix comprising a solid support having bound at least one primer and/or probe. Preferably said primers and/or probes are selected from those primers and/or probes mentioned hereinabove and below. Furthermore, in one embodiment the method of the present invention is provided, wherein the solid support comprises a wafer.

Experimental data provided herein (see the Examples section) indicates for the first time ncRNAs and precursors thereof, which are differentially expressed in pregnant women depending on the particular genetic status of the fetus. In one embodiment the method of the present invention is provided, wherein the miRNAs are selected from the group indicated in Table 12 consisting of: hsa-miR-589-3P, HSA-MIR-512-5P, hsa-miR-181a-3p, HSA-MIR-483-5P, HSA-MIR-362-5P, HSA-MIR-518c-5P, HSA-MIR-3667-5P, HSA-MIR-195-3P, HSA-MIR-124-5P, HSA-MIR-498, HSA-MIR-18B-3P, HSA-MIR-888-3P, HSA-MIR-3676-3P, HSA-MIR-3918, HSA-MIR-500A-5P, HSA-MIR-30C-5P, HSA-MIR-200C-3P, HSA-MIR-627, HSA-MIR-93-5P, HSA-MIR-20A-5P, HSA-MIR-4328, HSA-MIR-92a-1-5P, HSA-MIR-92b-3P, HSA-MIR-3652, HSA-MIR-25-3P, HSA-MIR-623, HSA-MIR-3195, HSA-MIR-423-5P, HSA-MIR-27B-3P, HSA-MIR-3135a, HSA-MIR-486-5P, HSA-MIR-3180-5P, HSA-MIR-629-5P, HSA-MIR-629-3P, HSA-MIR-155-5P, HSA-MIR-3907, HSA-MIR-1249, HSA-MIR-215, HSA-MIR-1227-3P, HSA-MIR-187-3P, HSA-MIR-219-5P, HSA-MIR-3913-5P, HSA-MIR-590-3P, HSA-MIR-3923, HSA-MIR-1228-5P, HSA-MIR-769-3P, HSA-MIR-127-5P, HSA-MIR-1293, HSA-LET-7f-1-3p, HSA-MIR-93-3P, HSA-MIR-892B, HSA-MIR-320B, HSA-MIR-106a-3P, HSA-MIR-92B-5P, HSA-MIR-328, HSA-MIR-196B-3P, HSA-MIR-548C-5P, HSA-MIR-512-3P, HSA-MIR-4306, HSA-MIR-29B-1-5P, HSA-MIR-30C-1-3P, HSA-MIR-199b-5P, HSA-MIR-855-3P, HSA-MIR-855-5P, HSA-MIR-199-5P, HSA-MIR-199-3P, HSA-MIR-122-5P, HSA-MIR-483-3P and having the SEQ ID NOs: 1-15, 17-62 and 75-81; the precursors thereof are selected from the group of precursors as indicated in Table 12 having SEQ ID NOs: 82-153 and 172-178; and the snRNA is RNU12 as indicated in Table 12 having SEQ ID NO: 16, wherein compared to a control sample or control fetus:
(a) a comparable level of the ncRNAs and/or precursors thereof is indicative of a normal euploidy and/or of the same fetal gender of the fetus; and
(b) a decreased or an increased level of the ncRNAs and/or precursors thereof is indicative of an increased risk of the fetus for fetal chromosomal aneuploidy and/or of different fetal gender than the control fetus.

In one embodiment, the method of the present invention is provided for determining fetal gender, wherein the miRNAs are selected from the group indicated in Table 12 consisting of: HSA-MIR-590-3P, HSA-MIR-3923, HSA-MIR-1228-5P, HSA-MIR-769-3P, HSA-MIR-127-5P, HSA-MIR-1293, HSA-LET-7f-1-3p, HSA-MIR-93-3P, HSA-MIR-892B, HSA-MIR-320B, HSA-MIR-106a-3P, HSA-MIR-92B-5P, HSA-MIR-328, HSA-MIR-196B-3P, HSA-MIR-548C-5P, HSA-MIR-512-3P, HSA-MIR-4306, HSA-MIR-29B-1-5P, HSA-MIR-30C-1-3P having SEQ ID NOs.: 44-62 and the precursors thereof are selected from the group of precursors as indicated in Table 12 having SEQ ID NOs.: 133-153, wherein compared to a control sample from a pregnant female:
(a) a comparable level of the one or more miRNAs and/or miRNA precursors thereof is indicative same fetal gender of the fetus; and
(b) a decreased or increased level of the one or more miRNAs and/or miRNA precursors thereof is indicative of different fetal gender than the control fetus.

In one embodiment the above method is provided for determining fetal aneuploidy and/or gender, wherein the expression of control miRNAs and/or precursors thereof is determined in the analyzed sample and in the control sample in addition to the miRNAs and/or precursors thereof indicated above. Furthermore, in one embodiment, the control miRNAs are selected from the group "Reference-miRNAs" and/or "not in plasma control" indicated in Table 12 consisting of: HSA-MIR-101-3P, HSA-MIR-103a-3P, HSA-MIR-16-5P, HSA-MIR-185-5P, HSA-MIR-223-3P, HSA-MIR-378-5P, HSA-MIR-378-3P, HSA-MIR-451a, HSA-LET-7A-5P, HSA-MIR-1206, HSA-MIR-132-3P, HSA-MIR-153 having SEQ ID NOs.: 63-74 and the precursors thereof are selected from the group of precursors as indicated in Table 12 having SEQ ID NOs.: 154-171. In one embodiment the evaluation of the control miRNAs and/or precursors thereof is used for determination of hybridization stringency conditions and/or for determination of non-specific hybridization. Further details concerning such use are described further below in connection with the arrays and biochips of the present invention.

In addition, the present invention also relates to the use of a primer or probe comprising a nucleic acid binding molecule or an affinity matrix comprising at least one said primer or probe in a method of the present invention as defined herein.

In one embodiment, the present invention also relates to the method or the use of the present invention as defined hereinabove and below, wherein the affinity matrix is a chip.

Preferably, the methods of the present invention are performed by the use of a device or devices designed, constructed and/or adapted in a way enabling for execution of the non-invasive methods of the present invention. Said device may comprise several units, or it may be split into several devices of different tasks. In general, the tasks to be performed by the device(s) may be defined as analysis and evaluation. Sais task(s) of analysis comprises determination of the presence and/or of the amount(s) of the RNAs, ncRNAs and/or precursors thereof, wherein the device unit or devices intended or build for analysis preferably comprise(s) one or more of detection agents for the RNAs, ncRNAs and/or precursors thereof as defined herein. The task of evaluation to be performed by a device or devices of the present invention comprises executing a comparison of the determined amounts obtained from the analyzing unit with the amounts obtained from an analysis of a control sample. Preferably, the one or more evaluation devices or units comprise a computer, wherein the computer tangibly comprises embedded one or more computer program(s) for carrying out the comparison. The tasks may be performed by a device comprising several units adapted therefore, or may be performed as well by several devices, interconnected or capable of exchanging data obtained during the analysis and/or evaluation, *e.g.,* via connecting wires, wireless and/or via data carriers.

In one preferred embodiment, the device of the present invention is adapted for a method of non-invasive evaluation of chromosomal aneuploidy in a subject comprising:
(a) an analyzing unit comprising a detection agent for the at least one ncRNA molecule and/or precursor thereof as defined herein, wherein said analyzing unit is adapted for determining the amount(s) of said at least one ncRNA molecule and/or precursor thereof in a sample detected by the detection agent; and
(b) an evaluation unit comprising a computer comprising tangibly embedded a computer program code for carrying out a comparison of the determined amounts obtained from the analyzing unit.

The present invention also relates to kits comprising devices and/or materials useful in or adapted for a method of the present invention, such as one or more nucleic acid(s) of the invention and/or sequences complementary thereto together with any or all of the following: assay reagents, buffers, probes and/or primers, and sterile saline or another pharmaceutically acceptable carrier. In addition, the kits may include instructional materials containing directions (e.g. protocols) for the practice of the methods of this invention.

In one embodiment of the present invention thus, a kit adapted for a method of non-invasive evaluation of chromosomal aneuploidy in a subject is provided comprising:
(a) a detection agent for at least one of the ncRNA molecules and/or precursors thereof as defined hereinabove and below, preferably wherein the detection agent is or comprises a primer and/or probe or an affinity matrix as defined hereinabove;
(b) a reverse transcriptase, a DNA polymerase, deoxynucleoside triphosphates (dNTPs), a Mg²⁺-ion solution, one or more primers and/or a reaction buffer; optionally
(c) a label specifically binding to a chimeric molecule consisting of the primer or probe as defined in (a) bound to the ncRNA or precursor thereof as defined hereinabove and/or to an amplification product of a PCR and/or an RT-qPCR as defined hereinabove;
(d) a device adapted for determining the amount at least one of the ncRNA molecules and/or precursors thereof detected by said detection agent, wherein said device is also adapted for carrying out the comparison of said amounts as defined before; and optionally
(e) instructions for use in the method of the present invention as defined hereinabove and below and/or a control sample or reference value thereof.

Preferably, one or more reference expression profiles are also provided with the kit of the present invention which show the expression profile of an identical set of miRNAs in the same type of biological sample, in particular in a blood and/or serum sample, obtained from one or more control subjects which are, e.g., pregnant subjects with a known genetic status of the fetus such as a pregnant woman with a healthy fetus, with a fetus with T21 and/or with a fetus of known gender. A comparison of the expression profile obtained according to the methods of the present invention to the reference expression profile(s) allows for the determination of the genetic status of the fetus, i.e. fetal T21 and/or of fetal gender. Furthermore, in one embodiment the kit of the present invention preferably comprises enzymes and reagents including reagents for cDNA synthesis from ncRNAs and/or precursors thereof prior to realtime PCR.

Moreover, the present invention also relates to the use of one or more of the ncRNAs and/or precursors thereof as defined herein above and below (see for example Table 12) for a non-invasive method of evaluation of a pregnancy-related disorder and/or pathology, preferably wherein the disorder is Down syndrome. In this context, in one embodiment the present invention relates to a method for determining fetal aneuploidy and/or gender using a miRNA precursor comprising the nucleic acid sequence of the miRNA as defined hereinbefore. The sequences of the respective miRNA precursors of the miRNAs indicated in Table 12 can, if not indicated already herein, be obtained from the respective entries in miRBase [71], available online at www.mirbase.org.

As indicated above, the presence and/or amount of particular types of these ncRNAs within a sample can be used for the estimation of the risk of aneuploidy and/or of the gender of the fetus. In one embodiment of the present invention, the method to diagnose as defined above is provided, wherein a comparable or increased level of one or more or of the at least two ncRNA and/or precursors thereof as enlisted in Table 12 compared to a control sample is indicative of a normal or increased risk of fetal aneuploidy.

### Definitions and embodiments:

It is to be noted that the term "a" or "an" entity refers to one or more of that entity; for example, "an polypeptide," is understood to represent one or more polypeptides. As such, the terms "a" (or "an"), "one or more," and "at least one" can be used interchangeably herein.

Unless stated otherwise, the terms "disorder" and "disease" are used interchangeably herein. By "subject", "individual", "animal", "patient" or "mammal" is meant any subject, particularly a mammalian subject, e.g., a human patient, for whom diagnosis, prognosis, prevention, or therapy is desired.

In general, the term "non-coding RNA" (ncRNA) as used in the present invention relates to RNAs fulfilling relatively generic functions in cells, such as the rRNAs and tRNAs involved in mRNA translation, small nuclear RNAs (snRNAs) involved in splicing and small nucleolar RNAs (snoRNAs) involved in the modification of rRNAs but also to RNAs involved gene regulation, such as microRNAs (miRNAs) and small interfering RNAs (siRNAs). Primarily however, the term ncRNAs is used herein in relation to miRNAs and snRNAs.

The term "microRNA" (miRNA) as used herein relates to a non-protein coding RNA, which may comprise 13-35, 18-25 or 21-24 nucleotides, generally of between approximately 19 to 25 nucleotides, preferably of about 18-22 nucleotides as the majority of the naturally occurring miRNAs, that negatively regulates expression of various genes [67-70]. For a recent review of miRNA biogenesis, see [66]. The primary transcript is termed a "pri-miRNA", a can be up to several kilobases long. This pri-miRNA is "cropped" by the nuclear RNase III Drosha to produce a shorter (∼70 nucleotides) miRNA precursor molecule known as a "pre-miRNA". Thereafter, pre-miRNAs are exported to the nucleus where the enzyme Dicer generates the short, mature miRNAs [73-76]. MiRNAs can thus be described in terms of RNA *(e.g.,* RNA sequence of a mature miRNA or a miRNA precursor molecule), or in terms of DNA *(e.g.,* DNA sequence corresponding to a mature miRNA, RNA sequence or DNA sequence encoding a MIR gene or fragment of a MIR gene or a miRNA precursor). The term "miRNA precursor" as used herein refers interchangeably to all precursor forms of a mature miRNA, *i.e.* pri-miRNA and pre-miRNA.

As a miRNA of the present invention, a miRNA consisting of a nucleotide sequence having an identity of 80% or more to the nucleotide sequence of any one of SEQ ID NOs: 1 to 15 and 17 to 81, preferably a miRNA consisting of a nucleotide sequence having an identity of 90% or more, more preferably 95% or more, can be mentioned and referred to as a variant thereof.

As a precursor miRNA of the present invention, a precursor miRNA consisting of a nucleotide sequence having an identity of 80% or more to the nucleotide sequence of any one precursor miRNA of SEQ ID NOs: 82-178 as indicated in miRBase within the entries of the respective miRNA molecules of the present invention indicated in Table 12 and of SEQ ID NOs: 82-178, preferably a miRNA consisting of a nucleotide sequence having an identity of 90% or more, more preferably 95% or more, can be mentioned and referred to as a variant thereof.

The term "snRNA" as used in the present invention refers to RNAs which are components of small nuclear ribonucleoprotein complexes (snRNPs) which, when assembled with additional proteins, form the large ribonucleoprotein complex known as the splicesome [85-86]. As a snRNA of the present invention, a snRNA consisting of a nucleotide sequence having an identity of 80% or more to the nucleotide sequence of SEQ ID. NO 16, preferably a snRNA consisting of a nucleotide sequence having an identity of 90% or more, more preferably 95% or more, can be mentioned and referred to as a variant thereof.

The variant may also be a nucleotide sequence which hybridizes under stringent conditions to the referenced nucleotide sequence, complements thereof, or nucleotide sequences substantially identical thereto. The depiction of a single-strand also defines the sequence of the complementary strand. Thus, a nucleic acid also encompasses the complementary strand of a depicted single-strand. Many variants of a nucleic acid may be used for the same purpose as a given nucleic acid. Thus, a nucleic acid also encompasses substantially identical nucleic acids and complements thereof. Furthermore, a single-strand provides a probe for a probe that may hybridize to the target sequence under stringent hybridization conditions. Thus, a nucleic acid also encompasses a probe that hybridizes under stringent hybridization conditions.

The term "snoRNA" as used in the present invention refers to a class of small RNA molecules that guide chemical modifications (methylation or pseudouridylation) of ribosomal RNAs (rRNAs) and other RNA genes (tRNAs and other small nuclear RNAs (snRNAs)). They are classified under snRNA in MeSH.

"Probe" as used herein refers to any oligonucleotide capable of binding to a target nucleic acid of complementary sequence through one or more types of chemical bonds, usually through complementary base pairing, usually through hydrogen bond formation. Probes may bind target sequences lacking complete complementarity with the probe sequence depending upon the stringency of the hybridization conditions. There may be any number of base pair mismatches which will interfere with hybridization between the target sequence and the single stranded nucleic acids of the present invention. However, if the number of mutations is so great that no hybridization can occur even under the least stringent of hybridization conditions, the sequence is not a complementary target sequence. As indicated beforehand, probes may be modified by attachment of, *e.g.,* labels and/or functional groups allowing cross-linking of the probes to a solid support, such as a blot or the surface of a biochip. Furthermore, probes may also be modified oligonucleotides such as locked nucleic acid (LNA) oligonucleotides with the LNA modification potentially resulting in enhanced hybridization properties [96].

"Stringent hybridization conditions" as used herein may mean conditions under which a first nucleic acid sequence *(e.g.,* probe) will hybridize to a second nucleic acid sequence *(e.g.,* target), such as in a complex mixture of nucleic acids, but to no other sequences. Stringent conditions are sequence- and circumstances-dependent. Generally, stringent conditions are selected (depending on the probe composition) to be about 5-24° C lower than the thermal melting point (Tm) for the specific sequence at a defined ionic strength pH. Stringent conditions may be those in which the salt concentration is less than about 1.0 M sodium ion, typically about 0.01-1.0 M sodium ion concentration (or other salts) at pH 7.0 to 8.3 and the temperature is at least approximately 30° C for short probes *(e.g.,* about 10-50 nucleotides) and at least about 60° C for long probes *(e.g.,* greater than about 50 nucleotides). For selective or specific hybridization, a positive signal may be at least 2 to 10 times the background hybridization. Exemplary stringent hybridization conditions include the following: 50% formamide, 5×SSC, and 1% SDS, incubating at 42° C., or, 5×SSC, 1% SDS, incubating at 65° C., with wash in 0.1-0.2×SSC, and 0.1% SDS at 65° C. Methods for probe design and hybridization conditions are known in the art and may be are performed as described, *e.g.,* in the "RNA labeling and hybridization", "Probe and microarray design" and "Tm determination" sections of Wang et al., RNA. 2007 13(1): 151-159, the disclosure content of which is herewith incorporated by reference in its entirety.

"Substantially complementary" used herein may mean that a first sequence is at least 60%, 65%, 70%, 75%, 80%, 85%, 90%, 95%, 97%, 98% or 99% identical to the complement of a second sequence over a region of 8, 9, 10, 11, 12, 13, 14, 15, 16, 17, 18, 19, 20, 21, 22, 23, 24, 25, 30, 35, 40, 45, 50 or more nucleotides, or that the two sequences hybridize under stringent hybridization conditions.

"Substantially identical" used herein may mean that a first and second sequence are at least 60%, 65%, 70%, 75%, 80%, 85%, 90%, 95%, 97%, 98% or 99% identical over a region of 8, 9, 10, 11, 12, 13, 14, 15, 16, 17, 18, 19, 20, 21, 22, 23, 24, 25, 30, 35, 40, 45, 50 or more nucleotides or amino acids, or with respect to nucleic acids, if the first sequence is substantially complementary to the complement of the second sequence.

As used herein, the term "binding molecule" refers to any agent *(e.g.,* peptide, protein, nucleic acid polymer, aptamer, spiegelmer or small molecule) that specifically binds to a target of interest. In a particular preferred embodiment, the term "binding molecule" in the sense of the present invention includes RNA-molecules, antibodies, aptamers (see, e.g., [87, 88]), spiegelmers (see, e.g. WO 1998/008856), and modified variants of small interfering RNA (siRNA), miRNA, single strand DNA molecules or a variant thereof, these modifications, e.g., helping protect the miRNA-related molecule from degradation, or being a label. The "target of interest" is herein defined as the region within the ncRNA molecules or precursors thereof of the present invention, which is sufficient for specific binding of a primer, thereby allowing the amplification of said ncRNA molecule or precursor thereof or specific hybridization of a probe allowing the detection of said ncRNA or precursor thereof.

"Antibodies" are generated by state of the art procedures, *e.g.,* as described in Tijssen, Practice and theory of enzyme immunoassays, 11, Elsevier Science Publishers B.V., Amsterdam, particularly on pages 43-78. The antibody of the present invention may exist in a variety of forms that contain an antigen binding site that specifically binds an antigen besides complete antibodies; including, for example, antigen-binding fragments as well as a single chains; see, *e.g.,* international applications WO88/09344, WO 2005/003169, WO 2005/003170 and WO 2005/003171.

The term "polynucleotide" is intended to encompass a singular nucleic acid as well as plural nucleic acids, and refers to an isolated nucleic acid molecule or construct, *e.g.,* messenger RNA (mRNA) or plasmid DNA (pDNA). The term "nucleic acid" refers to any one or more nucleic acid segments, *e.g.,* DNA or RNA fragments, present in a polynucleotide, comprising preferably the sequence encoding at least one ncRNA molecule of the present invention. Herein, as an "isolated" nucleic acid or polynucleotide a nucleic acid molecule is considered, which has been removed from its native environment such as a recombinant polynucleotide encoding at least one ncRNA or precursor thereof or the sequence complementary thereto contained in a vector, and recombinant polynucleotides maintained in heterologous host cells or purified (partially or substantially) polynucleotides in solution. Isolated nucleic acid molecules include *in vivo* or *in vitro* RNA transcripts and synthetically produced polynucleotides of the present invention, complementary sequences thereof and/or single strand DNA transcripts thereof. Isolated nucleic acid molecules may be or may include a regulatory element such as a promoter, ribosome binding site, or a transcription terminator.

In certain embodiments, the polynucleotide or nucleic acid comprising a nucleic acid which encodes a desired molecule, such as a RNA, ncRNA, a precursor thereof, a sequence complementary to any of the aforementioned or fragments thereof, normally may include a promoter and/or other transcription or translation control elements associated with one or more coding regions in such a way that induction of promoter function results in the transcription of the desired aforementioned molecule. In a particularly preferred embodiment, a polynucleotide of the present invention is RNA, for example, in the form of mRNA, miRNA, miRNA precursor, snRNA, small hairpin RNA (shRNA), siRNA, a RNA complementary to any of the aforementioned or any other RNA product.

"Similarity" between two peptides is determined by comparing the amino acid sequence of one peptide to the sequence of a second peptide. An amino acid of one peptide is similar to the corresponding amino acid of a second peptide if it is identical or a conservative amino acid substitution; see, *e.g.,* Dayhoff, M.O., ed., The Atlas of Protein Sequence and Structure 5, National Biomedical Research Foundation, Washington, D.C. (1978), and Argos et al. [89].

The determination of percent identity or similarity between two sequences is preferably accomplished using the mathematical algorithm of [90]. Such an algorithm is incorporated into the BLASTn and BLASTp programs of Altschul *et al.* [91] available at NCBI (http://www.ncbi.nlm.nih.gov/blastBlast.cge). When performing the algorithms, standard parameters of the BLASTn and BLASTp programs can be used or modified if necessary, as recommended on the NCBI webpage and in the "BLAST Program Selection Guide", in respect of sequences of a specific length and composition.

Within the scope of the present invention, a method is regarded as "non-invasive" if no intrusive method involving the usual operational risk is required for the collection of the biological sample. The term "biological sample", as used herein, refers to a sample obtained from an organism or from components (e.g., cells) of an organism. The sample may be of any biological fluid. Frequently, the sample will be a "clinical sample" which is a sample derived from a patient. The methods of the present invention require only the withdrawal of a small amount of venous blood from the patient, which is regarded as non-invasive. During amniocentesis (also referred to as amniotic fluid examination or analysis), a hollow needle is inserted through the mother's abdominal wall, then through the wall of the uterus, and finally into the amniotic sac to obtain cells of the child/fetus. In rare cases, the cells or tissue are taken directly from the fetus. Such methods are classified in obstetrics and herein as invasive.

The human genome consists of 23 pairs of chromosomes. Numeral aberrations thereof are defined as "aneuploidies". Depending on the aberration type, aneuploidies are further classified as a trisomy, if a chromosome is present in triplicate or as a monosomy, if only one copy of a chromosome is present. Chromosomal aneuploidy is a main cause of human prenatal and postnatal morbidity and mortality, and the most frequent genetic cause of mental retardation. The most aneuploidy among live-born babies is T21, phenotypically manifested as DS [1-3]. The incidence of 1 of 700 live births increases with advanced maternal age [3].

The term "quantitative-PCR", "qt-PCR" and "real-time PCR" are used interchangeably herein to refer both the qualitative and quantitative determination of nucleic acid sequences by a method using multiplying DNA/RNA sequences. During qt-PCR, an increase of fluorescence is measured and no further methods for read-out are required. The result is monitored in real time or after finalization of the PCR with the assistance of adequate computer software. For further information concerning see, e.g., Wittwer *et al.,* [92].

Qt-PCR involves the measurement of the amount of amplicon as a function of amplification cycle, and using this information to determine the amount of the nucleic acid sequence corresponding to the amplicon that was present in the original sample. The qt-PCR reaction is carried out with a pair of primers designed to amplify a given nucleic acid sequence in a sample and subjected to a number of amplification cycles. Initially, the amount of amplicon remains at or below background levels and an increase is not detectable, even though amplicon product accumulates exponentially. Eventually, enough amplified product accumulates to yield a detectable signal. The cycle number at which this occurs is called the threshold cycle or Ct. Since the Ct value is measured in the exponential phase when reagents are not limited, qt-PCR can be used to reliably and accurately calculate the initial amount of template present in the reaction. The Ct of a reaction is primarily determined by the amount of nucleic acid sequence corresponding to amplicon present at the start of the amplification reaction. If a large amount of template is present at the start of the reaction, relatively few amplification cycles will be required to accumulate enough products to provide a fluorescent signal above the background. Thus, the reaction will have a low, or early, Ct. If a small amount of template is present at the start of the reaction, the reaction will have a high, or late, Ct. The present invention allows for the measurement of the accumulation of multiple amplicons in a single fluid in a single amplification reaction, and thus the determination of the amount of multiple nucleic acid sequences in the same sample with the methodology of qt-PCR described above.

In general, the present invention is based on the idea that the genetic status of a fetus, such as fetal gender and deviations from the normal chromosomal number, i.e. aneuploidies might be determined by identifying and/or quantifying expression profiles of ncRNAs and/or precursors thereof in a sample from a subject. As indicated by the experimental data provided herein and described within the examples section, these profiles deviate from expression profiles of respective control subject, if the examined subject has a different status concerning pregnancy or concerning the genetic status of the fetus carried than the control subject or the fetus carried by the control subject.

For identification of the ncRNA expression profiles, preferably high throughput methods are used, such as microarray or NGS. However, as a means for data privacy protection of both the subject and fetus, the methods of the present invention are preferably used to identify a minimal data set required for the respective diagnosis of a chromosomal deviation of the fetus only and/or for determining the fetal gender. This provides two mean improvements in comparison to methods generating higher data input, as by reduction of the data investigated, the methods of the present invention are less costly and no excessive information is generated, as, e.g., by methods examining the whole fetal genome or bigger parts thereof.

In general, ncRNA expression profiles might be informative in regard to qualitative and/or quantitative differences measured between the analyzed and the control sample, wherein qualitative differences might be defined as follows:
1. A ncRNA might be expressed, which is usually not expressed.
2. A ncRNA might be absent, which is expressed during a normal pregnancy.

Quantitative differences might be defined as follows:
1. Expression of one or more ncRNAs is increased in comparison to the control sample.
2. Expression of one or more ncRNAs is reduced in comparison to the control sample.

All of these possible differential outcomes can be reliably measured by the methods of the present invention, such as RT-PCR based methods and determined differences in expression profiles may be generally used to determine whether the genetic status of the analyzed fetus is the same or different than the genetic status of a control fetus.

Unless stated otherwise, the terms "disorder" and "disease" are used interchangeably herein. The terms are in general used in reference to the symptoms associated with the occurrence of an aneuploidy in a fetus or subject.

The term "affinity matrix" refers to a substrate to which an affinity reagent (capture ligand) is attached. An affinity matrix of this invention comprises an affinity reagent that is capable of binding to a RNA, ncRNA and/or precursor thereof as described herein, attached to or complexed with a matrix material" *(i.e.,* a substrate or support). Generally, the affinity matrix comprises a matrix material attached to molecules capable of specifically binding of RNAs, ncRNAs and/or precursors thereof for determination of these molecules by methods as described in detail above, *e.g.,* hybridization and/or amplification based methods. Each of these molecules acts as a capture ligand.

In one embodiment, the RNAs, ncRNAs and precursors thereof are analyzed on an affinity matrix, wherein said affinity matrix is formed by irreversibly linking molecules specifically binding to said RNAs, ncRNAs and precursors thereof, preferably nucleic acid molecules such as primers or probes to a substrate. Suitable matrix materials include but are not limited to cross-linked polysaccharides, sepharose, agarose, ceramics, minerals, metals, glass, natural and synthetic organic and inorganic polymers *(e.g.,* polystyrene, latex), plastic, and cellulose. In one aspect, the present invention relates to an affinity matrix as defined above, *i.e*. comprising a covalently linked nucleic acid and thus being capable of specifically binding a RNA, ncRNA and precursor thereof, for example for use in the real time PCR based determination method of fetal DS of the present invention.

The matrix material can take any of a number of morphologies. These include, but are not limited to solid or porous beads or other particles, solid surfaces *(e.g.* array substrates), columns, capillaries, or wells. In one preferred embodiment, the matrix material takes a form of a solid surface. In certain embodiments, the affinity matrix is packed into a column, a mini-column, or a capillary or microcapillary, or a capillary electrophoresis tube.

In some embodiments, glass slides are used to prepare biochips. The substrates can also be made of silica, silicon, plastic, metal, metal-alloy, anopore, polymeric and nylon. The surfaces of substrates can be treated with chemicals prior to attaching probes to enhance the binding or to inhibit non-specific binding. Examples of chemical surface modifiers and preparation methods of glass slides with such chemically modified surfaces are known in the art; see, e.g., Microarray Biochip Technologies, Mark Schena, Editor, March 2000, Biotechniques Books.

Probes can be deposited onto the substrates, using either contact-mode printing methods using solid pins, ink-jet systems, etc. (see, *e.g.,* U.S. Pat. Nos. 6,083,763 and 6,110,426) or noncontact printing methods (using piezoelectric, bubble-jet, etc.)

For the arrays of the present invention, the plurality of probes is located on multiple addressable regions on the solid substrate. In some embodiments the solid substrate has about 2, 3, 4, 5, 6, or 7-10, 10-50, 50-100, 100-500, 500-1,000, 1,000-5,000, 5,000-10,000, 10,000-50,000, 50,000-100,000, 100,000-500,000, 500,000-1,000,000 or over 1,000,000 addressable regions with probes.

In some embodiments, the label can be bound to the probe by non-covalent means, such as by hybridization. In this embodiment, the microarray is first hybridized with a labeled oligonucleotide that is complementary to a part of the probe's sequence segment, resulting in an array in which the spots or features where the probes are located now contain fluorescent labels. These non-covalently bound labels can be bound to the probe such that FRET and/or quenching of the label occur upon binding of an amplicon to the portion of the probe specific for the amplicon.

In a further aspect, the present invention relates to a chip, for example a biochip comprising an affinity matrix as described above. For example, chips and in particular biochips are capable of discriminating complex biological samples via an array of discrete biological sensing elements immobilized onto a solid support; see, e.g., international applications WO97/049989 and WO01/040796, the disclosure content of which is incorporated herein by reference. Sensing elements include biological material such as nucleic acids, peptides, enzymes, glycans, etc. In one embodiment the biochip is prepared from gene-specific oligonucleotide probes as sensing elements, or parts thereof, generated from ncRNAs as indicated in Table 12. For example, the array may contain two different oligonucleotide probes for each ncRNA, one containing the active sequence and the other being specific for the precursor of the ncRNA. The array may also contain controls such as the "reference miRNAs" from Table 12 and/or one or more sequences differing from sequences as indicated in Table 12 by few bases, which can serve as controls for hybridization stringency conditions. tRNAs may also be printed on the microchip, providing an internal positive control for specific hybridization. One or more appropriate controls for non-specific hybridization may also be included on the chip, such as these indicated as "not in plasma control" in Table 12.

As described already in detail above, detection and quantification of the RNAs in question, e.g., ncRNAs such as snRNAs, miRNAs and/or precursors thereof, by hybridization may be accomplished according to standard techniques, *e.g.,* the Northern blotting technique using appropriately labeled DNA or RNA probes complementary to the RNA in question, wherein determination and quantification of the expression of said RNA molecules may be assessed by common imaging methods and by computerized imaging and analysis systems. In one embodiment, the method of the present invention for evaluation of chromosomal aneuploidy of a fetus and/or for determination of fetal gender is provided, wherein analyzing the presence and/or amount of the ncRNAs or precursors thereof comprises the use of a blotting based method, wherein preferably the blotting based method is Northern Blotting. Here, improved Northern Blot protocols adapted to monitor miRNAs are preferred, e.g., protocols using LNA-modified oligonucleotides. Due to their higher sensitivity, such protocols require much less isolated RNA material and are simultaneously highly specific, as described, *e.g.,* by Valoczi et al. [97]; see on Page 2 in the "Materials and Methods" section, the subsections "Synthesis of the LNA-modified oligonucleotide probes" and "RNA extraction and northern blot analysis" the disclosure content of which is incorporated herein in entirety.

These and other embodiments are disclosed and encompassed by the description and examples of the present invention. Further literature concerning any one of the materials, methods, uses and compounds to be employed in accordance with the present invention may be retrieved from public libraries and databases, such as the public database "Medline", hosted by the National Center for Biotechnology Information and/or the National Library of Medicine at the National Institutes of Health. Further databases and web addresses, are known to the person skilled in the art and can also be obtained using internet search engines. An overview of patent information in biotechnology and of sources of patent information useful for retrospective searching and for current awareness is given in Berks (1994) [98].

The above disclosure generally describes the present invention. Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2. Full bibliographic citations may be found in the References section preceding the claims. The contents of all cited references (including literature references, issued patents, published patent applications as cited throughout this application and manufacturer's specifications, instructions, etc.) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

### Examples

The examples following below illustrate the invention, but should not be construed to limit the scope of the invention in any way. The experiments in Examples 1 to 6 are illustrated and described with respect to the individual miRNA genes as identified in the serum samples, and new uses of these miRNAs, their precursors, variants and analogues in diagnosis of the genetic status of a fetus; please also refer to the Figures and Tables 1 to 13 in this respect.

### Materials and Methods

### Study group and sample preparation:

EDTA blood samples were obtained from seven T21 pregnancies and seven appropriately matched controls (maternal age, gestational week, fetal gender) (see Table 1, below) and were processed within 24 h. To harvest cell-free plasma, samples were spun at 3500 rpm (2.000 rcf) for 5 min. Plasma was then transferred to a clean tube followed by centrifugation at 14.000g for 10 minutes to remove cell debris. This study was approved by the local ethical committee, with all participants providing written consents.

**Table 1: Detailed overview of samples investigated**

| **Sample** | **Group** | **Maternal age [years]** | **Week of gestation** | **Fetal gender** | **Additional notes** |
|---|---|---|---|---|---|
| 1 | Trisomy 21 fetuses | 36 | 12+1 | female | increased NT |
| 2 | | 43 | 13+3 | female | NT 5.1 |
| 3 | | 35 | 19+0 | female | AV canal |
| 4 | | 37 | 13+3 | male | NT 4.2 |
| 5 | | 34 | 13+3 | male | NT 7.5, hem |
| 6 | | 42 | 13+4 | male | NT 6.6 |
| 7 | | 44 | 13+6 | female | NT 6.6, Softmarker |
| Match to 1 | Healthy fetuses | 42 | 13+1 | female | - |
| Match to 2 | | 41 | 13+0 | female | - |
| Match to 3 | | 42 | 17+0 | female | - |
| Match to 4 | | 40 | 13+3 | male | - |
| Match to 5 | | 31 | 13+3 | male | - |
| Match to 6 | | 41 | 14+2 | male | - |
| Match to 7 | | 35 | 13+5 | female | - |

| | | | | | |
|---|---|---|---|---|---|
| Abbreviations: NT = nuchal translucency, AV canal = atrioventricular canal defect, hem = hemolytic plasma | | | | | |

### MicroRNA Isolation

For application in NGS, miRNAs were isolated from 200 µl plasma using Trizol LS™ (Invitrogen; Carlsbad, USA) in combination with an automated mirVANA™ PARIS™ kit (Ambion; Carlsbad, USA) in accordance to the manufacturer's instructions. miRNA samples were subsequently pooled into two different pools: T21 pregnancies and healthy controls. For HT-qPCR, miRNAs were also isolated from 200 µl plasma with the miRNeasy® Mini Kit (Qiagen, Hilden, Germany) using a modified protocol. In brief, 750 µl fresh QIAzol™ master mix (800 µl QIAzol and 1.25 µl 0.8 µg / ml MS2 RNA per sample) was added, followed by 5 min incubation at RT. 200 µl chloroform were added and the sample was incubated for 2 min at RT, followed by centrifugation for 15 min at 12.000xg at 4°C. Further preparations of the miRNA samples were performed according to the manufacturer's instructions. RNA was eluted with 50 µl of RNase- and DNase free water. Concentrations were measured with the Agilent 2100 Bioanalyzer using small RNA chips (Agilent, CA, USA). As human plasma samples contain minimal amounts of extracellular cell-free RNA, glycogen (Roche, Germany) was always applied as carrier RNA to enhance extraction efficiency and yields.

### Small RNA Library construction

Small RNA libraries were prepared using a TruSeq™ Small RNA sample preparation kit, (Illumina Inc., San Diego USA) in accordance to the manufacturer's instructions. In brief, 100 ng/µl of sample from each pool was used. Barcoding was performed as follows: Pool 1 contained DS cases and was ligated to the *ATCACG* reference sequence; Pool 2 contained matched healthy pregnancy samples which were ligated to the *CGATGT* reference sequence. Following adapter ligation, cDNA synthesis and PCR amplification was performed as recommended by the manufacturer. Reverse transcription followed by PCR was used to create cDNA constructs based on the small RNA ligated with 3' and 5' adapters. PCR was performed with two primers which anneal to the ends of the adapters. Concentration of cDNA was estimated via Agilent high sensitivity DNA chips (Agilent, CA, USA) as recommended by the manufacturer. For small RNA library concentrations, KAPA quantification was used to calculate the concentration in nM. Individual pools were pooled prior to gel purification.

### Next Generation Sequencing on Illumina HiSeq® 2000

High-throughput sequencing of pooled samples was performed on the Illumina HiSeq® 2000 sequencer (Aarhus, Denmark). Three pools per lane were applied, resulting in a sequence depth over 10 million reads per pool. HT-qPCR was performed subsequently for profiling and validation of the differentially expressed ncRNAs found.

### High-throughput quantitative PCR (HT-qPCR)

HT-qPCR was performed on individual samples using the SmartChip Human miRNA Panel V3.0 (Wafergen, CA, USA) in accordance to the manufacturer's instructions. This SmartChip simultaneously and quantitatively measures the expression of the most disease-relevant 1100 human microRNAs in quadruplicate. Polyadenylation of miRNA (100 ng) was performed using a modified protocol of the A-Plus™ Poly (A) Polymerase Tailing Kit (Epicentre, WI, USA). Polyadenylation was performed by incubation at 37°C for 30 min, followed by a 5 min denaturation step at 70°C. Polyadenylated miRNAs were reverse transcribed to cDNA using an anchored oligo(dT)-containing primer. RT-PCR was performed by incubating the samples at 40°C for 60 min, followed by denaturation for 5 min at 80°C. Quantitative Real-time PCR with the cDNA was performed on SmartChip Human miRNA Panel v3.0 and the SmartChip Cycler with the following thermal profile: Polymerase activation for 6 min at 95°C, pre-amplification for 60 s at 50°C, followed by 39 cycles comprising of 95°C for 60 s and 60°C for 70 seconds. A No Template Control (NTC) was also performed.

### Bioinformatic and statistical analysis

Preparative analysis of the NGS data was performed using CLC Bio Software Version 5.0 (http://www.clcbio.com, Aarhus, Denmark). Sequences were trimmed of the adaptor sequence, and reads with no adaptors were removed. Annotations of the sequences clusters were performed against miRBase v17.0 and Ensembl GRCh37.57 ncRNA database. Expression values derived from the CLC Software were used for downstream statistical analysis using "R" and BioConductor packages (www.bioconductor.org [99] including total expression for ncRNAs and mature miRNAs, overall comparison of the expression of each pool via scatter plots, differential expression based on IDEG6 [100], MA-plots and edgeR [101].

For MA-plots, samples were normalized to have a total intensity of 1 x 10⁶. To avoid zero expression (and infinite log values), pseudo-counts of 1 were substituted for miRNAs with zero reads. Standard deviation in M (log2 fold change) was fitted across A (average log2 expression). Based on the z-score, p-values and FDRs were calculated. A FDR-adjusted p-value < 0.05 was termed as statistically significant.

HT-qPCR analysis was performed using both qBase Software (Biogazelle, Belgium) and BioConductor (HT-qPCR Package). The WaferGen qPCR Software generated a report which provides a short overview on raw data (replicates) Cq's, distance between sample and NTC, and normalized relative quantities (NRQ). These data were also used as a template for downstream analysis using "R" and Bioconductor. Data export was re-computed for each sample with the SmartChip qPCR Software to export RMDL data for the qBase Software. This tool which uses individual Cq data and takes into account the flagging system by the SmartCycler software was used for quality control, normalization using the "global mean on common targets", NTC correction, group comparison and for error propagation.

Downstream analysis of the HT-qPCR was also performed in R / Bioconductor using several functions from the HT-qPCR package. Many functions from the package did not work due to non-available values in the list of Cqs. Therefore, clustering and classification were performed on standard R functions which were adjusted to the data sets. A FDR-adjusted p-value < 0.05 was termed as statistically significant.

### Example 1: Potential Trisomy 21-associated miRNAs of pooled samples using NGS.

More than 6 million total reads were obtained for each of the libraries. These reads were then compared with the sequence data available in miRBase 17 database and Ensembl GRCh37.57ncrna. Using miRBase17, 3,002,323 (89.3%) reads were mapped to the human miRNA sequences in the database, whereas 3,031,556 (89.9%) reads were mapped from the control group. The average read length following trimming was 21.5 nucleotides.

Small RNA species in the sequencing libraries comprised of 363,364 reads in the DS group and 374,491 reads in the control group. Of these reads, 38,195 (10.5%) and 31,039 from the respective DA and control cohorts were annotated using CLC Bio. Annotation of these small RNAs using the miRBase 17 and Ensembl GRCh37.57ncrna databases revealed that 48.2% and 46.4% of the reads from DS and the healthy control group were annotated to miRBase 17.

miRNA-486 (HSA-MIR-486) was found to be highly abundant and present on all samples thus having the potential of being classified as a pregnancy-associated miRNA. For premature miRNAs: Down - 61 % of the total expression, 68% - Partner; 76% - Pregnant; Non-pregnant - 81%; RHD- - 88%; RHD+ - 89%. The values were slightly higher for mature miRNAs. Excluding mir-486 from analysis considerably alters the total expression. RHD+/- is then observed to have a reduction in total expression than Down or Partner - an observation which is in line with the previously detected number of distinct miRNAs. miRNA-486 was excluded eexcluded from analysis thus with the number of different detected premature miRNAs correlating with total expression then.

Expression values following its exclusion were analyzed via three different methods; IDEG6, MA-plots and edgeR. Gene expression analysis, however, was found to be most optimal with the use of MA-plots. Using this approach, 28 potential biomarkers (miRNAs and other small non-coding RNAs) were found between the DS and partner group as illustrated via the MA-plot (Fig. 1). In brief, only 13 of the 28 potential biomarkers were found to be mature miRNAs, which included HSA-MIR-885, HSA-MIR-122, HSA-MIR-375, HSA-MIR199b, HSA-MIR-99a, HSA-MIR-199a-1, HSA-MIR-199a-2, HSA-MIR-3614, HSA-MIR-483, HSA-MIR-4306, HSA-MIR-4772, HSA-MIR-30a and HSA-MIR-3690. The remaining 15 potential biomarkers were either snRNA or snoRNAs.

### Analysis of NGS-Data

### Data

For the analysis, expression values were extracted from the tables for grouped and mature miRNAs (which were derived by using CLC for trimming and mapping of reads). Expression values for six different samples were collected: Down, Partner, Pregnant, Non-pregnant, RHD+ and RHD-; see tables 2 and 3 below.

### Descriptive overall analysis

Total expression of Down, Partner and Pregnant samples were similar. Sequencing of sample 'Non-pregnant' seems to have failed. Total expression for the RHD +/- was higher than for the others, which is somewhat in contrast with the number of mapped miRNAs. Here, the Down and Partner groups demonstrated a considerably higher number (ca. 5000 distinct premature miRNAs) compared to RHD+/- with ca. 3000 premature miRNAs) - which could indicate a higher complexity in expression, as the majority of miRNAs detected in RHD samples were also detected in Down/Partner groups (see table of shared miRNAs). The number of distinct mature miRNAs ranged between 388 for the Down group and 215 for RHD +/-. Notably, the non-pregnant group is with only nine different microarrays an outlier.

**Table 2: Number of shared pre mature miRNAs**

| | Down | Partner | Pregnant | Non-pregnant | RHD- | RHD+ |
|---|---|---|---|---|---|---|
| Down | ***4936*** | ***3793*** | ***3414*** | ***56*** | ***2857*** | ***2801*** |
| Partner | ***3793*** | ***4866*** | ***3444*** | ***56*** | ***2758*** | ***2726*** |
| Pregnant | ***3414*** | ***3444*** | ***4337*** | ***56*** | ***461*** | ***2485*** |
| Non-pregnant | ***56*** | ***56*** | ***56*** | ***56*** | ***56*** | ***56*** |
| RHD- | ***2857*** | ***2758*** | ***2461*** | ***56*** | ***3199*** | ***2477*** |
| RHD+ | ***2801*** | ***2726*** | ***2485*** | ***56*** | ***2477*** | ***3273*** |

**Table 3: Number of shared mature micro RNAs**

| | Down | Partner | Pregnant | Non-pregnant | RHD- | RHD+ |
|---|---|---|---|---|---|---|
| Down | ***388*** | ***295*** | ***282*** | ***9*** | ***206*** | ***198*** |
| Partner | ***295*** | ***338*** | ***275*** | ***9*** | ***194*** | ***192*** |
| Pregnant | ***282*** | ***275*** | ***310*** | ***9*** | ***191*** | ***187*** |
| Non-pregnant | ***9*** | ***9*** | ***9*** | ***9*** | ***9*** | ***9*** |
| RHD- | ***206*** | ***194*** | ***9*** | ***9*** | ***215*** | ***172*** |
| RHD+ | ***198*** | ***192*** | ***187*** | ***9*** | ***172*** | ***215*** |

### Example 2: Potential T21-associated miRNAs of individual samples using HT-qPCR

Seven samples from DS pregnancies and seven equally matched controls were individually analyzed for 1100 ncRNAs (mature miRNAs) on the WaferGen SmartChip in quadruplicates. This was performed not only to validate the NGS data, but also used as a second profiling approach with different strategies based on Cqs and NRQs to identify potential new biomarkers. For flagging, delta cqs values were used. Cqs values with delta cqs<2 were flagged and qs values with delta cqs<3.3 were termed "marginal". Additionally, cq values equaling 0 were flagged.

The majority of miRNAs were flagged in all samples. Furthermore, 348 miRNAs were flagged in all samples and were excluded from downstream analysis, resulting in 695 miRNAs remaining. For the remaining miRNAs, many cqs values were flagged. This filtered data set included a total of 328 miRNAs. Comparison of the relative expression of these miRNAs was performed resulting in the identification of 37 mature miRNAs which were found to be significantly differentially expressed in T21 pregnancies in comparison to the healthy pregnant cohort (see Table 4). Venn analysis was performed on the miRNAs identified by HT-qPCR based on different strategies to identify significantly differentially expressed miRNAs. Ten miRNAs were identified as common elements by both Cq (Raw, GN, QN) and NRQ (Raw and GN) values via bioinformatical analysis, whereas 19 miRNAs were identified only by Cq values, and 8 only by NRQs.

**Table 4: List of 37 potential biomarkers identified to be significantly (p-value <0.05) differentially expressed by HT-qPCR.**

| **No.** | | **MicroRNA** | **Mean Ct of DS** | **Mean Ct of Control** | **Differences in mean Cts** | **p-value** | **p-value origin** |
|---|---|---|---|---|---|---|---|
| **1** | **Commonly identified by Cqs and NRQs** | HSA-MIR-512-5P | 30,97 | 33,66 | -2,69 | 0,00861 | Cqs GN |
| **2** | | HSA-MIR-483-5P | 31,80 | 33,82 | -2,02 | 0,00454 | Cqs GN |
| **3** | | HSA-MIR-888* | 30,58 | 34,67 | -4,09 | 0,00428 | Cqs GN |
| **4** | | HSA-MIR-3676 | 33,30 | 31,23 | 2,08 | 0,03754 | Cqs GN |
| **5** | | HSA-MIR-3918 | 27,66 | 28,39 | -0,73 | 0,02360 | Cqs GN |
| **6** | | HSA-MIR-500A | 30,58 | 31,40 | -0,82 | 0,02986 | Cqs GN |
| **7** | | RNU12 | 28,22 | 29,34 | -1,12 | 0,03806 | Cqs GN |
| **8** | | HSA-MIR-30C | 27,05 | 28,07 | -1,02 | 0,04203 | Cqs GN |
| **9** | | HSA-MIR-200C | 32,21 | 30,53 | 1,69 | 0,04393 | Cqs GN |
| **10** | | HSA-MIR-627 | 33,36 | 31,97 | 1,40 | 0,04518 | Cqs GN |
| **11** | **Identified by Cq's only** | HSA-MIR-589* | 31,40 | 33,86 | -2,46 | 0,00283 | Cqs raw |
| **12** | | HSA-MIR-181A* | 33,55 | 30,79 | 2,75 | 0,01864 | Cqs GN |
| **13** | | HSA-MIR-362-5P | 31,94 | 34,54 | -2,60 | 0,02495 | Cqs GN |
| **14** | | HSA-MIR-518C* | 31,35 | 35,04 | -3,69 | 0,01990 | Cqs GN |
| **15** | | HSA-MIR-3667-5P | 30,69 | 33,21 | -2,52 | 0,02596 | Cqs raw |
| **16** | | HSA-MIR-195* | 29,35 | 30,58 | -1,23 | 0,03372 | Cqs GN |
| **17** | | HSA-MIR-124* | 31,72 | 33,32 | -1,60 | 0,03635 | Cqs GN |
| **18** | | HSA-MIR-498 | 34,42 | 31,45 | 2,98 | 0,02934 | Cqs GN |
| **19** | | HSA-MIR-18B* | 30,71 | 33,30 | -2,59 | 0,04078 | Cqs raw |
| **20** | | HSA-MIR-1249 | 26,72 | 29,20 | -2,48 | 0,04587 | Cqs raw |
| **21** | | HSA-MIR-93 | 25,76 | 24,76 | 1,00 | 0,00944 | Cqs QN |
| **22** | | HSA-MIR-20A | 25,04 | 24,27 | 0,77 | 0,01837 | Cqs QN |
| **23** | | HSA-MIR-4328 | 32,22 | 31,25 | 0,97 | 0,02078 | Cqs QN |
| **24** | | HSA-MIR-92A | 22,95 | 22,10 | 0,85 | 0,02647 | Cqs QN |
| **25** | | HSA-MIR-92B | 27,65 | 26,78 | 0,88 | 0,02926 | Cqs QN |
| **26** | | HSA-MIR-3652 | 30,62 | 31,64 | -1,02 | 0,02994 | Cqs QN |
| **27** | | HSA-MIR-25 | 24,19 | 23,42 | 0,77 | 0,03759 | Cqs QN |
| **28** | | HSA-MIR-623 | 33,77 | 31,63 | 2,13 | 0,03945 | Cqs QN |
| **29** | | HSA-MIR-3195 | 23,11 | 24,60 | -1,49 | 0,04885 | Cqs QN |
| **30** | **Identified by NRQs only** | HSA-MIR-423-5P | 0,87 | 1,22 | -0,35 | 0,02007 | NRQ Raw |
| **31** | | HSA-MIR-27B | 1,41 | 0,85 | 0,57 | 0,03759 | NRQ Raw |
| **32** | | HSA-MIR-3135 | 1,56 | 0,66 | 0,91 | 0,04044 | NRQ Raw |
| **33** | | HSA-MIR-486-5P | 0,83 | 1,66 | -0,83 | 0,02598 | NRQ GN |
| **34** | | HSA-MIR-3180-5P | 0,71 | 1,17 | -0,46 | 0,04036 | NRQ GN |
| **35** | | HSA-MIR-629 | 0,81 | 1,62 | -0,81 | 0,04457 | NRQ GN |
| **36** | | HSA-MIR-155 | 0,93 | 1,89 | -0,96 | 0,04712 | NRQ GN |
| **37** | | HSA-MIR-3907 | 0,80 | 1,46 | -0,65 | 0,04983 | NRQ GN |

### Example 3: Comparison of miRNAs identified by NGS with HT-qPCR

Within NGS, 60 unique potential biomarkers could be detected, 37 unique potential biomarkers were identified using HT-qPCR strategy. It should also be mentioned that most of the potential biomarkers (n=35) were precursors, immature miRNAs, snRNA or snoRNAs, which have not been profiled within the HT-qPCR method. However, the remaining 25 mature miRNAs identified with NGS do not share common elements with the mature miRNAs identified by HT-qPCR. However, comparison of mature miRNAs identified by NGS did not share any similarities to those identified with HT-qPCR. Pooling of the samples was thought to be beneficial in order to reduce the bio-variability by simultaneous accumulating potential DS-specific miRNAs in the corresponding pool. However, results obtained by HT-qPCR on individual samples are more reliable and highly accurate in comparison to the data generated by NGS. Library preparation required for NGS tends to suffer from a bias that significantly influences the outcome of the results. Furthermore, HT-qPCR revealed that the miRNAs identified by NGS are the consequence of a high-abundance expression of the particular miRNA in only one of the samples in the pool, resulting in a false-positive identification of significantly differentially expressed miRNAs in one pool in comparison to the other. Running individual samples for a high amount of mature miRNAs also exhibits the advantage of visualizing the bio-variability of distinct miRNAs. As presented in Fig. 2, a specific DS miRNA allowing differentiation between healthy and DS pregnancies could not be identified with NGS.

Experiments provided and described herein provide a new method for non-invasive prenatal diagnosis of fetal T21 via identification and/or quantification of miRNAs in maternal plasma via NGS and HT-qPCR, which differentiated T21 pregnancies from healthy pregnancies in all seven cases. miRNAs such as miRNA-512-5P, miRNA-181A, miRNA-483-5P and miRNA-200C were previously identified as pregnancy-associated [49, 103], and were found to be overexpressed in the DS group, although, this was not observed for the abundant plasma placental miRNAs (miR-141, miR-149, miR-299-5p and miR-135b) described by Chim et al. [30], which may be due to the small sample size used herein. A combination of the data provided herein with the biochip technology allows customized chips, designed to contain only selected miRNAs. Targeted testing by arrays would detect only clinically significant abnormalities, minimizing detection of unclear findings [113, 114]. Furthermore, a selection of only disease-specific miRNAs would greatly reduce diagnostic costs, in comparison to, e.g., genome-wide sequencing.

### Example 4: Identification of subsets of miRNAs resulting in the differentiation between DS and healthy pregnancies.

Clustering of the miRNAs detected in the human plasma samples (full-set of miRNAs) and for subsets of the most significantly differentially expressed miRNAs were performed. Using the full set of miRNAs, it was not possible to identify a particular miRNA signature that allows for the differentiation between DS and healthy pregnancies, as demonstrated via heat map analysis (Fig. 3). However, using a subset of 10 or 20 miRNAs that were most significantly differentially expressed resulted in the separation of both groups. MiRNAs identified by Cq values (raw and GN) as well as miRNAs identified via the raw NRQ value demonstrated a distinct separation of the two groups (Fig. 4B). Only the global normalized NRQ values, which also use a minimum of 10 miRNAs, failed to result in the differentiation of both groups. Tables 5 to 9 below identify examples of miRNA subsets, which (except the GN NRQ subset in Table 9) could be used according to the methods of the present invention for a separation of either DS versus healthy pregnancies or female versus male fetuses.

**Table 5: Exemplary Raw Cqs data subset used for DS pregnancy evaluation. Up -indicates an increased, down-a decreased expression of the respective miRNA in the last column of the following Tables 6-12.**

| **Top 20** | **miRNA** | **DS mean Cqs** | **Control mean Cqs** | **Number of DS samples identified in** | **Number of control samples identified in** | **Difference of mean expression** | **p-value** | **Expression in DS sample s** |
|---|---|---|---|---|---|---|---|---|
| 1 | HSA-MIR-589* | 31,40 | 33,86 | 2 | 2 | -2,46 | 0,0028 | up |
| 2 | HSA-MIR-512-5P | 30,70 | 33,35 | 3 | 5 | -2,65 | 0,0035 | up |
| 3 | HSA-MIR-181A* | 33,24 | 30,11 | 4 | 2 | 3,13 | 0,0105 | down |
| 4 | HSA-MIR-483-5P | 31,57 | 33,55 | 4 | 5 | -1,98 | 0,0202 | up |
| 5 | HSA-MIR-362-5P | 31,49 | 34,47 | 4 | 2 | -2,98 | 0,0222 | up |
| 6 | HSA-MIR-518C* | 30,97 | 34,81 | 4 | 2 | -3,84 | 0,0259 | up |
| 7 | HSA-MIR-3667-5P | 30,69 | 33,21 | 2 | 3 | -2,52 | 0,0260 | up |
| 8 | HSA-MIR-195* | 29,04 | 30,39 | 5 | 6 | -1,35 | 0,0263 | up |
| 9 | HSA-MIR-124* | 31,36 | 33,24 | 4 | 3 | -1,88 | 0,0268 | up |
| 10 | HSA-MIR-498 | 33,85 | 31,54 | 3 | 4 | 2,31 | 0,0388 | down |
| 11 | HSA-MIR-18B* | 30,71 | 33,30 | 4 | 4 | -2,59 | 0,0408 | up |
| 12 | HSA-MIR-888* | 30,55 | 34,13 | 2 | 3 | -3,58 | 0,0453 | up |
| 13 | HSA-MIR-1249 | 26,72 | 29,20 | 2 | 6 | -2,48 | 0,0459 | up |
| 14 | HSA-MIR-3676 | 33,19 | 31,28 | 3 | 3 | 1,92 | 0,0477 | down |
| 15 | RNU12 | 27,95 | 29,17 | 6 | 5 | -1,22 | 0,0564 | up |
| 16 | HSA-MIR-215 | 30,15 | 31,11 | 2 | 3 | -0,97 | 0,0566 | up |
| 17 | HSA-MIR-623 | 33,69 | 31,78 | 6 | 3 | 1,90 | 0,0612 | down |
| 18 | HSA-MIR-1227 | 31,49 | 33,04 | 5 | 4 | -1,54 | 0,0687 | up |
| 19 | HSA-MIR-30C | 26,79 | 27,95 | 7 | 7 | -1,15 | 0,0716 | up |
| 20 | HSA-MIR-187 | 31,47 | 32,75 | 7 | 5 | -1,28 | 0,0747 | up |

**Table 6: Exemplary GN Cqs data subset used for DS pregnancy evaluation.**

| **Top 20** | **miRNA** | **DS mean Cqs** | **Contro I mean Cqs** | **Number of DS samples identified in** | **Number of control samples identified in** | **Difference of mean expression** | **p-value** | **Expression in DS samples** |
|---|---|---|---|---|---|---|---|---|
| 1 | HSA-MIR-888* | 30,58 | 34,67 | 2 | 3 | -4,09 | 0,0043 | up |
| 2 | HSA-MIR-483-5P | 31,80 | 33,82 | 4 | 5 | -2,02 | 0,0045 | up |
| 3 | HSA-MIR-512-5P | 30,97 | 33,66 | 3 | 5 | -2,69 | 0,0086 | up |
| 4 | HSA-MIR-181A* | 33,55 | 30,79 | 4 | 2 | 2,75 | 0,0186 | down |
| 5 | HSA-MIR-518C* | 31,35 | 35,04 | 4 | 2 | -3,69 | 0,0199 | up |
| 6 | HSA-MIR-3918 | 27,66 | 28,39 | 5 | 3 | -0,73 | 0,0236 | up |
| 7 | HSA-MIR-362-5P | 31,94 | 34,54 | 4 | 2 | -2,60 | 0,0249 | up |
| 8 | HSA-MIR-498 | 34,42 | 31,45 | 3 | 4 | 2,98 | 0,0293 | down |
| 9 | HSA-MIR-3676 | 33,51 | 31,40 | 3 | 3 | 2,11 | 0,0296 | down |
| 10 | HSA-MIR-500A | 30,58 | 31,40 | 6 | 4 | -0,82 | 0,0299 | up |
| 11 I | HSA-MIR-195* | 29,35 | 30,58 | 5 | 6 | -1,23 | 0,0337 | up |
| 12 | HSA-MIR-124* | 31,72 | 33,32 | 4 | 3 | -1,60 | 0,0363 | up |
| 13 | RNU12 | 28,22 | 29,34 | 6 | 5 | -1,12 | 0,0381 | up |
| 14 | HSA-MIR-30C | 27,05 | 28,07 | 7 | 7 | -1,02 | 0,0420 | up |
| 15 | HSA-MIR-200C | 32,21 | 30,53 | 5 | 7 | 1,69 | 0,0439 | down |
| 16 | HSA-MIR-627 | 33,36 | 31,97 | 4 | 7 | 1,40 | 0,0452 | down |
| 17 | HSA-MIR-219-5P | 31,42 | 33,65 | 2 | 4 | -2,24 | 0,0504 | up |
| 18 | HSA-MIR-4328 | 32,45 | 31,57 | 4 | 2 | 0,88 | 0,0506 | down |
| 19 | HSA-MIR-93* | 29,51 | 30,76 | 6 | 5 | -1,26 | 0,0591 | up |
| 20 | HSA-MIR-3913 | 32,15 | 33,80 | 4 | 2 | -1,65 | 0,0628 | up |

**Table 7: Exemplary QN Cqs data subset used for DS pregnancy evaluation. Only values 1-20 have been clustered for the analysis shown in Fig. 4.**

| **Top 20** | **miRNA** | **DS mean Cqs** | **Control mean Cqs** | **Number of DS samples identified in** | **Number of control samples identified in** | **Difference of mean expressi on** | **p-value** | **Expression in DS samples** |
|---|---|---|---|---|---|---|---|---|
| 1 | HSA-MIR-512-5P | 30,71 | 33,22 | 3 | 5 | -2,51 | 0,0062 | up |
| 2 | HSA-MIR-181A* | 33,29 | 30,30 | 4 | 2 | 2,98 | 0,0092 | down |
| 3 | HSA-MIR-93 | 25,76 | 24,76 | 7 | 7 | 1,00 | 0,0094 | down |
| 4 | HSA-MIR-30C | 26,97 | 27,90 | 7 | 7 | -0,93 | 0,0129 | up |
| 5 | HSA-MIR-483-5P | 31,63 | 33,48 | 4 | 5 | -1,84 | 0,0155 | up |
| 6 | HSA-MIR-20A | 25,04 | 24,27 | 7 | 7 | 0,77 | 0,0184 | down |
| 7 | HSA-MIR-888* | 30,48 | 34,07 | 2 | 3 | -3,59 | 0,0202 | up |
| 8 | HSA-MIR-4328 | 32,22 | 31,25 | 4 | 2 | 0,97 | 0,0208 | down |
| 9 | HSA-MIR-3918 | 27,64 | 28,24 | 5 | 3 | -0,60 | 0,0213 | up |
| 10 | HSA-MIR-518C* | 31,09 | 34,72 | 4 | 2 | -3,63 | 0,0242 | up |
| 11 | HSA-MIR-92A | 22,95 | 22,10 | 7 | 7 | 0,85 | 0,0265 | down |
| 12 | HSA-MIR-362-5P | 31,59 | 34,34 | 4 | 2 | -2,75 | 0,0269 | up |
| 13 | HSA-MIR-92B | 27,65 | 26,78 | 7 | 7 | 0,88 | 0,0293 | down |
| 14 | HSA-MIR-3652 | 30,62 | 31,64 | 3 | 2 | -1,02 | 0,0299 | up |
| 15 | HSA-MIR-124* | 31,39 | 33,11 | 4 | 3 | -1,73 | 0,0300 | up |
| 16 | HSA-MIR-498 | 33,96 | 31,40 | 3 | 4 | 2,56 | 0,0343 | down |
| 17 | HSA-MIR-3676 | 33,30 | 31,23 | 3 | 3 | 2,08 | 0,0375 | down |
| 18 | HSA-MIR-25 | 24,19 | 23,42 | 7 | 7 | 0,77 | 0,0376 | down |
| 19 | HSA-MIR-589* | 31,44 | 33,73 | 2 | 2 | -2,29 | 0,0392 | up |
| 20 | HSA-MIR-623 | 33,77 | 31,63 | 6 | 3 | 2,13 | 0,0394 | down |
| 21 | HSA-MIR-3667-5P | 30,76 | 33,07 | 2 | 3 | -2,31 | 0,0487 | up |
| 22 | HSA-MIR-3195 | 23,11 | 24,60 | 4 | 3 | -1,49 | 0,0488 | up |

**Table 8: DS pregnancy evaluation - exemplary Raw NRQ data subset. Only values 1-10 have been clustered for the analysis shown in Fig. 4.**

| **Top 10** | **MiRNA** | **DS mean Cqs** | **Control mean Cqs** | **Number of DS samples identified in** | **Number of control samples identified in** | **Difference of mean Express ion** | **p-value** | **Expression in DS samples** |
|---|---|---|---|---|---|---|---|---|
| 1 | HSA-MIR-888* | 6,90 | 0,31 | 2 | 3 | 6,60 | 0,0002 | up |
| 2 | HSA-MIR-483-5P | 2,35 | 0,55 | 4 | 5 | 1,80 | 0,0022 | up |
| 3 | HSA-MIR-512-5P | 3,33 | 0,73 | 3 | 5 | 2,61 | 0,0052 | up |
| 4 | HSA-MIR-423-5P | 0,87 | 1,22 | 7 | 7 | -0,35 | 0,0201 | down |
| 5 | HSA-MIR-500A | 1,97 | 0,98 | 6 | 4 | 0,99 | 0,0298 | up |
| 6 | RNU12 | 1,51 | 0,76 | 6 | 5 | 0,75 | 0,0327 | up |
| 7 | HSA-MIR-200C | 0,65 | 1,95 | 5 | 7 | -1,30 | 0,0349 | down |
| 8 | HSA-MIR-3918 | 1,45 | 0,75 | 5 | 3 | 0,70 | 0,0367 | up |
| 9 | HSA-MIR-27B | 1,41 | 0,85 | 7 | 7 | 0,57 | 0,0376 | up |
| 10 | HSA-MIR-30C | 1,58 | 0,73 | 7 | 7 | 0,85 | 0,0384 | up |
| 11 | HSA-MIR-3135 | 1,56 | 0,66 | 4 | 3 | 0,91 | 0,0404 | up |
| 12 | HSA-MIR-627 | 0,57 | 1,88 | 4 | 7 | -1,31 | 0,0436 | down |
| 13 | HSA-MIR-486-5P | 0,88 | 1,56 | 7 | 7 | -0,68 | 0,0460 | up |

**Table 9: DS pregnancy evaluation - exemplary GN NRQ data subset. Only values 1-10 have been clustered for the analysis shown in Fig. 4.**

| **Top 10** | **miRNA** | **DS mean Cqs** | **Control mean Cqs** | **Number of DS samples identified in** | **Number of control samples identified in** | **Difference of mean expression** | **p-value** | **Expression in DS samples** |
|---|---|---|---|---|---|---|---|---|
| 1 | HSA-MIR-483-5P | 2,07 | 0,60 | 4 | 5 | 1,46 | 0,0011 | up |
| 2 | HSA-MIR-3918 | 1,42 | 0,91 | 5 | 3 | 0,51 | 0,0134 | up |
| 3 | HSA-MIR-200C | 0,64 | 1,97 | 5 | 7 | -1,33 | 0,0184 | down |
| 4 | HSA-MIR-627 | 0,60 | 1,87 | 4 | 7 | -1,27 | 0,0238 | down |
| 5 | HSA-MIR-486-5P | 0,83 | 1,66 | 7 | 7 | -0,83 | 0,0260 | down |
| 6 | HSA-MIR-3676 | 0,54 | 2,09 | 3 | 3 | -1,55 | 0,0308 | down |
| 7 | HSA-MIR-3180-5P | 0,71 | 1,17 | 4 | 4 | -0,46 | 0,0404 | down |
| 8 | HSA-MIR-512-5P | 3,23 | 0,78 | 3 | 5 | 2,45 | 0,0427 | up |
| 9 | HSA-MIR-629 | 0,81 | 1,62 | 7 | 7 | -0,81 | 0,0446 | down |
| 10 | HSA-MIR-155 | 0,93 | 1,89 | 6 | 5 | -0,96 | 0,0471 | down |
| 11 | HSA-MIR-3907 | 0,80 | 1,46 | 6 | 7 | -0,65 | 0,0498 | down |

### Example 5: Identification of fetal gender specific miRNA subsets.

Clustering was performed for subsets of the most significantly differentially expressed miRNAs. MiRNAs identified by Cts values (raw and GN) demonstrated a distinct separation of the two groups (Fig. 4C). Tables 10 and 11 below identify examples of miRNA subsets, which could be used according to the methods of the present invention for a separation of either DS versus healthy pregnancies or female versus male fetuses.

The subsets of miRNAs used to differentiate between DS and healthy pregnancies (43 miRNAs, HSA-MIR93* and 42 identified in Table 12) and those used for the differentiation of the fetal sex (first 18 miRNAs in Tables 10 and 11) share two common elements.

**Table 10: Fetal gender evaluation - exemplary raw data subset.**

| **No** | **miRNAs** | **Mean CQs in male fetes** | **Mean CQs in female fetes** | **No. of samples with fetes identified as male** | **No. of samples with fetes identified as female** | **Difference of mean expression** | **p-value** | **Expression in female fetes compared to male fetes** |
|---|---|---|---|---|---|---|---|---|
| 1 | HSA-MIR-590-3P | 33,33 | 30,79 | 3 | 5 | 2,55 | 0,0017 | down |
| 2 | HSA-MIR-3923 | 32,14 | 30,78 | 2 | 4 | 1,36 | 0,0202 | down |
| 3 | HSA-MIR-1228* | 26,12 | 25,34 | 6 | 8 | 0,78 | 0,0211 | down |
| 4 | HSA-MIR-769-3P | 30,53 | 31,93 | 5 | 4 | -1,40 | 0,0229 | up |
| 5 | HSA-MIR-127-5P | 33,80 | 31,96 | 3 | 5 | 1,84 | 0,0241 | down |
| 6 | HSA-MIR-1293 | 32,36 | 30,19 | 4 | 5 | 2,17 | 0,0258 | down |
| 7 | HSA-LET-7F-1* | 33,96 | 30,70 | 5 | 8 | 3,27 | 0,0340 | down |
| 8 | HSA-MIR-93* | 30,68 | 29,26 | 4 | 7 | 1,41 | 0,0428 | down |
| 9 | HSA-MIR-892B | 31,61 | 29,86 | 3 | 3 | 1,75 | 0,0444 | down |
| 10 | HSA-MIR-320B | 31,90 | 32,63 | 3 | 6 | -0,73 | 0,0473 | up |
| 11 | HSA-MIR-106A* | 29,38 | 31,12 | 4 | 7 | -1,75 | 0,0601 | up |
| 12 | HSA-MIR-92B* | 31,46 | 29,76 | 6 | 7 | 1,69 | 0,0712 | down |
| 13 | HSA-MIR-328 | 30,34 | 29,13 | 6 | 7 | 1,21 | 0,0746 | down |
| 14 | HSA-MIR-4306 | 31,95 | 30,28 | 3 | 6 | 1,68 | 0,0784 | down |
| 15 | HSA-MIR-196B* | 31,95 | 31,53 | 2 | 5 | 0,42 | 0,0825 | down |
| 16 | HSA-MIR-548C-5P | 32,53 | 30,87 | 3 | 6 | 1,67 | 0,0862 | down |
| 17 | HSA-MIR-512-3P | 31,61 | 33,08 | 5 | 4 | -1,47 | 0,0988 | up |
| 18 | HSA-MIR-1249 | 29,61 | 27,35 | 4 | 4 | 2,26 | 0,0994 | down |
| 19 | HSA-MIR-29B-1* | 33,36 | 31,99 | 4 | 7 | 1,37 | 0,1007 | down |
| 20 | HSA-MIR-30C-1* | 33,06 | 31,73 | 3 | 5 | 1,33 | 0,1013 | down |

**Table 11: Fetal gender evaluation - exemplary GN data subset.**

| **No** | **miRNAs** | **Mean CQs in male fetes** | **Mean CQs in female fetes** | **No. of samples with fetes identified as male** | **No. of samples with fetes identified as female** | **Difference of mean Expression** | **p-value** | **Expression in female fetes compared to male fetes** |
|---|---|---|---|---|---|---|---|---|
| 1 | HSA-MIR-590-3P | 33,64 | 31,22 | 3 | 5 | 2,43 | 0,0017 | down |
| 2 | HSA-MIR-3923 | 32,60 | 30,98 | 2 | 4 | 1,61 | 0,0202 | down |
| 3 | HSA-MIR-1228* | 26,22 | 25,58 | 6 | 8 | 0,64 | 0,0211 | down |
| 4 | HSA-MIR-769-3P | 30,76 | 32,21 | 5 | 4 | -1,46 | 0,0229 | up |
| 5 | HSA-MIR-127-5P | 34,20 | 32,34 | 3 | 5 | 1,86 | 0,0241 | down |
| 6 | HSA-MIR-1293 | 32,43 | 30,45 | 4 | 5 | 1,98 | 0,0258 | down |
| 7 | HSA-LET-7F-1* | 34,22 | 30,99 | 5 | 8 | 3,22 | 0,0340 | down |
| 8 | HSA-MIR-93* | 30,90 | 29,61 | 4 | 7 | 1,29 | 0,0428 | down |
| 9 | HSA-MIR-892B | 31,86 | 29,93 | 3 | 3 | 1,94 | 0,0444 | down |
| 10 | HSA-MIR-320B | 32,23 | 32,97 | 3 | 6 | -0,74 | 0,0473 | up |
| 11 | HSA-MIR-106A* | 29,60 | 31,43 | 4 | 7 | -1,82 | 0,0601 | up |
| 12 | HSA-MIR-92B* | 31,58 | 30,10 | 6 | 7 | 1,47 | 0,0712 | down |
| 13 | HSA-MIR-328 | 30,45 | 29,47 | 6 | 7 | 0,98 | 0,0746 | down |
| 14 | HSA-MIR-4306 | 32,24 | 30,61 | 3 | 6 | 1,62 | 0,0784 | down |
| 15 | HSA-MIR-196B* | 32,61 | 31,92 | 2 | 5 | 0,69 | 0,0825 | down |
| 16 | HSA-MIR-548C-5P | 32,88 | 31,25 | 3 | 6 | 1,63 | 0,0862 | down |
| 17 | HSA-MIR-512-3P | 31,75 | 33,51 | 5 | 4 | -1,76 | 0,0988 | up |
| 18 | HSA-MIR-1249 | 29,91 | 27,68 | 4 | 4 | 2,23 | 0,0994 | down |
| 19 | HSA-MIR-29B-1* | 33,56 | 32,26 | 4 | 7 | 1,29 | 0,1007 | down |
| 20 | HSA-MIR-30C-1* | 32,87 | 31,92 | 3 | 5 | 0,96 | 0,1013 | down |

### Example 6: Exiqon Validation

A cross validation of the 37 significantly identified miRNAs (see Table 4) is performed to validate the results obtained from the HT-qPCRof a miRNA subset for the distinct separation of healthy pregnancies from those with DS fetuses. HT-qPCR utilizes Sybr green and normal primer sets to amplify the targets. Thus, the cross-validation study is performed via the application of a different method. Exiqon's LNA primer® are known to require lower annealing temperatures thus allowing for a more accurate and highly sensitive amplification in comparison to normal primer sets.

### Table 12: ncRNAs used by the methods of the present invention.

Enlisted ncRNAs and their precursors have been identified in experiments provided herein as "DS-specific" in samples of three different RNA-pools - in a first comparison, the pools comprised six DS-pregnancies against six corresponding controls (matched in respect to maternal age, fetal sex and week of gestation). The second comparison examined six DS samples with 24 healthy pregnancies, that are more heterogenic in respect to maternal age, fetal sex and week of gestation, in order to obtain a more reliable and robust comparison. The sequences of the respective miRNAs and precursors thereof might be obtained as well from the miRBase data repository by the use of the indicated sequence IDs and Accession Nos. Sequence of small nuclear RNU12 RNA may be obtained from National Center for Biotechnology Information (NCBI) by the use of the indicated Accession NOs.

miRNAs indicated as "Sex specific" may be used according to the methods of the present invention preferably in the determination of the fetal gender. "Reference-miRNAs" are controls showing no differential expression in different comparisons (not significantly altered in respect to DS and/or fetal gender). "not in plasma controls" are miRNAs which usually do not occur in human plasma samples and were investigated via HT-qPCR but have not been identified in any of the 14 samples examined. Expressional change indicates whether the expression of the respective miRNA was up- (up) or down-regulated (down) in the sample from a DS-pregnancy compared to a healthy control sample. Concerning miRNAs designated as gender specific, differential expression indicates whether the expression of the respective miRNA was up or down-regulated in the male fetus in comparison to a female fetus. Letter "W" in the Study column - stands for the Wafergen HT-qPCR study.

| **No** | **Study** | **Affiliation** | Identified miRNAs **// Mature Sequence ID /** Previous IDs | **Accession No. / Mature miRNA Sequence / SEQ ID NO:** | **Expressional change** | **Accession No. Pre-miRNA / Pre-miRNA (Stem-loop-)Sequence / SEQ ID NO:** |
|---|---|---|---|---|---|---|
| **1** | W | DS Specific | HSA-MIR-589* // **hsa-miR-589-3P** / hsa-miR-589; hsa-miR-589* | MIMAT0003256 UCAGAACAAAUGCCGGUUCCCAGA SEQ ID NO: 1 | Up (in DS) | MI0003599 UCCAGCCUGUGCCCAGCAGCCCCUGAGAACCACGUCU GCUCUGAGCUGGGUACUGCCUGUUCAGAACAAAUGCC GGUUCCCAGACGCUGCCAGCUGGCC SEQ ID NO: 82 |
| **2** | W | DS Specific | HSA-MIR-512-5P // **HSA-MIR-512-5P** / none | MIMAT0002822 CACUCAGCCUUGAGGGCACUUUC SEQ ID NO: 2 | Up | MI0003140 UCUCAGUCUGUGGCACUCAGCCUUGAGGGCACUUUCU GGUGCCAGAAUGAAAGUGCUGUCAUAGCUGAGGUCCA AUGACUGAGG                SEQ ID NO: 83 MI0003141 GGUACUUCUCAGUCUGUGGCACUCAGCCUUGAGGGCA CUUUCUGGUGCCAGAAUGAAAGUGCUGUCAUAGCUGA GGUCCAAUGACUGAGGCGAGCACC SEQ ID NO: 84 |
| **3** | W | DS Specific | HSA-MIR-181A^{*} // **hsa-miR-181a-3p** / hsa-miR-213; hsa-miR-181a* | MIMAT0000270 ACCAUCGACCGUUGAUUGUACC SEQ ID NO: 3 | Down | MI0000269 AGAAGGGCUAUCAGGCCAGCCUUCAGAGGACUCCAAG GAACAUUCAACGCUGUCGGUGAGUUUGGGAUUUGAAA AAACCACUGACCGUUGACUGUACCUUGGGGUCCUUA SEQ ID NO: 85 |
| **4** | W | DS Specific | HSA-MIR-483-5P // **HSA-MIR-483-5P** / none | MIMAT0004761 AAGACGGGAGGAAAGAAGGGAG SEQ ID NO: 4 | Up | MI0002467 GAGGGGAAGACGGGAGGAAAGAAGGGAGUGGUUCCA UCACGCCUCCUCACUCCUCUCCUCCCGUCUUCUCCUC UC                    SEQ ID NO: 86 |
| **5** | W | DS Specific | HSA-MIR-362-5P // **HSA-MIR-362-5P** / hsa-miR-362 | MIMAT0000705 AAUCCUUGGAACCUAGGUGUGAGU SEQ ID NO: 5 | Up | MI0000762 CUUGAAUCCUUGGAACCUAGGUGUGAGUGCUAUUUCA GUGCAACACACCUAUUCAAGGAUUCAAA SEQ ID NO: 87 |
| **6** | W | DS Specific | HSA-MIR-518C^{*} // **HSA-MIR-518c-5P** / HSA-MIR-518C^{*} | MIMAT0002847 UCUCUGGAGGGAAGCACUUUCUG SEQ ID NO: 6 | Up | MI0003159 GCGAGAAGAUCUCAUGCUGUGACUCUCUGGAGGGAAG CACUUUCUGUUGUCUGAAAGAAAACAAAGCGCUUCUC UUUAGAGUGUUACGGUUUGAGAAAAGC SEQ ID NO: 88 |
| **7** | W | DS Specific | HSA-MIR-3667-5P // **HSA-MIR-3667-5P** / none | MIMAT0018089 AAAGACCCAUUGAGGAGAAGGU SEQ ID NO: 7 | Up | MI0016068 UGAGGAUGAAAGACCCAUUGAGGAGAAGGUUCUGCUG GCUGAGAACCUUCCUCUCCAUGGGUCUUUCAUCCUCA SEQ ID NO: 89 |
| **8** | W | DS Specific | HSA-MIR-195* // **HSA-MIR-195-3P** / HSA-MIR-195* | MIMAT0004615 CCAAUAUUGGCUGUGCUGCUCC SEQ ID NO: 8 | Up | MI0000489 AGCUUCCCUGGCUCUAGCAGCACAGAAAUAUUGGCAC AGGGAAGCGAGUCUGCCAAUAUUGGCUGUGCUGCUCC AGGCAGGGUGGUG          SEQ ID NO: 90 |
| **9** | W | DS Specific | HSA-MIR-124* // **HSA-MIR-124-5P** / HSA-MIR-124* | MIMAT0004591 CGUGUUCACAGCGGACCUUGAU SEQ ID NO: 9 | Up | MI0000443 AGGCCUCUCUCUCCGUGUUCACAGCGGACCUUGAUUU AAAUGUCCAUACAAUUAAGGCACGCGGUGAAUGCCAA GAAUGGGGCUG                SEQ ID NO: 91 MI0000444 AUCAAGAUUAGAGGCUCUGCUCUCCGUGUUCACAGCG GACCUUGAUUUAAUGUCAUACAAUUAAGGCACGCGGU GAAUGCCAAGAGCGGAGCCUACGGCUGCACUUGAA SEQ ID NO: 92 MI0000445 UGAGGGCCCCUCUGCGUGUUCACAGCGGACCUUGAUU UAAUGUCUAUACAAUUAAGGCACGCGGUGAAUGCCAA GAGAGGCGCCUCC              SEQ ID NO: 93 |
| **10** | W | DS Specific | HSA-MIR-498 // **HSA-MIR-498** / none | MIMAT0002824 UUUCAAGCCAGGGGGCGUUUUUC SEQ ID NO: 10 | Down | MI0003142 AACCCUCCUUGGGAAGUGAAGCUCAGGCUGUGAUUUC AAGCCAGGGGGCGUUUUUCUAUAACUGGAUGAAAAGC ACCUCCAGAGCUUGAAGCUCACAGUUUGAGAGCAAUC GUCUAAGGAAGUU SEQ ID NO: 94 |
| **11** | W | DS Specific | HSA-MIR-18B* // **HSA-MIR-18B-3P** / HSA-MIR-18B* | MIMAT0004751 UGCCCUAAAUGCCCCUUCUGGC SEQ ID NO: 11 | Up | MI0001518 UGUGUUAAGGUGCAUCUAGUGCAGUUAGUGAAGCAGC UUAGAAUCUACUGCCCUAAAUGCCCCUUCUGGCA SEQ ID NO: 95 |
| **12** | W | DS Specific | HSA-MIR-888* // **HSA-MIR-888-3P** / HSA-MIR-888* | MIMAT0004917 GACUGACACCUCUUUGGGUGAA SEQ ID NO: 12 | Up | MI0005537 GGCAGUGCUCUACUCAAAAAGCUGUCAGUCACUUAGA UUACAUGUGACUGACACCUCUUUGGGUGAAGGAAGGC UCA SEQ ID NO: 96 |
| **13** | W | DS Specific | HSA-MIR-3676 // **HSA-MIR-3676-3P/** HSA-MIR-3676 | MIMAT0022734 AGGAGAUCCUGGGUU SEQ ID NO: 13 | Down | MI0016077 UUGGUUAAAGCGCCUGUCUAGUAAACAGGAGAUCCUG GGUUCGAAUCCCAGCGGUGCCUCCGUGUUUCCCCCAC GCUUUUGCCAA                SEQ ID NO: 97 |
| **14** | W | DS Specific | HSA-MIR-3918 // **HSA-MIR-3918/** none | MIMAT0018192 ACAGGGCCGCAGAUGGAGACU SEQ ID NO: 14 | Up | MI0016424 AGGCGGUUAAGCCAUGGACAGGGCCGCAGAUGGAGA CUGCUCAAGGUCAAAGGGGUCUCCAGCUGGGACCCUG CACCUGGUUCGUAGCCCCU     SEQ ID NO: 98 |
| **15** | W | DS Specific | HSA-MIR-500A // **HSA-MIR-500A-5P/** HSA-MIR-500; HSA-MIR-500A | MIMAT0004773 UAAUCCUUGCUACCUGGGUGAGA SEQ ID NO: 15 | Up | MI0003184 GCUCCCCCUCUCUAAUCCUUGCUACCUGGGUGAGAGU GCUGUCUGAAUGCAAUGCACCUGGGCAAGGAUUCUGA GAGCGAGAGC SEQ ID NO: 99 |
| **16** | W | DS Specific | RNU12 (small nuclear RNA) // none /none Gene ID: 267010 NCBI Ref.Seq: NR 029422.1 | TGCCTTAAACTTATGAGTAAGGAAAA TAACGATTCGGGGTGACGCCCGAATC CTCACTGCTAATGTGAGACGAATTTT TGAGCGGGTAAAGGTCGCCCTCAAGG TGACCCGCCTACTTTGCGGGATGCCT GGGAGTTGCGATCTGCCCG SEQ ID NO: 16 | Up | none / none |
| **17** | W | DS Specific | HSA-MIR-30C // **HSA-MIR-30C-5P** / HSA-MIR-30C | MIMAT0000244 UGUAAACAUCCUACACUCUCAGC SEQ ID NO: 17 | Up | MI0000254 AGAUACUGUAAACAUCCUACACUCUCAGCUGUGGAAA GUAAGAAAGCUGGGAGAAGGCUGUUUACUCUUUCU SEQ ID NO: 100 MI0000736 ACCAUGCUGUAGUGUGUGUAAACAUCCUACACUCUCA GCUGUGAGCUCAAGGUGGCUGGGAGAGGGUUGUUUAC UCCUUCUGCCAUGGA          SEQ ID NO: 101 |
| **18** | W | DS Specific | HSA-MIR-200C // **HSA-MIR-200C-3P** / HSA-MIR-200C | MIMAT0004657 CGUCUUACCCAGCAGUGUUUGG SEQ ID NO: 18 | Down | MI0000650 CCCUCGUCUUACCCAGCAGUGUUUGGGUGCGGUUGGG AGUCUCUAAUACUGCCGGGUAAUGAUGGAGG SEQ ID NO: 102 |
| **19** | W | DS Specific | HSA-MIR-627 // **HSA-MIR-627** / none | MIMAT0003296 GUGAGUCUCUAAGAAAAGAGGA SEQ ID NO: 19 | Down | MI0003641 UACUUAUUACUGGUAGUGAGUCUCUAAGAAAAGAGGA GGUGGUUGUUUUCCUCCUCUUUUCUUUGAGACUCACU ACCAAUAAUAAGAAAUACUACUA SEQ ID NO: 103 |
| **20** | W | DS Specific | HSA-MIR-93 // **HSA-MIR-93-5P** / HSA-MIR-93 | MIMAT0000093 CAAAGUGCUGUUCGUGCAGGUAG SEQ ID NO: 20 | Down | MI0000095 CUGGGGGCUCCAAAGUGCUGUUCGUGCAGGUAGUGUG AUUACCCAACCUACUGCUGAGCUAGCACUUCCCGAGC CCCCGG                    SEQ ID NO: 104 |
| **21** | W | DS Specific | HSA-MIR-20A // **HSA-MIR-20A-5P** / HSA-MIR-20; HSA-MIR-20A | MIMAT0000075 UAAAGUGCUUAUAGUGCAGGUAG SEQ ID NO: 21 | Down | MI0000076 GUAGCACUAAAGUGCUUAUAGUGCAGGUAGUGUUUAG UUAUCUACUGCAUUAUGAGCACUUAAAGUACUGC SEQ ID NO: 105 |
| **22** | W | DS Specific | HSA-MIR-4328 // **HSA-MIR-4328** / none | MIMAT0016926 CCAGUUUUCCCAGGAUU SEQ ID NO: 22 | Down | MI0015904 AACAGUUGAGUCCUGAGAACCAUUGAGAACCAGUUUU CCCAGGAUUAACUGUUCCG    SEQ ID NO: 106 |
| 23 | W | DS Specific | HSA-MIR-92A // HSA-MIR-**92a-1-5P** / HSA-MIR-92; HSA-MIR-92A | MIMAT0000092 UAUUGCACUUGUCCCGGCCUGU SEQ ID NO: 23 | Down | MI0000093 CUUUCUACACAGGUUGGGAUCGGUUGCAAUGCUGUGU UUCUGUAUGGUAUUGCACUUGUCCCGGCCUGUUGAGU UUGG                    SEQ ID NO: 107 |
| **24** | W | DS Specific | HSA-MIR-92B // **HSA-MIR-92b-3P** / HSA-MIR-92B | MIMAT0003218 UAUUGCACUCGUCCCGGCCUCC SEQ ID NO: 24 | Down | MI0003560 CGGGCCCCGGGCGGGCGGGAGGGACGGACGCGGUGC AGUGUUGUUUUUUCCCCCGCCAAUAUUGCACUCGUCC CGGCCUCCGGCCCCCCCGGCCC SEQ ID NO: 108 |
| **25** | W | DS Specific | HSA-MIR-3652 // **HSA-MIR-3652** / none | MIMAT0018072 CGGCUGGAGGUGUGAGGA SEQ ID NO: 25 | Up | MI0016052 CGGCUGGAGGUGUGAGGAUCCGAACCCAGGGGUGGGG GGUGGAGGCGGCUCCUGCGAUCGAAGGGGACUUGAGA CUCACCGGCCGCACGCCAUGAGGGCCCUGUGGGUGCU GGGCCUCUGCUGCGUCCUGC    SEQ ID NO: 109 |
| **26** | W | DS Specific | HSA-MIR-25 // **HSA-MIR-25-3P** / HSA-MIR-25 | MIMAT0000081 CAUUGCACUUGUCUCGGUCUGA SEQ ID NO: 26 | Down | MI0000082 GGCCAGUGUUGAGAGGCGGAGACUUGGGCAAUUGCUG GACGCUGCCCUGGGCAUUGCACUUGUCUCGGUCUGAC AGUGCCGGCC SEQ ID NO: 110 |
| **27** | W | DS Specific | HSA-MIR-623 // **HSA-MIR-623** / none | MIMAT0003292 AUCCCUUGCAGGGCUGUUGGGU SEQ ID NO: 27 | Down | MI0003637 GUACACAGUAGAAGCAUCCCUUGCAGGGGCUGUUGGG UUGCAUCCUAAGCUGUGCUGGAGCUUCCCGAUGUACU CUGUAGAUGUCUUUGCACCUUCUG SEQ ID NO: 111 |
| **28** | W | DS Specific | HSA-MIR-3195 // **HSA-MIR-3195** / none | MIMAT0015079 CGCGCCGGGCCCGGUU SEQ ID NO: 28 | Up | MI0014240 CCGCAGCCGCCGCGCCGGGCCCGGGUUGGCCGCUGAC CCCCGCGGGGCCCCCGGCGGCCGGGGCGGGGGCGGGG GCUGCCCGG SEQ ID NO: 112 |
| **29** | W | DS Specific | HSA-MIR-423-5P // **HSA-MIR-423-5P** / none | MIMAT0004748 UGAGGGCAGAGAGCGAGACUUU SEQ ID NO: 29 | Down | MI0001445 AUAAAGGAAGUUAGGCUGAGGGGCAGAGAGCGAGACU UUUCUAUUUUCCAAAAGCUCGGUCUGAGGCCCCUCAG UCUUGCUUCCUAACCCGCGC    SEQ ID NO: 113 |
| **30** | W | DS Specific | HSA-MIR-27B // **HSA-MIR-27B-3P** / HSA-MIR-27B | MIMAT0000419 UUCACAGUGGCUAAGUUCUGC SEQ ID NO: 30 | UP | MI0000440 ACCUCUCUAACAAGGUGCAGAGCUUAGCUGAUUGGUG AACAGUGAUUGGUUUCCGCUUUGUUCACAGUGGCUAA GUUCUGCACCUGAAGAGAAGGUG SEQ ID NO: 114 |
| **31** | W | DS Specific | HSA-MIR-3135 // **HSA-MIR-3135a** / HSA-MIR-3135 | MIMAT0015001 UGCCUAGGCUGAGACUGCAGUG SEQ ID NO: 31 | Up | MI0014156 UCACUUUGGUGCCUAGGCUGAGACUGCAGUGGUGCAA UCUCAGUUCACUGCAGCCUUGACCUCCUGGGCUCAGG UGA                    SEQ ID NO: 115 |
| **32** | W | DS Specific | HSA-MIR-486-5P // **HSA-MIR-486-5P** / HSA-MIR-486 | MIMAT0002177 UCCUGUACUGAGCUGCCCCGAG SEQ ID NO: 32 | Down | MI0002470 GCAUCCUGUACUGAGCUGCCCCGAGGCCCUUCAUGCU GCCCAGCUCGGGGCAGCUCAGUACAGGAUAC SEQ ID NO: 116 |
| **33** | W | DS Specific | HSA-MIR-3180-5P // **HSA-MIR-3180-5P** / none | MIMAT0015057 CUUCCAGACGCUCCGCCCCACGUCG SEQ ID NO: 33 | Down | MI0014214 CAGUGCGACGGGCGGAGCUUCCAGACGCUCCGCCCCA CGUCGCAUGCGCCCCGGGAAAGCGUGGGGCGGAGCUU CCGGAGGCCCCGCCCUGCUG   SEQ ID NO: 117 MI0014215 GCGACGGGCGGAGCUUCCAGACGCUCCGCCCCACGUC GCAUGCGCCCCGGGAAAGCGUGGGGCGGAGCUUCCGG AGGCCCCGCCCUGC         SEQ ID NO: 118 MI0014217 CAGUGCGACGGGCGGAGCUUCCAGACGCUCCGCCCCA CGUCGCAUGCGCCCCGGGAAAGCGUGGGGCGGAGCUU CCGGAGGCCCCGCCCUGCUG   SEQ ID NO: 119 |
| **34** | W | DS Specific | HSA-MIR-629 // **HSA-MIR-629-5P** / HSA-MIR-629 | MIMAT0004810 UGGGUUUACGUUGGGAGAACU SEQ ID NO: 34 | Down | MI0003643 UCCCUUUCCCAGGGGAGGGGCUGGGUUUACGUUGGGA GAACUUUUACGGUGAACCAGGAGGUUCUCCCAACGUA AGCCCAGCCCCUCCCCUCUGCCU SEQ ID NO: 120 |
| **35** | W | DS Specific | HSA-MIR-629 // **HSA-MIR-629-3P** / HSA-MIR-629; HSA-MIR-629* | MIMAT0003298 GUUCUCCCAACGUAAGCCCAGC SEQ ID NO: 35 | Down | MI0003643 UCCCUUUCCCAGGGGAGGGGCUGGGUUUACGUUGGGA GAACUUUUACGGUGAACCAGGAGGUUCUCCCAACGUA AGCCCAGCCCCUCCCCUCUGCCU SEQ ID NO: 121 |
| **36** | W | DS Specific | HSA-MIR-155 // **HSA-MIR-155-5P** / HSA-MIR-155 | MIMAT0000646 UUAAUGCUAAUCGUGAUAGGGGU SEQ ID NO: 36 | Down | MI0000681 CUGUUAAUGCUAAUCGUGAUAGGGGUUUUUGCCUCCA ACUGACUCCUACAUAUUAGCAUUAACAG SEQ ID NO: 122 |
| **37** | W | DS Specific | HSA-MIR-3907 // **HSA-MIR-3907** / none | MIMAT0018179 AGGUGCUCCAGGCUGGCUCACA SEQ ID NO: 37 | Down | MI0016410 GGGUUGGAAAGCUGUAGGUGUGGAGGGGCAUGGAUAC GGGGGCCAUGAGGGUGGGGUCCAGGCUGGACCAGGCC UGCCCUGAGUCCCCCAGCAGGUGCUCCAGGCUGGCUC ACACCCUCUGCCUCUCUCUCUUCCUUCCUGGCCCCAA CCC SEQ ID NO: 123 |
| **38** | W | DS and Sex Specific | HSA-MIR-1249 // **HSA-MIR-1249** / none | MIMAT0005901 ACGCCCUUCCCCCCCUUCUUCA SEQ ID NO: 38 | Up in Ds Down in men | MI0006384 GGGAGGAGGGAGGAGAUGGGCCAAGUUCCCUCUGGCU GGAACGCCCUUCCCCCCCUUCUUCACCUG SEQ ID NO: 124 |
| **39** | W | DS Specific | HSA-MIR-215 | MIMAT0000272 AUGACCUAUGAAUUGACAGAC SEQ ID NO: 39 | Up | MI0000291 AUCAUUCAGAAAUGGUAUACAGGAAAAUGACCUAUGA AUUGACAGACAAUAUAGCUGAGUUUGUCUGUCAUUUC UUUAGGCCAAUAUUCUGUAUGACUGUGCUACUUCAA SEQ ID NO: 125 |
| **40** | W | DS Specific | HSA-MIR-1227 // HSA-MIR-1227-3P | MIMAT0005580 CGUGCCACCCUUUUCCCCAG SEQ ID NO: 40 | Up | MI0006316 GUGGGGCCAGGCGGUGGUGGGCACUGCUGGGGUGGGC ACAGCAGCCAUGCAGAGCGGGCAUUUGACCCCGUGCC ACCCUUUUCCCCAG          SEQ ID NO: 126 |
| **41** | W | DS Specific | HSA-MIR-187 // HSA-MIR-187-3P | MIMAT0000262 UCGUGUCUUGUGUUGCAGCCGG SEQ ID NO: 41 | Up | MI0000274 GGUCGGGCUCACCAUGACACAGUGUGAGACCUCGGGC UACAACACAGGACCCGGGCGCUGCUCUGACCCCUCGU GUCUUGUGUUGCAGCCGGAGGGACGCAGGUCCGCA SEQ ID NO: 127 |
| **42** | W | DS Specific | HSA-MIR-219-5P | MIMAT0000276 UGAUUGUCCAAACGCAAUUCU SEQ ID NO: 42 | Up | MI0000740 ACUCAGGGGCUUCGCCACUGAUUGUCCAAACGCAAUU CUUGUACGAGUCUGCGGCCAACCGAGAAUUGUGGCUG GACAUCUGUGGCUGAGCUCCGGG SEQ ID NO: 128 MI0000296 CCGCCCCGGGCCGCGGCUCCUGAUUGUCCAAACGCAA UUCUCGAGUCUAUGGCUCCGGCCGAGAGUUGAGUCUG GACGUCCCGAGCCGCCGCCCCCAAACCUCGAGCGGG SEQ ID NO: 129 |
| **43** | W | DS Specific | HSA-MIR-3913 // HSA-MIR-3913-5P | MIMAT0018187 UUUGGGACUGAUCUUGAUGUCU SEQ ID NO: 43 | Up | MI0016417 UUGUUUAUAAUAAACUGAAAUAUUUGGGACUGAUCUU GAUGUCUGCCAAAACCUUGGCAGACAUCAAGAUCAGU CCCAAAUAUUUCAGUUUAUUAUAGACAG SEQ ID NO: 130 MI0016418 UGUCUAUAAUAAACUGAAAUAUUUGGGACUGAUCUUG AUGUCUGCCAAGGUUUUGGCAGACAUCAAGAUCAGUC CCAAAUAUUUCAGUUUAUUAUAAACA SEQ ID NO: 131 MIMAT0018187 UUUGGGACUGAUCUUGAUGUCU SEQ ID NO: 132 |
| **44** | W | Sex Specific | HSA-MIR-590-3P // **HSA-MIR-590-3P** / none | MIMAT0004801 UAAUUUUAUGUAUAAGCUAGU SEQ ID NO: 44 | Down (in men) | MI0003602 UAGCCAGUCAGAAAUGAGCUUAUUCAUAAAAGUGCAG UAUGGUGAAGUCAAUCUGUAAUUUUAUGUAUAAGCUA GUCUCUGAUUGAAACAUGCAGCA SEQ ID NO: 133 |
| **45** | W | Sex Specific | HSA-MIR-3923 // **HSA-MIR-3923** / none | MIMAT0018198 AACUAGUAAUGUUGGAUUAGGG SEQ ID NO: 45 | Down | MI0016430 GGUAGAGUGAGCUCUAAUCCAAUAUUACUAGCUUCUU UAUAAGAAGAGGAAACUAGUAAUGUUGGAUUAGGGCU CACUCUACU SEQ ID NO: 134 |
| **46** | W | Sex Specific | HSA-MIR-1228* // **HSA-MIR-1228-5P** / HSA-MIR-1228* | MIMAT0005582 GUGGGCGGGGGCAGGUGUGUG SEQ ID NO: 46 | Down | MI0006318 GUGGGCGGGGGCAGGUGUGUGGUGGGUGGUGGCCUGC GGUGAGCAGGGCCCUCACACCUGCCUCGCCCCCCAG SEQ ID NO: 135 |
| **47** | W | Sex Specific | HSA-MIR-769-3P // **HSA-MIR-769-3P** / none | MIMAT0003887 CUGGGAUCUCCGGGGUCUUGGUU SEQ ID NO: 47 | Up | MI0003834 GCCUUGGUGCUGAUUCCUGGGCUCUGACCUGAGACCU CUGGGUUCUGAGCUGUGAUGUUGCUCUCGAGCUGGGA UCUCCGGGGUCUUGGUUCAGGGCCGGGCCUCUGGGU UCCAAGC SEQ ID NO: 136 |
| **48** | W | Sex Specific | HSA-MIR-127-5P // **HSA-MIR-127-5P** / none | MIMAT0004604 CUGAAGCUCAGAGGGCUCUGAU SEQ ID NO: 48 | Down | MI0000472 UGUGAUCACUGUCUCCAGCCUGCUGAAGCUCAGAGGG CUCUGAUUCAGAAAGAUCAUCGGAUCCGUCUGAGCUU GGCUGGUCGGAAGUCUCAUCAUC SEQ ID NO: 137 |
| **49** | W | Sex Specific | HSA-MIR-1293 // **HSA-MIR-1293** / none | MIMAT0005883 UGGGUGGUCUGGAGAUUUGUGC SEQ ID NO: 49 | Down | MI0006355 AGGUUGUUCUGGGUGGUCUGGAGAUUUGUGCAGCUUG UACCUGCACAAAUCUCCGGACCACUUAGUCUUUA SEQ ID NO: 138 |
| **50** | W | Sex Specific | HSA-LET-7F-1* // hsa-let-7f-1-3p / hsa-let-7f-1* | MIMAT0004486 CUAUACAAUCUAUUGCCUUCCC SEQ ID NO: 50 | Down | MI0000067 UCAGAGUGAGGUAGUAGAUUGUAUAGUUGUGGGGUAG UGAUUUUACCCUGUUCAGGAGAUAACUAUACAAUCUA UUGCCUUCCCUGA          SEQ ID NO: 139 |
| **51** | W | Sex Specific | HSA-MIR-93* // **HSA-MIR-93-3P** / HSA-MIR-93* | MIMAT0004509 ACUGCUGAGCUAGCACUUCCCG SEQ ID NO: 51 | Down | MI0000095 CUGGGGGCUCCAAAGUGCUGUUCGUGCAGGUAGUGUG AUUACCCAACCUACUGCUGAGCUAGCACUUCCCGAGC CCCCGG SEQ ID NO: 140 |
| **52** | W | Sex Specific | HSA-MIR-892B // **HSA-MIR-**892B / none | MIMAT0004918 CACUGGCUCCUUUCUGGGUAGA SEQ ID NO: 52 | Down | MI0005538 UGCAAUGCCCUACUCAGAAAGGUGCCAUUUAUGUAGA UUUUAUGUCACUGGCUCCUUUCUGGGUAGAGCAAGGC UCA SEQ ID NO: 141 |
| **53** | W | Sex Specific | HSA-MIR-320B // **HSA-MIR-320B** / none | MIMAT0005792 AAAAGCUGGGUUGAGAGGGCAA SEQ ID NO: 53 | Up | MI0003776 AAUUAAUCCCUCUCUUUCUAGUUCUUCCUAGAGUGAG GAAAAGCUGGGUUGAGAGGGCAAACAAAUUAACUAAU UAAUU                    SEQ ID NO: 142 MI0003839 / UGUUAUUUUUUGUCUUCUACCUAAGAAUUCUGUCUCU UAGGCUUUCUCUUCCCAGAUUUCCCAAAGUUGGGAAA AGCUGGGUUGAGAGGGCAAAAGGAAAAAAAAAGAAUU CUGUCUCUGACAUAAUUAGAUAGGGAA SEQ ID NO: 143 |
| **54** | W | Sex Specific | HSA-MIR-106A* // **HSA-MIR-106a-3P** | MIMAT0004517 CUGCAAUGUAAGCACUUCUUAC SEQ ID NO: 54 | Up | MI0000113 CCUUGGCCAUGUAAAAGUGCUUACAGUGCAGGUAGCU UUUUGAGAUCUACUGCAAUGUAAGCACUUCUUACAUU ACCAUGG SEQ ID NO: 144 |
| **55** | W | Sex Specific | HSA-MIR-92B* // **HSA-MIR-92B-5P** | MIMAT0004792 AGGGACGGGACGCGGUGCAGUG SEQ ID NO: 55 | down | MI0003560 CGGGCCCCGGGCGGGCGGGAGGGACGGGACGCGGUGC AGUGUUGUUUUUUCCCCCGCCAAUAUUGCACUCGUCC CGGCCUCCGGCCCCCCCGGCCC SEQ ID NO: 145 |
| **56** | W | Sex Specific | HSA-MIR-328 | MIMAT0000752 CUGGCCCUCUCUGCCCUUCCGU SEQ ID NO: 56 | down | MI0000804 UGGAGUGGGGGGCAGGAGGGGCUCAGGGAGAAAGUG CAUACAGCCCCUGGCCCUCUCUGCCCUUCCGUCCCCU G                    SEQ ID NO: 146 |
| **57** | W | Sex Specific | HSA-MIR-196B* // **HSA-MIR-196B-3P** | MIMAT0009201 UCGACAGCACGACACUGCCUUC SEQ ID NO: 57 | down | MI0001150 ACUGGUCGGUGAUUUAGGUAGUUUCCUGUUGUUGGGA UCCACCUUUCUCUCGACAGCACGACACUGCCUUCAUU ACUUCAGUUG SEQ ID NO: 147 |
| **58** | W | Sex Specific | HSA-MIR-548C-5P | MIMAT0004806 AAAAGUAAUUGCGGUUUUUGCC SEQ ID NO: 58 | down | MI0003630 CAUUGGCAUCUAUUAGGUUGGUGCAAAAGUAAUUGCG GUUUUUGCCAUUACUUUCAGUAGCAAAAAUCUCAAUU ACUUUUGCACCAACUUAAUACUU SEQ ID NO: 148 |
| **59** | W | Sex Specific | HSA-MIR-512-3P | MIMAT0002823 AAGUGCUGUCAUAGCUGAGGUC SEQ ID NO: 59 | up | MI0003140 UCUCAGUCUGUGGCACUCAGCCUUGAGGGCACUUUCU GGUGCCAGAAUGAAAGUGCUGUCAUAGCUGAGGUCCA AUGACUGAGG SEQ ID NO: 149 MI0003141 GGUACUUCUCAGUCUGUGGCACUCAGCCUUGAGGGCA CUUUCUGGUGCCAGAAUGAAAGUGCUGUCAUAGCUGA GGUCCAAUGACUGAGGCGAGCACC SEQ ID NO: 150 |
| **60** | W | Sex Specific | HSA-MIR-4306 | MIMAT0016858 UGGAGAGAAAGGCAGUA SEQ ID NO: 60 | down | MI0015836 AAGCUGCUUAGUGUCCUUAGAGUCUCCAGAGGCAUCC CUAACCCAGAAUCUUUUGACUGUCCUCUGGAGAGAAA GGCAGUAGGUCUGUACC        SEQ ID NO: 151 |
| **61** | W | Sex Specific | HSA-MIR-29B-1-// **HSA-MIR-29B-1-5P** | MIMAT0004514 GCUGGUUUCAUAUGGUGGUUUAGA SEQ ID NO: 61 | down | MI0000105 CUUCAGGAAGCUGGUUUCAUAUGGUGGUUUAGAUUUA AAUAGUGAUUGUCUAGCACCAUUUGAAAUCAGUGUUC UUGGGGG                    SEQ ID NO: 152 |
| **62** | W | Sex Specific | HSA-MIR-30C-1*// **HSA-MIR-30C-1-3P** | MIMAT0004674 CUGGGAGAGGGUUGUUUACUCC SEQ ID NO: 62 | down | MI0000736 ACCAUGCUGUAGUGUGUGUAAACAUCCUACACUCUCA GCUGUGAGCUCAAGGUGGCUGGGAGAGGGUUGUUUAC UCCUUCUGCCAUGGA          SEQ ID NO: 153 |
| **63** | W | Reference miRNAs | HSA-MIR-101 // **HSA-MIR-101-3P** / HSA-MIR-101 | MIMAT0000099 UACAGUACUGUGAUAACUGAA SEQ ID NO: 63 | Not different ially expressed | MI0000103 UGCCCUGGCUCAGUUAUCACAGUGCUGAUGCUGUCUA UUCUAAAGGUACAGUACUGUGAUAACUGAAGGAUGGC A                         SEQ ID NO: 154 MI0000739 ACUGUCCUUUUUCGGUUAUCAUGGUACCGAUGCUGUA UAUCUGAAAGGUACAGUACUGUGAUAACUGAAGAAUG GUGGU                     SEQ ID NO: 155 |
| **64** | W | Reference miRNAs | HSA-MIR-103 // **HSA-MIR-103a-3P** / HSA-MIR-103; HSA-MIR-103a | MIMAT0007402 UCAUAGCCCUGUACAAUGCUGCU SEQ ID NO: 64 | Not different ially expressed | MI0000108 UUGUGCUUUCAGCUUCUUUACAGUGCUGCCUUGUAGC AUUCAGGUCAAGCAGCAUUGUACAGGGCUAUGAAAGA ACCA                    SEQ ID NO: 156 MI0000109 UACUGCCCUCGGCUUCUUUACAGUGCUGCCUUGUUGC AUAUGGAUCAAGCAGCAUUGUACAGGGCUAUGAAGGC AUUG                    SEQ ID NO: 157 |
| **65** | W | Reference miRNAs | HSA-MIR-16 // **HSA-MIR-16-5P** / HSA-MIR-16 | MIMAT0000069 UAGCAGCACGUAAAUAUUGGCG SEQ ID NO: 65 | Not different ially expressed | MI0000070 GUCAGCAGUGCCUUAGCAGCACGUAAAUAUUGGCGUU AAGAUUCUAAAAUUAUCUCCAGUAUUAACUGUGCUGC UGAAGUAAGGUUGAC          SEQ ID NO: 158 MI0000115 GUUCCACUCUAGCAGCACGUAAAUAUUGGCGUAGUGA AAUAUAUAUUAAACACCAAUAUUACUGUGCUGCUUUA GUGUGAC                  SEQ ID NO: 159 |
| **66** | W | Reference miRNAs | HSA-MIR-185 // **HSA-MIR-185-5P** / HSA-MIR-185 | MIMAT0000455 UGGAGAGAAAGGCAGUUCCUGA SEQ ID NO: 66 | Not different ially expressed | MI0000482 AGGGGGCGAGGGAUUGGAGAGAAAGGCAGUUCCUGAU GGUCCCCUCCCCAGGGGCUGGCUUUCCUCUGGUCCUU CCCUCCCA SEQ ID NO: 160 |
| **67** | W | Reference miRNAs | HSA-MIR-223 // **HSA-MIR-223-3P** / HSA-MIR-223 | MIMAT0000280 UGUCAGUUUGUCAAAUACCCCA SEQ ID NO: 67 | Not different ially expressed | MI0000300 CCUGGCCUCCUGCAGUGCCACGCUCCGUGUAUUUGAC AAGCUGAGUUGGACACUCCAUGUGGUAGAGUGUCAGU UUGUCAAAUACCCCAAGUGCGGCACAUGCUUACCAG SEQ ID NO: 161 |
| **68** | W | Reference miRNAs | HSA-MIR-378 // **HSA-MIR-378-5P** / HSA-MIR-378; HSA-MIR-378* | MIMAT0000732 ACUGGACUUGGAGUCAGAAGG SEQ ID NO: 68 | Not different ially expressed | MI0000786 AGGGCUCCUGACUCCAGGUCCUGUGUGUUACCUAGAA AUAGCACUGGACUUGGAGUCAGAAGGCCU SEQ ID NO: 162 |
| **69** | W | Reference miRNAs | HSA-MIR-378 // **HSA-MIR-378-3P** / HSA-MIR-422b; HSA-MIR-378 | MIMAT0000732 ACUGGACUUGGAGUCAGAAGG SEQ ID NO: 69 | Not different ially expressed | MI0000786 AGGGCUCCUGACUCCAGGUCCUGUGUGUUACCUAGAA AUAGCACUGGACUUGGAGUCAGAAGGCCU SEQ ID NO: 163 |
| **70** | W | Reference miRNAs | HSA-MIR-451 // **HSA-MIR-451a** / HSA-MIR-451 | MIMAT0001631 AAACCGUUACCAUUACUGAGUU SEQ ID NO: 70 | Not different ially expressed | MI0001729 CUUGGGAAUGGCAAGGAAACCGUUACCAUUACUGAGU UUAGUAAUGGUAAUGGUUCUCUUGCUAUACCCAGA SEQ ID NO: 164 |
| **71** | W | Reference miRNAs | HSA-LET-7A // **HSA-LET-7A-5P** / HSA-LET-7A | MIMAT0000062 UGAGGUAGUAGGUUGUAUAGUU SEQ ID NO: 71 | Not different ially expressed | MI0000060 UGGGAUGAGGUAGUAGGUUGUAUAGUUUUAGGGUCAC ACCCACCACUGGGAGAUAACUAUACAAUCUACUGUCU UUCCUA                    SEQ ID NO: 165 MI0000061 AGGUUGAGGUAGUAGGUUGUAUAGUUUAGAAUUACAU CAAGGGAGAUAACUGUACAGCCUCCUAGCUUUCCU SEQ ID NO: 166 MI0000062 GGGUGAGGUAGUAGGUUGUAUAGUUUGGGGCUCUGCC CUGCUAUGGGAUAACUAUACAAUCUACUGUCUUUCCU SEQ ID NO: 167 |
| **72** | W | not in Plasma control | HSA-MIR-1206 // **HSA-MIR-1206** / none | MIMAT0005870 UGUUCAUGUAGAUGUUUAAGC SEQ ID NO: 72 | Not present in plasma | MI0006339 CAGUGUUCAUGUAGAUGUUUAAGCUCUUGCAGUAGGU UUUUGCAAGCUAGUGAACGCUG SEQ ID NO: 168 |
| **73** | W | not in Plasma control | HSA-MIR-132 // **HSA-MIR-132-3P** / HSA-MIR-132 | MIMAT0000426 UAACAGUCUACAGCCAUGGUCG SEQ ID NO: 73 | Not present in plasma | MI0000449 CCGCCCCCGCGUCUCCAGGGCAACCGUGGCUUUCGAU UGUUACUGUGGGAACUGGAGGUAACAGUCUACAGCCA UGGUCGCCCCGCAGCACGCCCACGCGC SEQ ID NO: 169 |
| **74** | W | not in Plasma control | HSA-MIR-153 // **HSA-MIR-153** / none | MIMAT0000428 UAUGGCUUUUUAUUCCUAUGUGA SEQ ID NO: 74 | Not present in plasma | MI0000463 CUCACAGCUGCCAGUGUCAUUUUUGUGAUCUGCAGCU AGUAUUCUCACUCCAGUUGCAUAGUCACAAAAGUGAU CAUUGGCAGGUGUGGC          SEQ ID NO: 170 MI0000464 AGCGGUGGCCAGUGUCAUUUUUGUGAUGUUGCAGCUA GUAAUAUGAGCCCAGUUGCAUAGUCACAAAAGUGAUC AUUGGAAACUGUG             SEQ ID NO: 171 |
| **75** | N G S | DS Specific | HSA-MIR-199b // **HSA-MIR-199b-5P** / HSA-MIR-199b | MIMAT0000263 CCCAGUGUUUAGACUAUCUGUUC SEQ ID NO: 75 | Down | MI0000282 CCAGAGGACACCUCCACUCCGUCUACCCAGUGUUUAG ACUAUCUGUUCAGGACUCCCAAAUUGUACAGUAGUCU GCACAUUGGUUAGGCUGGGCUGGGUUAGACCCUCGG SEQ ID NO: 172 |
| **76** | N G S | DS Specific | HSA-MIR-885 // **HSA-MIR-855-3P** / HSA-MIR-885 | MIMAT0004948 AGGCAGCGGGGUGUAGUGGAUA SEQ ID NO: 76 | Up (in DS) | MI0005560 CCGCACUCUCUCCAUUACACUACCCUGCCUCUUCUCC AUGAGAGGCAGCGGGGUGUAGUGGAUAGAGCACGGGU SEQ ID NO: 173 |
| **77** | N G S | DS Specific | HSA-MIR-885 // **HSA-MIR-855-5P** / HSA-MIR-885 | MIMAT0004947 UCCAUUACACUACCCUGCCUCU SEQ ID NO: 77 | UP | MI0005560 CCGCACUCUCUCCAUUACACUACCCUGCCUCUUCUCC AUGAGAGGCAGCGGGGUGUAGUGGAUAGAGCACGGGU SEQ ID NO: 174 |
| **78** | N G S | DS Specific | HSA-MIR-199a-1 // **HSA-MIR-199-5P** / HSA-MIR-199a | MIMAT0000231 CCCAGUGUUCAGACUACCUGUUC SEQ ID NO: 78 | Up | MI0000242 GCCAACCCAGUGUUCAGACUACCUGUUCAGGAGGCUC UCAAUGUGUACAGUAGUCUGCACAUUGGUUAGGC SEQ ID NO: 175 |
| **79** | N G S | DS Specific | HSA-MIR-199a-2 // **HSA-MIR-199-3P** / HSA-MIR-199a* | MIMAT0000232 ACAGUAGUCUGCACAUUGGUUA SEQ ID NO: 79 | Up | MI0000242 GCCAACCCAGUGUUCAGACUACCUGUUCAGGAGGCUC UCAAUGUGUACAGUAGUCUGCACAUUGGUUAGGC SEQ ID NO: 176 |
| **80** | N G S | DS Specific | HSA-MIR-122 // **HSA-MIR-122-5P** / HSA-MIR-122 | MIMAT0000421 UGGAGUGUGACAAUGGUGUUUG SEQ ID NO: 80 | Up | MI0000442 CCUUAGCAGAGCUGUGGAGUGUGACAAUGGUGUUUGU GUCUAAACUAUCAAACGCCAUUAUCACACUAAAUAGC UACUGCUAGGC SEQ ID NO: 177 |
| **81** | N G S | DS Specific | HSA-MIR-483 // **HSA-MIR-483-3P** / HSA-MIR-483 | MIMAT0002173 UCACUCCUCUCCUCCCGUCUU SEQ ID NO: 81 | Up | MI0002467 GAGGGGAAGACGGGAGGAAAGAAGGGAGUGGUUCCA UCACGCCUCCUCACUCCUCUCCUCCCGUCUUCUCCUC UC                    SEQ ID NO: 178 |

### References

1. Bogart et al., Prenat Diagn, 1987. 7(9): p. 623-30.
2. Pont-Kingdon and Lyon, Clin Chem, 2003. 49(7): p. 1087-94.
3. Cuckle et al., Br J Obstet Gynaecol, 1987. 94(5): p. 387-402.
4. Cohen, Health Care Guidelines for Individuals with Down Syndrome Down Syndrome Quarterly, 1999. Volume 4 (Number 3).
5. Chiu et al., Trends Genet, 2009. 25(7): p. 324-31.
6. Dudarewicz et al., J Appl Genet, 2005. 46(2): p. 207-15.
7. Nicolaides, Am J Obstet Gynecol, 2004. 191(1): p. 45-67.
14. Bischoff et al., Hum Reprod Update, 2002. 8(6): p. 493-500.
15. Simpson and Bischoff, JAMA, 2004. 291(9): p. 1135-7.
16. Kuhn et al., Biochem Biophys Res Commun, 2008. 370(3): p. 473-7.
17. Benjamini and Hochberg, Journal of the Royal Statistical Society. Series B (Methodological), 1995. 57(1): p. 289-300.
18. Yekutieli and Benjamini, Journal of Stat Plan and Infer, 1999. 82(1-2): p. 171-196.
19. Benjamini and Yekutieli, Annals of Science, 2001. 29(4): p. 1165-1188.
20. Hahn et al., Placenta, 2005. 26(7): p. 515-26.
21. Lo et al., Lancet, 1997. 350(9076): p. 485-7.
22. Lo, J Histochem Cytochem, 2005. 53(3): p. 293-6.
23. Lo, Clin Chem, 2000. 46(12): p. 1903-6.
24. Poon et al., Clin Chem, 2000. 46(11): p. 1832-4.
25. Lo et al., N Engl J Med, 1998. 339(24): p. 1734-8.
26. Faas et al., Lancet, 1998. 352(9135): p. 1196.
27. Costa et al., N Engl J Med, 2002. 346(19): p. 1502.
28. Chiu et al., Lancet, 2002. 360(9338): p. 998-1000.
29. Ng et al., Clin Chem, 2003. 49(5): p. 727-31.
30. Chim et al., Clin Chem, 2008. 54(3): p. 482-90.
31. Lo et al., Proc Natl Acad Sci U S A, 2007. 104(32): p. 13116-21.
32. Lo et al., Nat Med, 2007. 13(2): p. 218-23.
33. Fan et al., Proc Natl Acad Sci U S A, 2008. 105(42): p. 16266-71.
34. Busch et al., J Histochem Cytochem, 2005. 53(3): p. 341-3.
35. Nagalla et al., J Proteome Res, 2007. 6(4): p. 1245-57.
36. Kolialexi et al., Prenat Diagn, 2008. 28(8): p. 691-8.
37. Kolla et al., J Biomed Biotechnol, 2010. 2010: p. 952047.
38. Papageorgiou et al., Nat Med, 2011. 17(4): p. 510-3.
39. Liang et al., BMC Genomics, 2007. 8: p. 166.
40. Zhu et al., Am J Obstet Gynecol, 2009. 200(6): p. 661 e1-7
41. Bushati and Cohen, Annu Rev Cell Dev Biol, 2007. 23: p. 175-205.
42. Eisenberg et al., Proc Natl Acad Sci U S A, 2007. 104(43): p. 17016-21.
43. Kuhn et al., J Biol Chem, 2010. 285(2): p. 1529-43.
44. Reid et al., Crit Rev Oncol Hematol, 2011. 80(2): p. 193-208.
45. Tijssen, P., Hybridization with nucleic acid probes. Laboratory techniques in biochemistry and molecular biology1993, Amsterdam ; New York: Elsevier.
46. Miura et al., Clin Chem, 2010. 56(11): p. 1767-71.
47. Yang et al., Clin Chim Acta, 2011. 412(23-24): p. 2167-73.
48. Gunel et al., Genet Mol Res, 2011. 10(4): p. 4034-40.
49. Mayor-Lynn et al., Reprod Sci, 2011. 18(1): p. 46-56.
50. Li et al., Clin Chim Acta, 2012. 413(5-6): p. 568-76.
51. Bissels et al., RNA, 2009. 15(12): p. 2375-84.
52. Hua et al., Genomics, 2008. 92(2): p. 122-8.
53. Git et al., RNA, 2010. 16(s): p. 991-1006.
54. Rigby et al., I. J Mol Biol, 1977. 113(1): p. 237-51.
55. Feinberg, and Vogelstein, Anal Biochem, 1983. 132(1): p. 6-13.
56. Sambrook, Fritsch, and Maniatis, Molecular cloning : a laboratory manual. 2nd ed1989, Cold Spring Harbor, N.Y. : Cold Spring Harbor Laboratory Press 1626.
57. Chun et al., Analytica Chimica Acta, 2000. 411: p. 31-36.
58. Forster et al., Nucleic Acids Res, 1985. 13(3): p. 745-61.
59. Hacia et al., Nat Genet, 1996. 14(4): p. 441-7.
60. Shoemaker et al., Nat Genet, 1996. 14(4): p. 450-6.
61. Pease et al., Proc Natl Acad Sci U S A, 1994. 91(11): p. 5022-6.
62. Fodor et al., Science, 1991. 251(4995): p. 767-73.
63. Rhodius et al., Annu Rev Microbiol, 2002. 56: p. 599-624.
64. Rhodius et al., PLoS Biol, 2006. 4(1): p. e2.
65. Schena et al., Science, 1995. 270(5235): p. 467-70.
66. Kim, Nat Rev Mol Cell Biol, 2005. 6(5): p. 376-85.
67. Bartel, Cell, 2004. 116(2): p. 281-97.
68. He and Hannon, Nat Rev Genet, 2004. 5(7): p. 522-31.
69. Lagos-Quintana et al., Science, 2001. 294(5543): p. 853-8.
70. Ambros, Cell, 2003. 113(6): p. 673-6.
71. Griffiths-Jones et al., Nucleic Acids Res, 2003. 31(1): p. 439-41.
72. Saini et al., Proc Natl Acad Sci U S A, 2007. 104(45): p. 17719-24.
73. Lee et al., EMBO J, 2002. 21(17): p. 4663-70.
74. Reinhart et al., Genes Dev, 2002. 16(13): p. 1616-26.
75. Lund et al., Science, 2004. 303(5654): p. 95-8.
76. Millar, and Waterhouse, Funct Integr Genomics, 2005. 5(3): p. 129-35.
85. Alberts, Molecular biology of the cell. 3rd ed1994, New York: Garland Pub. xliii, 1294, 67 p.
86. Mattaj et al., FASEB J, 1993. 7(1): p. 47-53.
87. Cox and Ellington, Bioorg Med Chem, 2001. 9(10): p. 2525-31.
88. Lee et al., Nucleic Acids Res, 2004. 32(Database issue): p. D95-100.
89. Argos, EMBO J, 1989. 8(3): p. 779-85.
90. Karlin and Altschul, Proc Natl Acad Sci U S A, 1993. 90(12): p. 5873-7.
91. Altschul et al., J Mol Biol, 1990. 215(3): p. 403-10.
92. Wittwer et al., Methods, 2001. 25(4): p. 430-42.
96. Jepsen et al., Oligonucleotides, 2004. 14(2): p. 130-46.
97. Valoczi et al., Nucleic Acids Res, 2004. 32(22): p. e175.
98. Berks, Trends Biotechnol, 1994. 12(9): p. 352-64.
99. Smyth, Bioinformatics and Computational Biology Solutions using R and Bioconductor, ed. R. Gentleman, et al. 2005, New York: Springer. 397-420.
100. Romualdi et al., Physiol Genomics, 2003. 12(2): p. 159-62.
101. Robinson et al., Bioinformatics, 2010. 26(1): p. 139-40.
102. Keck-Wherley et al., Dev Neurosci, 2011. 33(5): p. 451-67.
103. Kotlabova et al., J Reprod Immunol, 2011. 89(2): p. 185-91.
113. Faas et al., Semin Fetal Neonatal Med, 2011. 16(2): p. 81-7.
114. van Zwieten, The Target of Testing. Dealing With 'Unexpected' Findings In Prenatal Diagnosis. (Uitgeverij Buijten & Schipperheijn, Amsterdam, 2006).

## Claims

1. A non-invasive method for evaluation of fetal chromosomal aneuploidy comprising analyzing a body fluid sample obtained from a subject for one or more specific sequences of cell-free non-coding RNAs (ncRNAs) and/or precursors thereof, wherein said analyzing comprises the step of detecting and optionally quantitating said ncRNA(s) and/or the precursor(s).

2. The method of claim 1, wherein at least two specific sequences of ncRNAs and/or precursors thereof are analyzed.

3. The method of claim 1, wherein the ncRNA is a microRNA (miRNA) or a small nuclear RNA (snRNA).

4. The method of any one of claims 1 to 3, wherein the sample is obtained from a female within the first trimester or up to 16 weeks after gestation.

5. The method of any one of claims 1 to 4, wherein the chromosomal aneuploidy is Trisomy 21 (Down syndrome).

6. The method of any one of claims 1 to 5, wherein the sample is a body fluid comprising blood, blood serum and/or blood plasma.

7. The method of any one of claims 1 to 6, wherein analyzing the presence and/or amount of the ncRNA(s) and/or precursor(s) thereof comprises:
(a) amplification of the ncRNA(s) and/or precursor(s) thereof by a polymerase chain reaction (PCR), preferably by a real time polymerase chain reaction (qRT-PCR);
(b) hybridization of the ncRNA(s) and/or precursor(s) thereof or of the product(s) obtained in (a) with one or more binding molecules specific for the ncRNA(s) and/or precursor(s) thereof; and/or
(c) sequencing of the proliferation product(s) of (a).

8. The method of any one of claims 1 to 7, wherein analyzing the presence and/or amount of the ncRNA(s) and/or precursor(s) thereof is performed on an affinity matrix comprising a solid support having bound at least one primer and/or probe.

9. The method of claim 8, wherein the solid support comprises a wafer.

10. The method of any one of claims 1 to 9, wherein the miRNAs are selected from the group indicated in Table 12 consisting of: HSA-MIR-589-3P, HSA-MIR-512-5P, HSA-MIR-181a-3p, HSA-MIR-483-5P, HSA-MIR-362-5P, HSA-MIR-518c-5P, HSA-MIR-3667-5P, HSA-MIR-195-3P, HSA-MIR-124-5P, HSA-MIR-498, HSA-MIR-18B-3P, HSA-MIR-888-3P, HSA-MIR-3676-3P, HSA-MIR-3918, HSA-MIR-500A-5P, HSA-MIR-30C-5P, HSA-MIR-200C-3P, HSA-MIR-627, HSA-MIR-93-5P, HSA-MIR-20A-5P, HSA-MIR-4328, HSA-MIR-92a-1-5P, HSA-MIR-92b-3P, HSA-MIR-3652, HSA-MIR-25-3P, HSA-MIR-623, HSA-MIR-3195, HSA-MIR-423-5P, HSA-MIR-27B-3P, HSA-MIR-3135a, HSA-MIR-486-5P, HSA-MIR-3180-5P, HSA-MIR-629-5P, HSA-MIR-629-3P, HSA-MIR-155-5P, HSA-MIR-3907, HSA-MIR-1249, HSA-MIR-215, HSA-MIR-1227-3P, HSA-MIR-187-3P, HSA-MIR-219-5P, HSA-MIR-3913-5P, HSA-MIR-590-3P, HSA-MIR-3923, HSA-MIR-1228-5P, HSA-MIR-769-3P, HSA-MIR-127-5P, HSA-MIR-1293, HSA-LET-7f-1-3p, HSA-MIR-93-3P, HSA-MIR-892B, HSA-MIR-320B, HSA-MIR-106a-3P, HSA-MIR-92B-5P, HSA-MIR-328, HSA-MIR-196B-3P, HSA-MIR-548C-5P, HSA-MIR-512-3P, HSA-MIR-4306, HSA-MIR-29B-1-5P, HSA-MIR-30C-1-3P, HSA-MIR-199b-5P, HSA-MIR-855-3P, HSA-MIR-855-5P, HSA-MIR-199-5P, HSA-MIR-199-3P, HSA-MIR-122-5P, HSA-MIR-483-3P and having the SEQ ID NOs: 1-15, 17-62, and 75-81; the precursors thereof are selected from the group of precursors as indicated in Table 12 having SEQ ID NOs: 82-153 and 172-178; and the snRNA is RNU12 as indicated in Table 12 having SEQ ID NO: 16, wherein compared to a control sample or a control fetus:
(a) a comparable level of the ncRNAs and/or precursors thereof is indicative of a normal euploidy and/or of the same fetal gender of the fetus; and
(b) a decreased or an increased level of the ncRNAs and/or precursors thereof is indicative of an increased risk of the fetus for fetal chromosomal aneuploidy and/or of different fetal gender than the control fetus.

11. Use of a primer or probe comprising a nucleic acid binding molecule or an affinity matrix comprising at least one said primer or probe in a method of any one of claims 1 to 10.

12. The method of any one of claims 8 to 10 or the use of claim 11, wherein the affinity matrix is a chip.

13. A device adapted for a method of non-invasive evaluation of chromosomal aneuploidy in a subject comprising:
(a) an analyzing unit comprising a detection agent and at least one of the ncRNAs and/or precursors thereof as defined in any one of claims 1 to 10 or 12, wherein said analyzing unit is adapted for determining the amounts of said ncRNAs and/or precursors thereof in a sample detected by the detection agent; and
(b) an evaluation unit comprising a computer comprising tangibly embedded a computer program code for carrying out a comparison of the determined amounts obtained from the analyzing unit.

14. A kit adapted for a method of non-invasive evaluation of chromosomal aneuploidy in a subject comprising:
(a) a detection agent for at least one of the ncRNAs and/or precursors thereof as defined in any one of claims 1 to 10 or 12, preferably wherein the detection agent is or comprises a primer and/or probe as defined in any one of claims 1 to 10 or an affinity matrix as defined in any one of claims 8 to 12;
(b) a reverse transcriptase, a DNA polymerase, deoxynucleoside triphosphates (dNTPs), a Mg²⁺-ion solution, one or more primers and/or a reaction buffer;
(c) a label specifically binding to a chimeric molecule consisting of the primer or probe as defined in (a) bound to the ncRNA and/or precursor thereof as defined in any one of claims 1 to 10 or 12 and/or to an amplification product of a PCR and/or an RT-qPCR as defined in any one of claims 7 to 10 or 12;
(d) a device adapted for determining the amount of at least one of the ncRNAs and/or precursors thereof detected by said detection agent, wherein said device is also adapted for carrying out the comparison of said amounts as defined in any one of claims 1 to 10 or 12; and optionally
(e) instructions for use in the method of any one of claims 1 to 10 or 12 and/or a control sample or reference value thereof.

15. Use of one or more of the ncRNAs and/or precursors thereof as defined in any one of claims 1 to 3 or 10 for a non-invasive method of evaluation of a disorder associated with pregnancy and/or pathology, preferably wherein said disorder is Down syndrome.


SEQUENCE LISTING

<110>  Charité - Universitaetsmedizin Berlin

<120>  Non-invasive prenatal genetic diagnostic methods

<130>  CH43A02/P-EP

<160>  178

<170>  PatentIn version 3.5

<210>  1
<211>  24
<212>  RNA
<213>  Homo sapiens

<400>  1
ucagaacaaa ugccgguucc caga                                              24

<210>  2
<211>  23
<212>  RNA
<213>  Homo sapiens

<400>  2
cacucagccu ugagggcacu uuc                                               23

<210>  3
<211>  22
<212>  RNA
<213>  Homo sapiens

<400>  3
accaucgacc guugauugua cc                                                22

<210>  4
<211>  22
<212>  RNA
<213>  Homo sapiens

<400>  4
aagacgggag gaaagaaggg ag                                                22

<210>  5
<211>  24
<212>  RNA
<213>  Homo sapiens

<400>  5
aauccuugga accuaggugu gagu                                              24

<210>  6
<211>  23
<212>  RNA
<213>  Homo sapiens

<400>  6
ucucuggagg gaagcacuuu cug                                               23

<210>  7
<211>  22
<212>  RNA
<213>  Homo sapiens

<400>  7
aaagacccau ugaggagaag gu                                                22


<210>  8
<211>  22
<212>  RNA
<213>  Homo sapiens

<400>  8
ccaauauugg cugugcugcu cc                                                22


<210>  9
<211>  22
<212>  RNA
<213>  Homo sapiens

<400>  9
cguguucaca gcggaccuug au                                                22


<210>  10
<211>  23
<212>  RNA
<213>  Homo sapiens

<400>  10
uuucaagcca gggggcguuu uuc                                               23


<210>  11
<211>  22
<212>  RNA
<213>  Homo sapiens

<400>  11
ugcccuaaau gccccuucug gc                                                22


<210>  12
<211>  22
<212>  RNA
<213>  Homo sapiens

<400>  12
gacugacacc ucuuugggug aa                                                22


<210>  13
<211>  15
<212>  RNA
<213>  Homo sapiens

<400>  13
aggagauccu ggguu                                                        15


<210>  14

<211>  21
<212>  RNA
<213>  Homo sapiens

<400>  14
acagggccgc agauggagac u                                                21


<210>  15
<211>  23
<212>  RNA
<213>  Homo sapiens

<400>  15
uaauccuugc uaccugggug aga                                              23


<210>  16
<211>  149
<212>  DNA
<213>  Homo sapiens


<220>
<221>  snRNA
<222>  (1)..(149)
<223>  RNU12, gene_synonym="dJ222E13.7; RNU12-1; RNU12L; RNU12P"
       U12 small nuclear (RNU12), small nuclear RNA

<300>
<308>  NR_029422.1
<309>  2011-02-26
<313>  (1)..(149)

<400>  16
tgccttaaac ttatgagtaa ggaaaataac gattcggggt gacgcccgaa tcctcactgc      60

taatgtgaga cgaatttttg agcgggtaaa ggtcgccctc aaggtgaccc gcctactttg     120

cgggatgcct gggagttgcg atctgcccg                                       149


<210>  17
<211>  23
<212>  RNA
<213>  Homo sapiens

<400>  17
uguaaacauc cuacacucuc agc                                              23


<210>  18
<211>  22
<212>  RNA
<213>  Homo sapiens

<400>  18
cgucuuaccc agcaguguuu gg                                               22


<210>  19
<211>  22
<212>  RNA
<213>  Homo sapiens

<400>  19
gugagucucu aagaaaagag ga                                              22


<210>  20
<211>  23
<212>  RNA
<213>  Homo sapiens

<400>  20
caaagugcug uucgugcagg uag                                             23


<210>  21
<211>  23
<212>  RNA
<213>  Homo sapiens

<400>  21
uaaagugcuu auagugcagg uag                                             23


<210>  22
<211>  17
<212>  RNA
<213>  Homo sapiens

<400>  22
ccaguuuucc caggauu                                                    17


<210>  23
<211>  22
<212>  RNA
<213>  Homo sapiens

<400>  23
uauugcacuu gucccggccu gu                                              22


<210>  24
<211>  22
<212>  RNA
<213>  Homo sapiens

<400>  24
uauugcacuc gucccggccu cc                                              22


<210>  25
<211>  18
<212>  RNA
<213>  Homo sapiens

<400>  25
cggcuggagg ugugagga                                                   18


<210>  26
<211>  22
<212>  RNA
<213>  Homo sapiens

<400>  26
cauugcacuu gucucggucu ga                                              22

<210>  27
<211>  23
<212>  RNA
<213>  Homo sapiens

<400>  27
aucccuugca ggggcuguug ggu                                               23


<210>  28
<211>  17
<212>  RNA
<213>  Homo sapiens

<400>  28
cgcgccgggc ccggguu                                                      17


<210>  29
<211>  23
<212>  RNA
<213>  Homo sapiens

<400>  29
ugaggggcag agagcgagac uuu                                               23


<210>  30
<211>  21
<212>  RNA
<213>  Homo sapiens

<400>  30
uucacagugg cuaaguucug c                                                 21


<210>  31
<211>  22
<212>  RNA
<213>  Homo sapiens

<400>  31
ugccuaggcu gagacugcag ug                                                22


<210>  32
<211>  22
<212>  RNA
<213>  Homo sapiens

<400>  32
uccuguacug agcugccccg ag                                                22


<210>  33
<211>  25
<212>  RNA
<213>  Homo sapiens

<400>  33
cuuccagacg cuccgcccca cgucg                                             25

<210> 34
<211> 21
<212> RNA
<213> Homo sapiens

<400> 34
   uggguuuacg uugggagaac u 21

<210> 35
<211> 22
<212> RNA
<213> Homo sapiens

<400> 35
guucucccaa cguaagccca gc 22

<210> 36
<211> 23
<212> RNA
<213> Homo sapiens

<400> 36
uuaaugcuaa ucgugauagg ggu 23

<210> 37
<211> 22
<212> RNA
<213> Homo sapiens

<400> 37
aggugcucca ggcuggcuca ca 22

<210> 38
<211> 22
<212> RNA
<213> Homo sapiens

<400> 38
acgcccuucc cccccuucuu ca 22

<210> 39
<211> 21
<212> RNA
<213> Homo sapiens

<400> 39
augaccuaug aauugacaga c 21

<210> 40
<211> 20
<212> RNA
<213> Homo sapiens

<400> 40
cgugccaccc uuuuccccag 20

<210> 41
<211> 22

<212>  RNA
<213>  Homo sapiens

<400>  41
ucgugucuug uguugcagcc gg                                                22


<210>  42
<211>  21
<212>  RNA
<213>  Homo sapiens

<400>  42
ugauugucca aacgcaauuc u                                                 21


<210>  43
<211>  22
<212>  RNA
<213>  Homo sapiens

<400>  43
uuugggacug aucuugaugu cu                                                22


<210>  44
<211>  21
<212>  RNA
<213>  Homo sapiens

<400>  44
uaauuuuaug uauaagcuag u                                                 21


<210>  45
<211>  22
<212>  RNA
<213>  Homo sapiens

<400>  45
aacuaguaau guuggauuag gg                                                22


<210>  46
<211>  21
<212>  RNA
<213>  Homo sapiens

<400>  46
gugggcgggg gcaggugugu g                                                 21


<210>  47
<211>  23
<212>  RNA
<213>  Homo sapiens

<400>  47
cugggaucuc cggggucuug guu                                               23


<210>  48
<211>  22
<212>  RNA
<213>  Homo sapiens

<400>  48
cugaagcuca gagggcucug au                                              22


<210>  49
<211>  22
<212>  RNA
<213>  Homo sapiens

<400>  49
   uggguggucu ggagauuugu gc                                             22


<210>  50
<211>  22
<212>  RNA
<213>  Homo sapiens

<400>  50
cuauacaauc uauugccuuc cc                                              22


<210>  51
<211>  22
<212>  RNA
<213>  Homo sapiens

<400>  51
acugcugagc uagcacuucc cg                                              22


<210>  52
<211>  22
<212>  RNA
<213>  Homo sapiens

<400>  52
cacuggcucc uuucugggua ga                                              22


<210>  53
<211>  22
<212>  RNA
<213>  Homo sapiens

<400>  53
aaaagcuggg uugagagggc aa                                              22


<210>  54
<211>  22
<212>  RNA
<213>  Homo sapiens

<400>  54
cugcaaugua agcacuucuu ac                                              22


<210>  55
<211>  22
<212>  RNA
<213>  Homo sapiens

<400>  55

agggacggga cgcggugcag ug                                                22

<210>  56
<211>  22
<212>  RNA
<213>  Homo sapiens

<400>  56
cuggcccucu cugcccuucc gu                                                22

<210>  57
<211>  22
<212>  RNA
<213>  Homo sapiens

<400>  57
ucgacagcac gacacugccu uc                                                22

<210>  58
<211>  22
<212>  RNA
<213>  Homo sapiens

<400>  58
aaaaguaauu gcgguuuuug cc                                                22

<210>  59
<211>  22
<212>  RNA
<213>  Homo sapiens

<400>  59
aagugcuguc auagcugagg uc                                                22

<210>  60
<211>  17
<212>  RNA
<213>  Homo sapiens

<400>  60
uggagagaaa ggcagua                                                17

<210>  61
<211>  24
<212>  RNA
<213>  Homo sapiens

<400>  61
gcugguuuca uauggugguu uaga                                                24

<210>  62
<211>  22
<212>  RNA
<213>  Homo sapiens

<400>  62
cugggagagg guuguuuacu cc                                                22

<210>  63
<211>  21
<212>  RNA
<213>  Homo sapiens

<400>  63
uacaguacug ugauaacuga a                                                21


<210>  64
<211>  23
<212>  RNA
<213>  Homo sapiens

<400>  64
ucauagcccu guacaaugcu gcu                                              23


<210>  65
<211>  22
<212>  RNA
<213>  Homo sapiens

<400>  65
uagcagcacg uaaauauugg cg                                               22


<210>  66
<211>  22
<212>  RNA
<213>  Homo sapiens

<400>  66
uggagagaaa ggcaguuccu ga                                               22


<210>  67
<211>  22
<212>  RNA
<213>  Homo sapiens

<400>  67
ugucaguuug ucaaauaccc ca                                               22


<210>  68
<211>  21
<212>  RNA
<213>  Homo sapiens

<400>  68
acuggacuug gagucagaag g                                                21


<210>  69
<211>  21
<212>  RNA
<213>  Homo sapiens

<400>  69
acuggacuug gagucagaag g                                                21


<210>  70

<211>  22
<212>  RNA
<213>  Homo sapiens

<400>  70
aaaccguuac cauuacugag uu                                                22


<210>  71
<211>  22
<212>  RNA
<213>  Homo sapiens

<400>  71
ugagguagua gguuguauag uu                                                22


<210>  72
<211>  21
<212>  RNA
<213>  Homo sapiens

<400>  72
uguucaugua gauguuuaag c                                                 21


<210>  73
<211>  22
<212>  RNA
<213>  Homo sapiens

<400>  73
uaacagucua cagccauggu cg                                                22


<210>  74
<211>  23
<212>  RNA
<213>  Homo sapiens

<400>  74
uauggcuuuu uauuccuaug uga                                               23


<210>  75
<211>  23
<212>  RNA
<213>  Homo sapiens

<400>  75
cccaguguuu agacuaucug uuc                                               23


<210>  76
<211>  22
<212>  RNA
<213>  Homo sapiens

<400>  76
aggcagcggg guguagugga ua                                                22


<210>  77
<211>  22
<212>  RNA

<213>  Homo sapiens

<400>  77
uccauuacac uacccugccu cu                                                22


<210>  78
<211>  23
<212>  RNA
<213>  Homo sapiens

<400>  78
cccaguguuc agacuaccug uuc                                               23


<210>  79
<211>  22
<212>  RNA
<213>  Homo sapiens

<400>  79
acaguagucu gcacauuggu ua                                                22


<210>  80
<211>  22
<212>  RNA
<213>  Homo sapiens

<400>  80
uggaguguga caaugguguu ug                                                22


<210>  81
<211>  21
<212>  RNA
<213>  Homo sapiens

<400>  81
ucacuccucu ccucccgucu u                                                 21


<210>  82
<211>  99
<212>  RNA
<213>  Homo sapiens

<400>  82
uccagccugu gcccagcagc cccugagaac cacgucugcu cugagcuggg uacugccugu      60

ucagaacaaa ugccgguucc cagacgcugc cagcuggcc                              99


<210>  83
<211>  84
<212>  RNA
<213>  Homo sapiens

<400>  83
ucucagucug uggcacucag ccuugagggc acuuucuggu gccagaauga aagugcuguc      60

auagcugagg uccaaugacu gagg                                              84


<210>  84

<211>  98
<212>  RNA
<213>  Homo sapiens

<400>  84
   gguacuucuc agucuguggc acucagccuu gagggcacuu ucuggugcca gaaugaaagu        60
   gcugucauag cugaggucca augacugagg cgagcacc                              98


<210>  85
<211>  110
<212>  RNA
<213>  Homo sapiens

<400>  85
agaagggcua ucaggccagc cuucagagga cuccaaggaa cauucaacgc ugucggugag      60

uuugggauuu gaaaaaacca cugaccguug acuguaccuu ggggucuuua                110


<210>  86
<211>  76
<212>  RNA
<213>  Homo sapiens

<400>  86
gagggggaag acgggaggaa agaagggagu gguuccauca cgccuccuca cuccucuccu      60

cccgucuucu ccucuc                                                      76


<210>  87
<211>  65
<212>  RNA
<213>  Homo sapiens

<400>  87
cuugaauccu uggaaccuag gugugagugc uauuucagug caacacaccu auucaaggau      60

ucaaa                                                                  65


<210>  88
<211>  101
<212>  RNA
<213>  Homo sapiens

<400>  88
gcgagaagau cucaugcugu gacucucugg agggaagcac uuucuguugu cugaaagaaa      60

acaaagcgcu ucucuuuaga guguuacggu uugagaaaag c                         101


<210>  89
<211>  74
<212>  RNA
<213>  Homo sapiens

<400>  89
ugaggaugaa agacccauug aggagaaggu ucugcuggcu gagaaccuuc cucuccaugg      60

gucuuucauc cuca                                                        74

<210> 90
<211> 87
<212> RNA
<213> Homo sapiens

<400> 90
agcuucccug gcucuagcag cacagaaaua uuggcacagg gaagcgaguc ugccaauauu 60

ggcugugcug cuccaggcag gguggug 87


<210> 91
<211> 85
<212> RNA
<213> Homo sapiens

<400> 91
aggccucucu cuccguguuc acagcggacc uugauuuaaa uguccauaca auuaaggcac 60

gcggugaaug ccaagaaugg ggcug 85


<210> 92
<211> 109
<212> RNA
<213> Homo sapiens

<400> 92
aucaagauua gaggcucugc ucuccguguu cacagcggac cuugauuuaa ugucauacaa 60

uuaaggcacg cggugaaugc caagagcgga gccuacggcu gcacuugaa 109


<210> 93
<211> 87
<212> RNA
<213> Homo sapiens

<400> 93
ugagggcccc ucugcguguu cacagcggac cuugauuuaa ugucuauaca auuaaggcac 60

gcggugaaug ccaagagagg cgccucc 87


<210> 94
<211> 124
<212> RNA
<213> Homo sapiens

<400> 94
aacccuccuu gggaagugaa gcucaggcug ugauuucaag ccagggggcg uuuuucuaua 60

acuggaugaa aagcaccucc agagcuugaa gcucacaguu ugagagcaau cgucuaagga 120

aguu 124


<210> 95
<211> 71
<212> RNA
<213> Homo sapiens

<400> 95
uguguuaagg ugcaucuagu gcaguuagug aagcagcuua gaaucuacug cccuaaaugc 60

cccuucuggc a                                                          71

<210>  96
<211>  77
<212>  RNA
<213>  Homo sapiens

<400>  96
ggcagugcuc uacucaaaaa gcugucaguc acuuagauua caugugacug acaccucuuu      60

gggugaagga aggcuca                                                    77

<210>  97
<211>  85
<212>  RNA
<213>  Homo sapiens

<400>  97
uugguuaaag cgccugucua guaaacagga gauccugggu ucgaauccca gcggugccuc      60

cguguuuccc ccacgcuuuu gccaa                                           85

<210>  98
<211>  93
<212>  RNA
<213>  Homo sapiens

<400>  98
aggcgguuaa gccaugggac agggccgcag auggagacug cucaagguca aaggggucuc      60

cagcugggac ccugcaccug guucguagcc ccu                                  93

<210>  99
<211>  84
<212>  RNA
<213>  Homo sapiens

<400>  99
gcucccccuc ucuaauccuu gcuaccuggg ugagagugcu gucugaaugc aaugcaccug      60

ggcaaggauu cugagagcga gagc                                            84

<210>  100
<211>  72
<212>  RNA
<213>  Homo sapiens

<400>  100
agauacugua aacauccuac acucucagcu guggaaagua agaaagcugg gagaaggcug      60

uuuacucuuu cu                                                         72

<210>  101
<211>  89
<212>  RNA
<213>  Homo sapiens

<400>  101
   accaugcugu agugugugua aacauccuac acucucagcu gugagcucaa gguggcuggg      60
   agaggguugu uuacuccuuc ugccaugga                                    89

<210>  102
<211>  68
<212>  RNA
<213>  Homo sapiens

<400>  102
cccucgucuu acccagcagu guuugggugc gguugggagu cucuaauacu gccggguaau      60

gauggagg                                                                68

<210>  103
<211>  97
<212>  RNA
<213>  Homo sapiens

<400>  103
uacuuauuac ugguagugag ucucuaagaa aagaggaggu gguuguuuuc cuccucuuuu      60

cuuugagacu cacuaccaau aauaagaaau acuacua                               97

<210>  104
<211>  80
<212>  RNA
<213>  Homo sapiens

<400>  104
cugggggcuc caaagugcug uucgugcagg uagugugauu acccaaccua cugcugagcu      60

agcacuuccc gagcccccgg                                                   80

<210>  105
<211>  71
<212>  RNA
<213>  Homo sapiens

<400>  105
guagcacuaa agugcuuaua gugcagguag uguuuaguua ucuacugcau uaugagcacu      60

uaaaguacug c                                                            71

<210>  106
<211>  56
<212>  RNA
<213>  Homo sapiens

<400>  106
aacaguugag uccugagaac cauugagaac caguuuuccc aggauuaacu guuccg          56

<210>  107
<211>  78
<212>  RNA
<213>  Homo sapiens

<400>  107
cuuucuacac agguugggau cgguugcaau gcuguguuuc uguaugguau ugcacuuguc      60

ccggccuguu gaguuugg                                                    78

<210>  108
<211>  96
<212>  RNA
<213>  Homo sapiens

<400>  108
cgggccccgg gcgggcggga gggacgggac gcggugcagu guuguuuuuu cccccgccaa      60

uauugcacuc gucccggccu ccggcccccc cggccc                                96

<210>  109
<211>  131
<212>  RNA
<213>  Homo sapiens

<400>  109
cggcuggagg ugugaggauc cgaacccagg gguggggggu ggaggcggcu ccugcgaucg      60

aaggggacuu gagacucacc ggccgcacgc caugagggcc cugugggugc ugggccucug      120

cugcguccug c                                                          131

<210>  110
<211>  84
<212>  RNA
<213>  Homo sapiens

<400>  110
ggccaguguu gagaggcgga gacuugggca auugcuggac gcugcccugg gcauugcacu      60

ugucucgguc ugacagugcc ggcc                                             84

<210>  111
<211>  98
<212>  RNA
<213>  Homo sapiens

<400>  111
guacacagua gaagcauccc uugcaggggc uguuggguug cauccuaagc ugugcuggag      60

cuucccgaug uacucuguag augucuuugc accuucug                              98

<210>  112
<211>  84
<212>  RNA
<213>  Homo sapiens

<400>  112
ccgcagccgc cgcgccgggc ccggguuggc cgcugacccc cgcggggccc ccggcggccg      60

gggcgggggc gggggcugcc ccgg                                             84

<210>  113
<211>  94
<212>  RNA
<213>  Homo sapiens

<400>  113
auaaaggaag uuaggcugag gggcagagag cgagacuuuu cuauuuucca aaagcucggu      60

cugaggcccc ucagucuugc uuccuaaccc gcgc                                  94


<210>  114
<211>  97
<212>  RNA
<213>  Homo sapiens

<400>  114
accucucuaa caaggugcag agcuuagcug auuggugaac agugauuggu uuccgcuuug      60

uucacagugg cuaaguucug caccugaaga gaaggug                               97


<210>  115
<211>  77
<212>  RNA
<213>  Homo sapiens

<400>  115
ucacuuuggu gccuaggcug agacugcagu ggugcaaucu caguucacug cagccuugac      60

cuccugggcu cagguga                                                     77


<210>  116
<211>  68
<212>  RNA
<213>  Homo sapiens

<400>  116
gcauccugua cugagcugcc ccgaggcccu ucaugcugcc cagcucgggg cagcucagua      60

caggauac                                                               68


<210>  117
<211>  94
<212>  RNA
<213>  Homo sapiens

<400>  117
cagugcgacg ggcggagcuu ccagacgcuc cgccccacgu cgcaugcgcc ccgggaaagc      60

guggggcgga gcuuccggag gccccgcccu gcug                                  94


<210>  118
<211>  88
<212>  RNA
<213>  Homo sapiens

<400>  118
gcgacgggcg gagcuuccag acgcuccgcc ccacgucgca ugcgccccgg gaaagcgugg      60

ggcggagcuu ccggaggccc cgcccugc                                         88


<210>  119
<211>  94
<212>  RNA
<213>  Homo sapiens

<400>  119
cagugcgacg ggcggagcuu ccagacgcuc cgccccacgu cgcaugcgcc ccgggaaagc      60

guggggcgga gcuuccggag gccccgcccu gcug                                  94


<210>  120
<211>  97
<212>  RNA
<213>  Homo sapiens

<400>  120
ucccuuuccc aggggagggg cuggguuuac guugggagaa cuuuuacggu gaaccaggag      60

guucucccaa cguaagccca gccccucccc ucugccu                               97


<210>  121
<211>  97
<212>  RNA
<213>  Homo sapiens

<400>  121
ucccuuuccc aggggagggg cuggguuuac guugggagaa cuuuuacggu gaaccaggag      60

guucucccaa cguaagccca gccccucccc ucugccu                               97


<210>  122
<211>  65
<212>  RNA
<213>  Homo sapiens

<400>  122
cuguuaaugc uaaucgugau agggguuuuu gccuccaacu gacuccuaca uauuagcauu      60

aacag                                                                   65


<210>  123
<211>  151
<212>  RNA
<213>  Homo sapiens

<400>  123
ggguuggaaa gcuguaggug uggaggggca uggauacggg ggccaugagg guggggucca      60

ggcuggacca ggccugcccu gaguccccca gcaggugcuc caggcuggcu cacacccucu     120

gccucucucu cuuccuuccu ggccccaacc c                                     151


<210>  124
<211>  66
<212>  RNA
<213>  Homo sapiens

<400>  124
gggaggaggg aggagauggg ccaaguuccc ucuggcugga acgcccuucc cccccuucuu      60

caccug                                                                  66


<210>  125

<211>  110
<212>  RNA
<213>  Homo sapiens

<400>  125
aucauucaga aaugguauac aggaaaauga ccuaugaauu gacagacaau auagcugagu      60

uugucuguca uuucuuuagg ccaauauucu guaugacugu gcuacuucaa                110


<210>  126
<211>  88
<212>  RNA
<213>  Homo sapiens

<400>  126
guggggccag gcgguggugg gcacugcugg ggugggcaca gcagccaugc agagcgggca      60

uuugaccccg ugccacccuu uuccccag                                          88


<210>  127
<211>  109
<212>  RNA
<213>  Homo sapiens

<400>  127
ggucgggcuc accaugacac agugugagac cucgggcuac aacacaggac ccgggcgcug      60

cucugacccc ucgugucuug uguugcagcc ggagggacgc agguccgca                 109


<210>  128
<211>  97
<212>  RNA
<213>  Homo sapiens

<400>  128
acucaggggc uucgccacug auuguccaaa cgcaauucuu guacgagucu gcggccaacc      60

gagaauugug gcuggacauc uguggcugag cuccggg                               97


<210>  129
<211>  110
<212>  RNA
<213>  Homo sapiens

<400>  129
ccgccccggg ccgcggcucc ugauugucca aacgcaauuc ucgagucuau ggcuccggcc      60

gagaguugag ucuggacguc ccgagccgcc gcccccaaac cucgagcggg                110


<210>  130
<211>  102
<212>  RNA
<213>  Homo sapiens

<400>  130
uuguuuauaa uaaacugaaa uauuugggac ugaucuugau gucugccaaa accuuggcag      60

acaucaagau cagucccaaa uauuucaguu uauuauagac ag                        102

<210>  131
<211>  100
<212>  RNA
<213>  Homo sapiens

<400>  131
ugucuauaau aaacugaaau auuugggacu gaucuugaug ucugccaagg uuuuggcaga      60

caucaagauc agucccaaau auuucaguuu auuauaaaca                          100


<210>  132
<211>  22
<212>  RNA
<213>  Homo sapiens

<400>  132
uuugggacug aucuugaugu cu                                               22


<210>  133
<211>  97
<212>  RNA
<213>  Homo sapiens

<400>  133
uagccaguca gaaaugagcu uauucauaaa agugcaguau ggugaaguca aucuguaauu      60

uuauguauaa gcuagucucu gauugaaaca ugcagca                               97


<210>  134
<211>  83
<212>  RNA
<213>  Homo sapiens

<400>  134
gguagaguga gcucuaaucc aauauuacua gcuucuuuau aagaagagga aacuaguaau      60

guuggauuag ggcucacucu acu                                              83


<210>  135
<211>  73
<212>  RNA
<213>  Homo sapiens

<400>  135
gugggcgggg gcaggugugu gguggguggu ggccugcggu gagcagggcc cucacaccug      60

ccucgccccc cag                                                         73


<210>  136
<211>  118
<212>  RNA
<213>  Homo sapiens

<400>  136
gccuuggugc ugauuccugg gcucugaccu gagaccucug gguucugagc ugugauguug      60

cucucgagcu gggaucuccg gggucuuggu ucagggccgg ggccucuggg uuccaagc       118


<210>  137

<211>  97
<212>  RNA
<213>  Homo sapiens

<400>  137
ugugaucacu gucuccagcc ugcugaagcu cagagggcuc ugauucagaa agaucaucgg      60

auccgucuga gcuuggcugg ucggaagucu caucauc                              97


<210>  138
<211>  71
<212>  RNA
<213>  Homo sapiens

<400>  138
agguuguucu ggguggucug gagauuugug cagcuuguac cugcacaaau cuccggacca      60

cuuagucuuu a                                                          71


<210>  139
<211>  87
<212>  RNA
<213>  Homo sapiens

<400>  139
ucagagugag guaguagauu guauaguugu gggguaguga uuuuacccug uucaggagau      60

aacuauacaa ucuauugccu ucccuga                                         87


<210>  140
<211>  80
<212>  RNA
<213>  Homo sapiens

<400>  140
cugggggcuc caaagugcug uucgugcagg uagugugauu acccaaccua cugcugagcu      60

agcacuuccc gagcccccgg                                                 80


<210>  141
<211>  77
<212>  RNA
<213>  Homo sapiens

<400>  141
ugcaaugccc uacucagaaa ggugccauuu auguagauuu uaugucacug gcuccuuucu      60

ggguagagca aggcuca                                                    77


<210>  142
<211>  79
<212>  RNA
<213>  Homo sapiens

<400>  142
aauuaauccc ucucuuucua guucuuccua gagugaggaa aagcuggguu gagagggcaa      60

acaaauuaac uaauuaauu                                                  79

<210> 143
<211> 138
<212> RNA
<213> Homo sapiens

<400> 143
uguuauuuuu ugucuucuac cuaagaauuc ugucucuuag gcuuucucuu cccagauuuc 60

ccaaaguugg gaaaagcugg guugagaggg caaaaggaaa aaaaaagaau ucugucucug 120

acauaauuag auagggaa 138


<210> 144
<211> 81
<212> RNA
<213> Homo sapiens

<400> 144
ccuuggccau guaaaagugc uuacagugca gguagcuuuu ugagaucuac ugcaauguaa 60

gcacuucuua cauuaccaug g 81


<210> 145
<211> 96
<212> RNA
<213> Homo sapiens

<400> 145
cgggccccgg gcgggcggga gggacgggac gcggugcagu guuguuuuuu cccccgccaa 60

uauugcacuc gucccggccu ccggcccccc cggccc 96


<210> 146
<211> 75
<212> RNA
<213> Homo sapiens

<400> 146
uggagugggg gggcaggagg ggcucaggga gaaagugcau acagccccug gcccucucug 60

cccuuccguc cccug 75


<210> 147
<211> 84
<212> RNA
<213> Homo sapiens

<400> 147
acuggucggu gauuuaggua guuuccuguu guugggaucc accuuucucu cgacagcacg 60

acacugccuu cauuacuuca guug 84


<210> 148
<211> 97
<212> RNA
<213> Homo sapiens

<400> 148
cauuggcauc uauuagguug gugcaaaagu aauugcgguu uuugccauua cuuucaguag 60

caaaaaucuc aauuacuuuu gcaccaacuu aauacuu 97

<210> 149
<211> 84
<212> RNA
<213> Homo sapiens

<400> 149
ucucagucug uggcacucag ccuugagggc acuuucuggu gccagaauga aagugcuguc 60

auagcugagg uccaaugacu gagg 84

<210> 150
<211> 98
<212> RNA
<213> Homo sapiens

<400> 150
gguacuucuc agucuguggc acucagccuu gagggcacuu ucuggugcca gaaugaaagu 60

gcugucauag cugaggucca augacugagg cgagcacc 98

<210> 151
<211> 91
<212> RNA
<213> Homo sapiens

<400> 151
aagcugcuua guguccuuag agucuccaga ggcaucccua acccagaauc uuuugacugu 60

ccucuggaga gaaaggcagu aggucuguac c 91

<210> 152
<211> 81
<212> RNA
<213> Homo sapiens

<400> 152
cuucaggaag cugguuucau auggugguuu agauuuaaau agugauuguc uagcaccauu 60

ugaaaucagu guucuugggg g 81

<210> 153
<211> 89
<212> RNA
<213> Homo sapiens

<400> 153
accaugcugu agugugugua aacauccuac acucucagcu gugagcucaa gguggcuggg 60

agaggguugu uuacuccuuc ugccaugga 89

<210> 154
<211> 75
<212> RNA
<213> Homo sapiens

<400> 154
ugcccuggcu caguuaucac agugcugaug cugucuauuc uaaagguaca guacugugau 60

aacugaagga uggca                                                        75


<210>  155
<211>  79
<212>  RNA
<213>  Homo sapiens

<400>  155
acuguccuuu uucgguuauc augguaccga ugcuguauau cugaaaggua caguacugug        60

auaacugaag aaugguggu                                                     79


<210>  156
<211>  78
<212>  RNA
<213>  Homo sapiens

<400>  156
uugugcuuuc agcuucuuua cagugcugcc uuguagcauu caggucaagc agcauuguac        60

agggcuauga aagaacca                                                      78


<210>  157
<211>  78
<212>  RNA
<213>  Homo sapiens

<400>  157
uacugcccuc ggcuucuuua cagugcugcc uuguugcaua uggaucaagc agcauuguac        60

agggcuauga aggcauug                                                      78


<210>  158
<211>  89
<212>  RNA
<213>  Homo sapiens

<400>  158
gucagcagug ccuuagcagc acguaaauau uggcguuaag auucuaaaau uaucuccagu        60

auuaacugug cugcugaagu aagguugac                                          89


<210>  159
<211>  81
<212>  RNA
<213>  Homo sapiens

<400>  159
guuccacucu agcagcacgu aaauauuggc guagugaaau auauauuaaa caccaauauu        60

acugugcugc uuuaguguga c                                                  81


<210>  160
<211>  82
<212>  RNA
<213>  Homo sapiens

<400>  160

agggggcgag ggauuggaga gaaaggcagu uccugauggu ccccucccca ggggcuggcu 60

uuccucuggu ccuucccucc ca 82

<210> 161
<211> 110
<212> RNA
<213> Homo sapiens

<400> 161
ccuggccucc ugcagugcca cgcuccgugu auuugacaag cugaguugga cacuccaugu 60

gguagagugu caguuuguca aauaccccaa gugcggcaca ugcuuaccag 110

<210> 162
<211> 66
<212> RNA
<213> Homo sapiens

<400> 162
agggcuccug acuccagguc cuguguguua ccuagaaaua gcacuggacu uggagucaga 60

aggccu 66

<210> 163
<211> 66
<212> RNA
<213> Homo sapiens

<400> 163
agggcuccug acuccagguc cuguguguua ccuagaaaua gcacuggacu uggagucaga 60

aggccu 66

<210> 164
<211> 72
<212> RNA
<213> Homo sapiens

<400> 164
cuugggaaug gcaaggaaac cguuaccauu acugaguuua guaaugguaa ugguucucuu 60

gcuauaccca ga 72

<210> 165
<211> 80
<212> RNA
<213> Homo sapiens

<400> 165
ugggaugagg uaguagguug uauaguuuua gggucacacc caccacuggg agauaacuau 60

acaaucuacu gucuuuccua 80

<210> 166
<211> 72
<212> RNA
<213> Homo sapiens

<400>  166
agguugaggu aguagguugu auaguuuaga auuacaucaa gggagauaac uguacagccu      60

ccuagcuuuc cu                                                          72


<210>  167
<211>  74
<212>  RNA
<213>  Homo sapiens

<400>  167
gggugaggua guagguugua uaguuugggg cucugcccug cuaugggaua acuauacaau      60

cuacugucuu uccu                                                        74


<210>  168
<211>  59
<212>  RNA
<213>  Homo sapiens

<400>  168
caguguucau guagauguuu aagcucuugc aguagguuuu ugcaagcuag ugaacgcug       59


<210>  169
<211>  101
<212>  RNA
<213>  Homo sapiens

<400>  169
ccgcccccgc gucuccaggg caaccguggc uuucgauugu uacuguggga acuggaggua      60

acagucuaca gccauggucg ccccgcagca cgcccacgcg c                         101


<210>  170
<211>  90
<212>  RNA
<213>  Homo sapiens

<400>  170
cucacagcug ccagugucau uuuugugauc ugcagcuagu auucucacuc caguugcaua      60

gucacaaaag ugaucauugg caggugugge                                       90


<210>  171
<211>  87
<212>  RNA
<213>  Homo sapiens

<400>  171
agcgguggcc agugucauuu uugugauguu gcagcuagua auaugagccc aguugcauag      60

ucacaaaagu gaucauugga aacugug                                          87


<210>  172
<211>  110
<212>  RNA
<213>  Homo sapiens

<400>  172

ccagaggaca ccuccacucc gucuacccag uguuuagacu aucuguucag gacucccaaa 60

uuguacagua gucugcacau ugguuaggcu gggcuggguu agacccucgg 110

<210> 173
<211> 74
<212> RNA
<213> Homo sapiens

<400> 173
ccgcacucuc uccauuacac uacccugccu cuucuccaug agaggcagcg ggguguagug 60

gauagagcac gggu 74

<210> 174
<211> 74
<212> RNA
<213> Homo sapiens

<400> 174
ccgcacucuc uccauuacac uacccugccu cuucuccaug agaggcagcg ggguguagug 60

gauagagcac gggu 74

<210> 175
<211> 71
<212> RNA
<213> Homo sapiens

<400> 175
gccaacccag uguucagacu accuguucag gaggcucuca auguguacag uagucugcac 60

auugguuagg c 71

<210> 176
<211> 71
<212> RNA
<213> Homo sapiens

<400> 176
gccaacccag uguucagacu accuguucag gaggcucuca auguguacag uagucugcac 60

auugguuagg c 71

<210> 177
<211> 85
<212> RNA
<213> Homo sapiens

<400> 177
ccuuagcaga gcuguggagu gugacaaugg uguuuguguc uaaacuauca aacgccauua 60

ucacacuaaa uagcuacugc uaggc 85

<210> 178
<211> 76
<212> RNA
<213> Homo sapiens

<400> 178
gagggggaag acgggaggaa agaagggagu gguuccauca cgccuccuca cuccucuccu 60

cccgucuucu ccucuc 76